# EUROPEAN PATENT APPLICATION

(11) **EP 4 714 975 A1**
(43) Date of publication of application: **25.03.2026**
(21) Application number: 24806666.4
(22) Date of filing: 17.05.2024
(51) Int. Cl.: C07K 16/28, C07K 16/46, C07K 19/00

(54) **ANTIBODY BINDING LILRB4 AND CD3, AND USE THEREOF**

(30) Priority: 18.05.2023 WO PCT/CN2023/095114
(71) Applicant: Nanjing Leads Biolabs Co., Ltd., Jiangbei New Area Nanjing Jiangsu 210031 (CN)
(72) Inventor: DANG, Yujia, Nanjing, Jiangsu 210019 (CN); LIU, Xiaoya, Nanjing, Jiangsu 210019 (CN); JIANG, Duqing, Nanjing, Jiangsu 210019 (CN); DING, Mi, Nanjing, Jiangsu 210019 (CN); HOU, Xiaofan, Nanjing, Jiangsu 210019 (CN); SUN, Jianming, Nanjing, Jiangsu 210019 (CN); HUANG, Xiao, Nanjing, Jiangsu 210019 (CN); LING, Hong, Nanjing, Jiangsu 210019 (CN)
(74) Representative: Bianchetti & Minoja with Trevisan & Cuonzo IPS SRL
(86) International application number: PCT/CN2024/093935
(87) International publication number: WO 2024/235317

(57) **Abstract**

The present disclosure provides an antibody capable of binding to LILRB4 and a bispecific antibody capable of binding to LILRB4 and CD3. Further provided are a nucleic acid molecule encoding the antibodies, an expression vector for use in expressing the antibodies, a host cell, and a method. The present disclosure also relates to a method for treating a solid tumor, multiple myeloma, or leukemia such as acute myeloid leukemia (AML) using the anti-LILRB4×CD3 bispecific antibody of the present disclosure.

## Description

### TECHNICAL FIELD

The present disclosure provides an antibody capable of binding to LILRB4 and a bispecific antibody capable of binding to LILRB4 and CD3. Further provided are a nucleic acid molecule encoding the antibodies, an expression vector for use in expressing the antibodies, a host cell, and a method. The present disclosure also relates to a method for treating a solid tumor, multiple myeloma, or leukemia such as acute myeloid leukemia (AML) using the anti-LILRB4×CD3 bispecific antibody of the present disclosure.

### BACKGROUND

Leukocyte Ig-like receptor B4 (LILRB4), also known as immunoglobulin-like transcript 3 (ILT3 or ILT-3), leukocyte immunoglobulin-like receptor 5 (LIR5 or LIR-5), and CD85k or CD85K, is a type I transmembrane protein that comprises two immunoglobulin domains in the extracellular region and cytoplasmic immunoreceptor tyrosine-based inhibitory motifs (ITIMs) in the intracellular region, and is involved in the down-regulation of immune responses.

LILRB4 supports tumor cell infiltration into tissues and inhibits T cell activity through signaling pathways involving APOE, LILRB4, SHP-2, µPAR, and ARG1 in tumor cells (LILRB4 signalling in leukaemia cells mediates T cell suppression and tumor infiltration, Nature (2018) 562:605-09).

The LILRB4 antibody can activate dendritic cells, relieve the immunosuppressive effect of MSDCs, and have the effect of treating cancer (WO2019099597A2).

Acute myeloid leukemia (AML) is a heterogeneous hematological malignancy, and the most common acute leukemia in adults as well as a common cancer in pediatrics. It is characterized by clonal expansion of myeloid cells in peripheral blood, bone marrow (BM), and/or other tissues (NCCN GUIDELINES^{®} INSIGHTS, Acute Myeloid Leukemia, Version 2.2021). Despite significant advances in the understanding of AML biology, the five-year survival rate for AML patients is only 20%-40% (Expression of Immune Inhibitory Receptor ILT3 in Acute Myeloid Leukemia with Monocytic Differentiation, Cytometry Part B (Clinical Cytometry) 84B:21-29 (2013)). Currently, the treatment of acute myeloid leukemia includes intensive cytotoxic chemotherapy, which is usually followed by myeloablation and stem cell transplantation. Despite treatment, relapsed and refractory diseases remain a major problem in adults and children with acute myeloid leukemia, with the survival rate of relapsed patients being less than 15%. Monocytic AML (FAB M5) is a common subtype in AML patients, accounting for 20% of pediatric AML cases and no less than half of infant AML cases. Clinical studies have also shown that monocytic AML carries a greater risk of bone marrow and extramedullary relapse after stem cell transplantation compared to non-monocytic AML. Therefore, new therapeutic strategies are needed to ameliorate the poor prognosis of patients with relapsed or refractory monocytic AML (A Novel Anti-LILRB4 CAR-T Cell for the Treatment of Monocytic AML. Molecular Therapy 2018, 26 (10), 2487-2495.). LILRB4 is expressed on monocytes, macrophages, and dendritic cells, but not on normal CD34+ hematopoietic stem cells. In addition, LILRB4, a tumor-associated antigen, is significantly upregulated in acute monocytic leukemia (FAB M4, M5), and meanwhile, it is expressed in a leukemic stem cell population. The data indicate that LILRB4 expression is inversely correlated with the survival rate of AML patients.

CD3 bispecific antibodies that are currently being developed for the treatment of AML target antigens such as CD33, CD123, and FLT3, and bispecific antibodies targeting these antigens can cause non-targeted elimination of hematopoietic stem cells (HSCs), resulting in bone marrow suppression or bone marrow ablation and increased susceptibility to infection (A Novel Anti-LILRB4 CAR-T Cell for the Treatment of Monocytic AML. Molecular Therapy 2018, 26 (10), 2487-2495.). In a clinical study (NCT02785900) of SGN-33A targeting CD33 in combination with azacitidine/decitabine, the trial was terminated early due to side effects, particularly relatively high mortality caused by infection (Acute Myeloid Leukemia: Therapeutic Targeting of Stem Cells. Expert Opinion on Therapeutic Targets 2022, 26 (6), 547-556.). Therefore, it is particularly important to identify and target AML cells rather than normal hematopoietic stem cells. LILRB4 is expressed on LSCs, but not on hematopoietic stem cells and multipotent progenitor cells (MPPs). Therefore, the antibody therapy targeting LILRB4 has relatively low bone marrow toxicity, and LILRB4 is a relatively ideal antigen target (A Novel Anti-LILRB4 CAR-T Cell for the Treatment of Monocytic AML. Molecular Therapy 2018, 26 (10), 2487-2495.).

The LILRB4 monoclonal antibodies can exert anti-tumor activity by activating immune effector cells (such as macrophages and NK cells). The LILRB4×CD3 bispecific antibodies can mediate the killing of tumor cells expressing LILRB4 by T cells. CAR-T cells targeting and binding to LILRB4 can provide effective anti-tumor ability through T cell-mediated cytotoxicity. LILRB4 antibodies binding to the toxin can destroy tumor cells. After binding to LILRB4 and being endocytosed by target tumor cells, the therapeutically toxic drug is internalized and can then exert a cytotoxic effect for the purpose of treating tumors (LILRB4, an immune checkpoint on myeloid cells. Blood Science, (2022) 4, 49-56).

The principle of bispecific antibodies is that the bispecific antibodies can simultaneously target two antigens, integrating the binding specificity and biological functions of two antibodies into a single molecule: one is for tumor-associated surface antigens, and the other is for surface antigens on effector cells (such as T cells and NK cells). Through this dual specificity, effector cells are used to produce specific killing of tumor cells (Acute myeloid leukemia targets for bispecific antibodies, Citation: Blood Cancer Journal (2017) 7, e522).

There is a need to develop an antibody specifically binding to LILRB4, and particularly, to develop a bispecific antibody specifically binding to CD3 and LILRB4, thereby utilizing the cytotoxic effect produced by T cell activation to kill tumor cells.

### SUMMARY

The present disclosure provides a novel anti-LILRB4 antibody or an antigen-binding fragment thereof.

In some embodiments, the anti-LILRB4 antibody of the present disclosure is a full-length antibody or an antigen-binding fragment thereof, or a VHH antibody or an antibody comprising a VHH, such as a VHH-Fc.

The present disclosure further provides a multispecific binding molecule, e.g., a multispecific antibody, e.g., a bispecific antibody, constructed using the anti-LILRB4 antibody of the present disclosure. In some embodiments, the bispecific antibody specifically binds to LILRB4 and CD3.

Therefore, the present disclosure provides an LILRB4×CD3 bispecific antibody, which can specifically bind to AML cells highly expressing LILRB4, thereby activating T cells and producing a T-cell cytotoxic effect to kill tumor cells.

In some embodiments, the anti-LILRB4 antibody of the present disclosure specifically binds to LILRB4 (e.g., human LILRB4 and/or monkey (e.g., cynomolgus monkey) LILRB4) with high affinity. In some embodiments, the anti-LILRB4 antibody specifically binds to a cell expressing LILRB4, e.g., a tumor cell.

In some embodiments, the anti-LILRB4 antibody or the antigen-binding fragment thereof of the present disclosure is capable of activating dendritic cells, e.g., inducing the dendritic cells to release cytokines such as tumor necrosis factors, e.g., TNF-α.

In some embodiments, the anti-LILRB4 antibody or the antigen-binding fragment thereof of the present disclosure can promote the differentiation of myeloid cells in PBMCs to a pro-inflammatory phenotype, e.g., reduce the release of IL-10 in a PBMC system.

The anti-CD3/LILRB4 bispecific antibody of the present disclosure has one or more of the following properties:
(1) binding to human and/or monkey (e.g., cynomolgus monkey) LILRB4, e.g., binding to LILRB4 expressed on a cell, e.g., binding to LILRB4 with high affinity;
(2) binding to human and/or monkey (e.g., cynomolgus monkey) CD3 with moderate affinity;
(3) having binding affinity for human LILRB4 that is stronger than that for human CD3 (e.g., the K_{D} value is two orders of magnitude higher), thereby having fewer side effects caused by targeting CD3;
(4) inducing T cell proliferation;
(5) more specifically inducing a T cell to kill a cell expressing LILRB4, e.g., still capable of specifically inducing a T cell to kill a cell expressing LILRB4 at a relatively low effector-to-target ratio;
(6) having relatively good cytokine induction specificity, e.g., inducing inflammatory cytokine release only in a cell expressing LILRB4 (e.g., capable of inducing inflammatory cytokine release even at a relatively low effector-to-target ratio), e.g., an interleukin (e.g., IL-2), an interferon (e.g., IFNγ), and/or a tumor necrosis factor (e.g., TNFα); or
(7) effective in treating an indication associated with LILRB4, such as a solid tumor, multiple myeloma, or leukemia, e.g., acute myeloid leukemia (AML).

### BRIEF DESCRIPTION OF THE DRAWINGS

The preferred embodiments of the present disclosure described in detail below will be better understood when read in conjunction with the following drawings. For the purpose of illustrating the present disclosure, currently preferred embodiments are shown in the drawings. However, it should be understood that the present disclosure is not limited to the precise arrangement and means of the embodiments shown in the drawings.
FIGs. 1A-C show schematic structural diagrams of three anti-LILRB4×CD3 bispecific antibodies.
FIG. 1A shows a schematic structural diagram of huB1G5-CD3Fab, FIG. 1B shows a schematic structural diagram of huB1G5-CD3-2VHH, and FIG. 1C shows a schematic structural diagram of huB1G5-ScFv-2VHH.
FIGs. 2A-B show binding of anti-LILRB4 chimeric antibodies to engineered cells. FIG. 2A shows binding of anti-LILRB4 chimeric antibodies (designated as antibody B1A2 and antibody B1G5) and antibody h193 to engineered cells expressing human/cynomolgus monkey LILRB4 (CHO-K1-huLILRB4 cells and CHO-K1-cynoLILRB4). FIG. 2B shows binding of anti-LILRB4 chimeric antibodies (designated as antibody 2A3 and antibody 1H12) and antibody h193 to cells expressing the human/cynomolgus monkey LILRB4 protein (CHO-K1-huLILRB4 cells and CHO-K1-cynoLILRB4).
FIG. 3 shows binding of humanized anti-LILRB4 antibodies (designated as antibody huB1G5-1 and antibody huB1G5-3) and antibody h193 to tumor cells (MV-411) expressing human LILRB4.
FIG. 4 shows the results of the activation of a Jurkat/NFAT-luc reporter gene system by humanized anti-CD3 antibodies 017-Roche-CD3, DA023AH23L2, and 017-2.

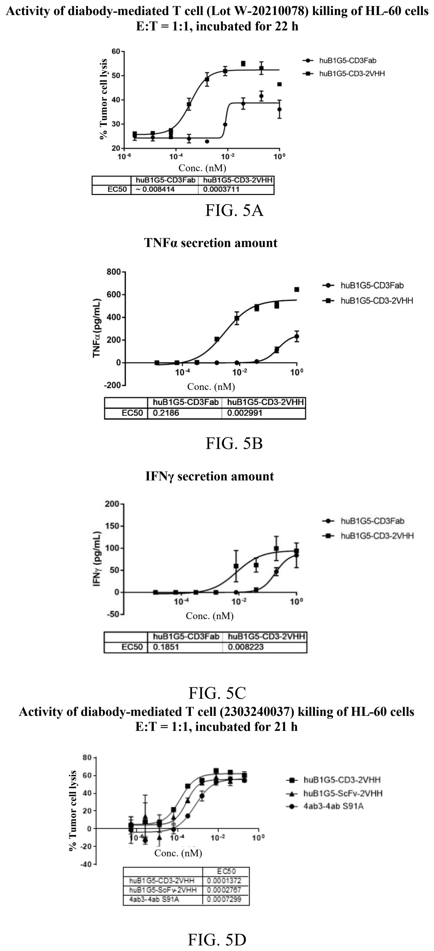

FIGs. 5A-D show that the anti-LILRB4×CD3 bispecific antibodies (designated as huB1G5-CD3Fab and huB1G5-CD3-2VHH) are capable of specifically inducing T cells to kill HL-60 tumor cells (FIG. 5A) and releasing inflammatory cytokines TNFα (FIG. 5B) and IFNγ (FIG. 5C) at a relatively low effector-to-target ratio (E:T = 1:1), and the anti-LILRB4×CD3 bispecific antibodies (designated as huB1G5-CD3-2VHH and huB1G5-ScFv-2VHH) and the bispecific antibody 4ab3-4ab S91A are capable of specifically inducing T cells to kill HL-60 tumor cells (FIG. 5D) at a relatively low effector-to-target ratio (E:T = 1:1).

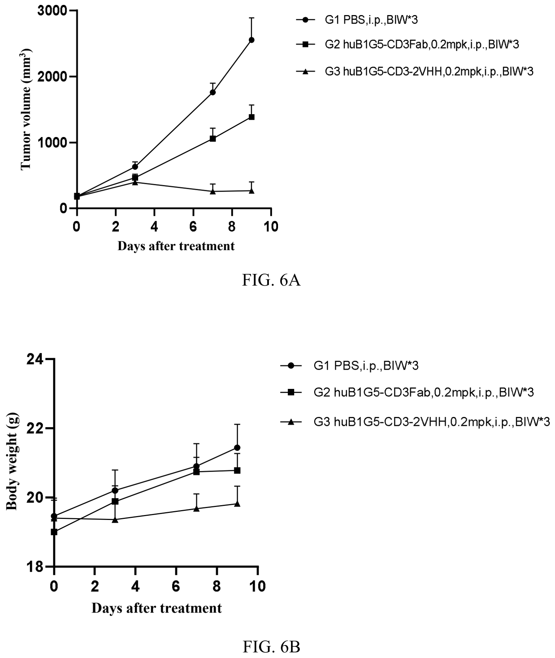

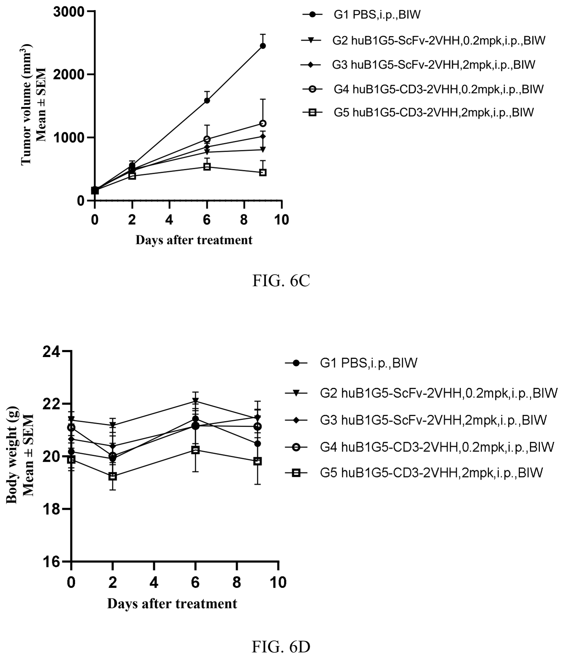

FIGs. 6A-D show the efficacy of the LILRB4×CD3 bispecific antibodies in a solid tumor animal model. FIG. 6A shows the changes in the tumor volume of mice after administration of anti-LILRB4×CD3 bispecific antibodies (designated as huB1G5-CD3Fab and huB1G5-CD3-2VHH) to tumor-bearing mice. FIG. 6B shows the changes in the body weight of mice after administration of anti-LILRB4×CD3 bispecific antibodies (designated as huB1G5-CD3Fab and huB1G5-CD3-2VHH) to tumor-bearing mice. FIG. 6C shows the changes in the tumor volume of mice after administration of anti-LILRB4×CD3 bispecific antibodies (designated as huB1G5-CD3-2VHH and huB1G5-ScFv-2VHH) to tumor-bearing mice. FIG. 6D shows the changes in the body weight of mice after administration of anti-LILRB4×CD3 bispecific antibodies (designated as huB1G5-CD3-2VHH and huB1G5-ScFv-2VHH) to tumor-bearing mice.
FIGs. 7A-C show the changes in the survival of mice (7A), the changes in the fluorescence intensity of tumors in mice (7B), and the changes in the *in vivo* imaging of mice (7C) after administration of anti-LILRB4×CD3 bispecific antibodies (designated as huB1G5-CD3-2VHH and huB1G5-ScFv-2VHH).

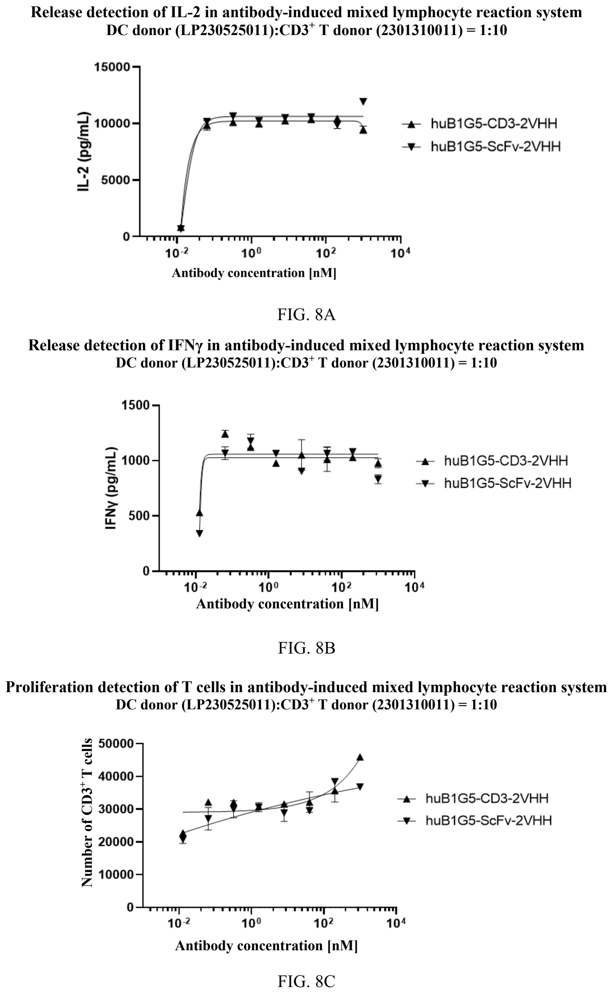

FIGs. 8A-C show the results of the mixed lymphocyte reaction of anti-LILRB4×CD3 bispecific antibodies. It is shown that the anti-LILRB4×CD3 bispecific antibodies (designated as huB 1G5-CD3-2VHH and huB1G5-ScFv-2VHH) can promote the release of IL-2 (FIG. 8A) and IFN-γ (FIG. 8B) in a DC-T mixed lymphocyte reaction system, and the release amount is in a dose-dependent relationship with the antibody concentration. FIG. 8C shows that the anti-LILRB4×CD3 bispecific antibodies (designated as huB1G5-CD3-2VHH and huB1G5-ScFv-2VHH) can promote the proliferation of T cells in a DC-T mixed lymphocyte reaction system, and the amount of cell proliferation is in a dose-dependent relationship with the antibody concentration.
FIGs. 9A-C show that the humanized anti-LILRB4 antibody (designated as huB1G5-3) and antibody h193 can both significantly reduce IL-10 secretion in the system at three test concentrations as compared to two negative controls.

### DETAILED DESCRIPTION OF THE PRESENT DISCLOSURE

It will be appreciated that the present disclosure is not limited to the particular methodology, schemes, and reagents described herein, as these may vary. It will also be appreciated that the terms used herein are only intended to describe specific embodiments rather than limit the scope of the present disclosure, which will be limited only by the claims.

### I. Definitions

In order to illustrate this description, the following definitions will be used, and wherever appropriate, terms used in the singular form may also include the plural form, and vice versa. Unless otherwise defined, any technical and scientific term used herein has the same meaning as commonly understood by those of ordinary skill in the art to which the present disclosure belongs.

The term "about" used in combination with a numerical value is intended to encompass the numerical values in a range from a lower limit less than the specified numerical value by 5% to an upper limit greater than the specified numerical value by 5%.

As used herein, the term "and/or" refers to any one of the options or two or more or all of the options.

As used herein, the term "comprise" or "include" is intended to refer to the inclusion of the elements, integers, or steps, but not the exclusion of any other elements, integers, or steps. The term "comprise" or "include" used herein, unless indicated otherwise, also encompasses the situation where the entirety consists of the described elements, integers, or steps. For example, when referring to an antibody variable region "comprising" a specific sequence, it is also intended to encompass an antibody variable region consisting of the specific sequence.

When referring to "first" and "second" herein, it is only to distinguish two domains or two chains, and does not indicate the locations of the two domains in any way.

The term "CD3" as used herein refers to an antigen expressed on a T cell as part of a multimolecular T cell receptor (TCR), i.e., a T-cell engaging antigen, T-cell surface glycoprotein CD3, which consists of a homodimer or heterodimer formed from two of the following four receptor chains: CD3-ε, CD3-δ, CD3-ζ, and CD3-y. Human CD3-εn (hCD3ε) comprises an amino acid sequence described in UniProtKB/Swiss-Prot: P07766. Human CD3-δ (hCD3δ) comprises an amino acid sequence described in UniProtKB/Swiss-Prot: P04234. In some embodiments, the CD3 described in the present disclosure refers to CD3 from a human or monkey (e.g., cynomolgus monkey).

The term "antibody binding to CD3" or "anti-CD3 antibody" as used herein includes an antibody and an antigen-binding fragment thereof that specifically recognize or bind to a single CD3 subunit (e.g., ε, δ, γ, or ζ), as well as an antibody and an antigen-binding fragment thereof that specifically recognize and bind to a dimeric complex of two CD3 subunits (e.g., γ/ε, δ/ε, and ζ/ζ CD3 dimers). The antibody and the antigen-binding fragment of the present disclosure may bind to soluble CD3, binding CD3, and/or CD3 expressed on the cell surface. The soluble CD3 includes native CD3 proteins and recombinant CD3 protein variants, e.g., monomeric and dimeric CD3 structures that lack a transmembrane region or otherwise do not bind to the cell membrane. In one embodiment, an antigen-binding region binding to CD3 in the anti-CD3 antibody or the antigen-binding fragment thereof or the bispecific antibody of the present disclosure may have relatively low binding activity to CD3 or cells expressing CD3 (e.g., T cells). The binding affinity of the antibody to CD3 may be detected by flow cytometry or bio-layer interferometry. Preferably, in the bispecific antibody molecule of the present disclosure, the antigen-binding region binding to CD3 has binding affinity of 1-1000 nM, e.g., 1-100 nM, for human or monkey (cynomolgus monkey) CD3. In some embodiments, the antigen-binding region binding to CD3 in the anti-CD3 antibody or the antigen-binding fragment thereof or the bispecific antibody of the present disclosure binds to human and/or monkey (e.g., cynomolgus monkey) CD3 with relatively low binding affinity, thereby enabling the activation of human and/or monkey (e.g., cynomolgus monkey) T cells.

Effector cells include effector T cells (T lymphocytes), such as CD4+ T cells, CD8+ T cells, Th1, Th2, and regulatory T cells (Tregs). The effector cells may also include natural killer cells, macrophages, granulocytes, plasma cells, or B cells (lymphocytes).

Leukocyte immunoglobulin-like receptor subfamily B member 4 (LILRB4) is a protein encoded by the LILRB4 gene in humans. This gene is a member of the leukocyte immunoglobulin-like receptor (LIR) family, which is found in a genomic cluster in chromosomal region 19q13.4. The encoded proteins belong to the subfamily B class of LIR receptors, which contain two or four extracellular immunoglobulin domains, a transmembrane domain, and two to four cytoplasmic immunoreceptor tyrosine-based inhibitory motifs (ITIMs). In one embodiment, the human LILRB4 protein of the present disclosure comprises or consists of the amino acid sequence set forth in SEQ ID NO: 65, or an amino acid sequence having at least 85%, 90%, 91%, 92%, 93%, 94%, 95%, 96%, 97%, 98%, or 99% identity. In one embodiment, the cynomolgus monkey LILRB4 protein of the present disclosure comprises or consists of the amino acid sequence set forth in SEQ ID NO: 66, or an amino acid sequence having at least 85%, 90%, 91%, 92%, 93%, 94%, 95%, 96%, 97%, 98%, or 99% identity.

In one embodiment, the antigen-binding region binding to LILRB4 in the anti-LILRB4 antibody or the antigen-binding fragment thereof (e.g., Fab) or the bispecific antibody of the present disclosure has high affinity binding activity for cells expressing human LILRB4. In some embodiments, the bispecific antibody of the present disclosure has higher binding affinity for human LILRB4 than for human CD3, e.g., 1-2 orders of magnitude higher. In one embodiment, the assay is performed by flow cytometry. In one embodiment, the antigen-binding region binding to LILRB4 in the anti-LILRB4 antibody or the antigen-binding fragment thereof or the bispecific antibody of the present disclosure has cross-reactivity to human and monkey (e.g., cynomolgus monkey) LILRB4, i.e., the antigen-binding region is capable of binding to human and monkey (e.g., cynomolgus monkey) LILRB4.

The terms "full antibody", "full-length antibody", "complete antibody", and "intact antibody" may be used interchangeably herein to refer to a naturally occurring glycoprotein comprising at least two heavy (H) chains and two light (L) chains interconnected by disulfide bonds. Each heavy chain consists of a heavy chain variable region (abbreviated herein as VH) and a heavy chain constant region. The heavy chain constant region consists of3 domains, CH1, CH2, and CH3. Each light chain consists of a light chain variable region (abbreviated herein as VL) and a light chain constant region. The light chain constant region consists of one domain, CL. The VH region and the VL region can be further divided into hypervariable regions (complementarity determining regions, or CDRs), with relatively conservative regions (framework regions, or FRs) inserted therebetween. Each VH or VL consists of three CDRs and four FRs, arranged from amino-terminus to carboxyl-terminus in the following order: FR1, CDR1, FR2, CDR2, FR3, CDR3, and FR4. The constant regions are not directly involved in the binding of antibodies to antigens, but exhibit a variety of effector functions. In some embodiments, the antibody heavy chain constant region HC of the present disclosure is a heavy chain constant region of IgG1, IgG2, IgG3, or IgG4, preferably a heavy chain constant region of IgG1. In some embodiments, the heavy chain constant region comprises an LALA mutation. In some embodiments, the heavy chain constant region comprises a D265A mutation and a P329A mutation. In some embodiments, the heavy chain constant region comprises an LALA mutation, a D265A mutation, and a P329A mutation. In some preferred embodiments, the antibody heavy chain constant region HC of the present disclosure
(i) comprises or consists of an amino acid sequence having at least 85%, 90%, 91%, 92%, 93%, 94%, 95%, 96%, 97%, 98%, or 99% identity to an amino acid sequence selected from SEQ ID NOs: 52 and 57;
(ii) comprises or consists of an amino acid sequence selected from SEQ ID NOs: 52 and 57; or
(iii) comprises or consists of an amino acid sequence having one or more (preferably no more than 20 or 10, and more preferably no more than 5, 4, 3, 2, or 1) amino acid modifications (preferably amino acid substitutions, and more preferably conservative amino acid substitutions) compared to an amino acid sequence selected from SEQ ID NOs: 52 and 57.

In some embodiments, the antibody light chain constant region LC of the present disclosure is a lambda or kappa light chain constant region. In some embodiments, the antibody light chain constant region LC of the present disclosure
(i) comprises or consists of an amino acid sequence having at least 85%, 90%, 91%, 92%, 93%, 94%, 95%, 96%, 97%, 98%, or 99% identity to an amino acid sequence selected from SEQ ID NOs: 56 and 58;
(ii) comprises or consists of an amino acid sequence selected from SEQ ID NOs: 56 and 58; or
(iii) comprises or consists of an amino acid sequence having one or more (preferably no more than 20 or 10, and more preferably no more than 5, 4, 3, 2, or 1) amino acid modifications (preferably amino acid substitutions, and more preferably conservative amino acid substitutions) compared to an amino acid sequence selected from SEQ ID NOs: 56 and 58.

The term "antibody fragment" comprises a portion of an intact antibody. In a preferred embodiment, the antibody fragment is an antigen-binding fragment.

The term "antigen-binding fragment" is a portion or segment of an intact antibody or a complete antibody that has fewer amino acid residues than the intact antibody or the complete antibody, which can bind to an antigen or compete with an intact antibody (i.e., an intact antibody from which the antigen-binding fragment is derived) for binding to an antigen. The antigen-binding fragment may be prepared by recombinant DNA techniques, or by enzymatic or chemical cleavage of an intact antibody. The antigen-binding fragments include, but are not limited to, Fab, Fab', F(ab')2, Fv, single-chain Fv, diabodies, and single-domain antibodies (sdAbs). The Fab fragment is a monovalent fragment consisting of VL, VH, CL, and CH1 domains and can be obtained, for example, by papain digestion of a complete antibody. In addition, the F(ab')2, a dimer of the Fab', is a bivalent antibody fragment produced by pepsin digestion of a portion below disulfide bonds in a hinge region of a complete antibody. The F(ab')2 can be reduced by disrupting the disulfide bonds in the hinge region in neutral conditions, and the F(ab')2 dimer is thus converted into Fab' monomers. The Fab' monomer is substantially a Fab fragment with a hinge region (for more detailed descriptions of other antibody fragments, see Fundamental Immunology, edited by W. E. Paul, Raven Press, N.Y. (1993)). The Fv fragment consists of the VL and VH domains of a single arm of an antibody. In addition, although the two domains VL and VH of the Fv fragment are encoded by separate genes, the domains can be linked, using recombinant methods, by a synthetic linker peptide capable of making these two domains produced as a single protein chain in which the VL and VH regions are paired to form a single-chain Fv (scFv). The antibody fragment can be obtained by a chemical method, a recombinant DNA method, or a protease digestion method.

The "Fab fragment" and "Fab" can be used interchangeably herein to refer to an immunoglobulin fragment consisting of two polypeptide chains and comprising an immunoglobulin heavy chain variable domain VH, a heavy chain constant domain CH1, a light chain variable domain VL, and a light chain constant domain CL, wherein one polypeptide chain comprises, from N-terminus to C-terminus, a VH and one constant region selected from CH1 and CL, and the other polypeptide chain comprises, from N-terminus to C-terminus, a VL and the other constant region selected from CL and CH1, wherein the VH and VL domains are paired to form an antigen-binding site. Herein, a Fab chain comprising a heavy chain constant region CH1 is also referred to as a "Fab heavy chain"; correspondingly, a Fab chain comprising a light chain constant region CL is also referred to as a "Fab light chain".

The terms "VHH" and "VHH antibody" are used interchangeably herein and generally refer to an antibody that comprises or consists of only one heavy chain variable region and has an antigen-binding activity. The VHH generally comprises three CDRs and 4 highly-conserved framework regions, and generally has a structure of the following formula: FR1-CDR-FR2-CDR2-FR3-CDR3-FR4, wherein FR1 to FR4 refer to framework regions 1-4; CDR1 to CDR3 refer to complementarity determining regions 1-3. The CDR sequences in the VHH variable region may be determined according to any of the CDR definition schemes described in the "Definition" section, and preferably, the boundaries of the three CDRs in the variable region sequence may be defined according to IMGT. The VHH generally comprises only a heavy chain variable domain derived from a heavy-chain antibody lacking a light chain, also referred to as a nanobody. The VHH used in the present disclosure is preferably from animals of the Camelidae family, such as an alpaca, or a humanized or sequence-optimized form thereof (e.g., an affinity-matured form to increase the binding affinity). In some embodiments, the VHH of the present disclosure is a monovalent monospecific polypeptide molecule consisting of, or consisting essentially of, a single heavy chain variable region (e.g., a heavy chain variable region of a heavy-chain antibody).

The single-domain antibody or the VHH of the present disclosure may also be comprised in a larger polypeptide/protein. Examples of polypeptides/proteins comprising the VHH of the present disclosure include, but are not limited to, a heavy-chain antibody (HcAb), or a bispecific antibody, or a fusion protein. The "heavy-chain antibody" described in the present disclosure refers to an antibody having no light chain, which may comprise, for example, from the N-segment to the C-segment, VH-Fc or VH-CH2-CH3 or VH-hinge region-CH2-CH3, or may comprise VH-CH1-CH2-CH3. The heavy-chain antibody of the present disclosure may also encompass a homodimer, such as a heavy-chain dimer antibody having no light chain. The heavy-chain antibody may comprise a VH from a standard antibody or a VH from a single-domain antibody. For example, a VH in the heavy-chain antibody may be a VHH. In some embodiments, the heavy-chain antibody of the present disclosure may be a heavy-chain antibody having a framework region and/or a heavy chain constant region derived from animals of the Camelidae family (llamas, camels, particularly alpacas), a humanized form thereof or a sequence-optimized form thereof (affinity-matured form), or a fragment thereof (e.g., a fragment comprising at least a part of the constant region). The heavy-chain antibody of the present disclosure further encompasses an antibody formed by fusing the heavy chain variable region or the VHH to an Fc region (e.g., a human IgG Fc region, such as a human IgG1 or IgG4 Fc region). When reference is made to "VHH" in the context of a heavy-chain antibody or a bispecific antibody or a fusion protein, it should be understood that it is a portion of the bispecific antibody and not as a separate molecule.

The term "target" refers to the bound substance against which the binding molecule is directed. The target may be an antigen, or may be a ligand or a receptor.

The term "antigen" refers to a molecule that induces an immune response. Such an immune response may involve antibody production or activation of specific immune cells, or both. Those skilled in the art will appreciate that any macromolecules, including substantially all proteins or peptides, can be used as antigens. In addition, an antigen may be derived from recombinant or genomic DNA. As used herein, the term "epitope" refers to a portion of an antigen that specifically interacts with an antibody molecule.

The term "target-binding region" as used herein refers to a portion of a multispecific binding molecule, e.g., a bispecific binding molecule, that binds to a particular target or an antigen. The target-binding region may be, e.g., an antibody or immunoglobulin *per se* or an antibody fragment. Such a target-binding region may or may not have a tertiary structure independent of the remainder of the bispecific antibody molecule, and may or may not bind to its target as a separate entity. The target-binding region may also be a receptor or a ligand, or a domain of a receptor capable of binding to a ligand. In the case of multispecific antibodies or bispecific antibodies, the "target-binding region" is also referred to as the "antigen-binding region". In one embodiment, the antigen-binding region used in the bispecific antibody molecule of the present disclosure comprises a VH/VL pair consisting of a light chain variable region (VL) and a heavy chain variable region (VH) of the antibody, and the VH/VL pair may be contained in a single polypeptide chain (e.g., scFv) or in two separate polypeptide chains (e.g., contained in a Fab heavy chain and a Fab light chain, respectively). In one embodiment, the antigen-binding region used in the bispecific antibody molecule of the present disclosure may be a Fab. In one embodiment, the antigen-binding region used in the bispecific antibody molecule of the present disclosure may comprise only a VH, e.g., from a VHH, or be a VHH.

When referring to "a target-binding region derived from an antibody", it means that the binding domain of the target-binding region is or is derived from the binding domain of the antibody that specifically binds to an antigen; for example, the heavy chain variable region and/or the light chain variable region of the target-binding region is or is derived from the heavy chain variable region and/or the light chain variable region of the antibody; or 1, 2, 3, 4, 5, or 6 CDRs of the target-binding region are the CDRs of the antibody.

The term "derived from" means that the fragment in the target-binding region is substantially identical to the fragment of the antibody from which it is derived, but has a mutation(s), such as a substitution, a deletion, or an addition, at one or more positions. In one specific embodiment, the mutation is not in the CDR of the antibody. In one specific embodiment, the mutation is not in the variable region of the antibody.

As used herein, the term "monospecific" antibody refers to an antibody having one or more binding regions, each of which binds to the same epitope of the same antigen. For example, the present disclosure provides a monospecific antibody against LILRB4.

As used herein, the term "multispecific" antibody refers to an antibody having at least two antigen-binding regions, in which each antigen-binding site binds to a different epitope of the same antigen or a different epitope of a different antigen. The multispecific antibody is an antibody having binding specificities for at least two different antigen epitopes. In one embodiment, provided herein is a bispecific antibody having binding specificities for a first antigen and a second antigen. For example, the present disclosure provides a bispecific antibody against LILRB4 and CD3.

The term "multispecific binding molecule" refers to a multispecific binding molecule with at least two specificities, e.g., a bispecific binding molecule, that is, the molecule comprises at least a first target-binding region and a second target-binding region, where the first target-binding region binds to one target, and the second target-binding region binds to another target. Accordingly, the multispecific binding molecule according to the present disclosure comprises specificities for binding to at least two different targets. The molecule according to the present disclosure further encompasses a multispecific molecule comprising multiple target-binding regions, e.g., a trispecific binding molecule. In some embodiments, in the case that the binding molecule is an antibody, the target is an antigen. In some embodiments, the bispecific binding molecule of the present disclosure is a bispecific antibody.

Herein, the terms "antibody", "multispecific binding molecule", and "multispecific antibody" are not mutually exclusive when referred to herein.

The term "variable region" or "variable domain" refers to a domain of a heavy chain or light chain of an antibody involved in the binding of the antibody to an antigen. Variable regions of heavy and light chains of native antibodies generally have similar structures, wherein each domain comprises four conserved framework regions (FRs) and three complementarity determining regions.

The "complementarity determining region" or "CDR region" or "CDR" is a region in an antibody variable domain that is highly variable in sequence and forms a structurally defined loop ("hypervariable loop") and/or comprises antigen-contacting residues ("antigen contact sites"). The CDRs are primarily responsible for binding to antigenic epitopes. The CDRs of the heavy and light chains are generally referred to as CDR1, CDR2, and CDR3, and are numbered sequentially from the N-terminus. The CDRs located in the heavy chain variable domain of the antibody are referred to as HCDR1, HCDR2, and HCDR3, whereas the CDRs located in the light chain variable domain of the antibody are referred to as LCDR1, LCDR2, and LCDR3. In a given amino acid sequence of a light chain variable region or a heavy chain variable region, the exact amino acid sequence boundary of each CDR may be determined using any one or a combination of many well-known antibody CDR assignment schemes including, e.g., Chothia based on the three-dimensional structure of antibodies and the topology of the CDR loops (Chothia et al., (1989) Nature, 342: 877-883; Al-Lazikani et al., Standard conformations for the canonical structures of immunoglobulins, Journal of Molecular Biology, 273: 927-948 (1997)), Kabat based on antibody sequence variability (Kabat et al., Sequences of Proteins of Immunological Interest, 4th ed., U.S. Department of Health and Human Services, National Institutes of Health (1987)), AbM (University of Bath), Contact (University College London), International ImMunoGeneTics database (IMGT) (imgt.cines.fr/ on the World Wide Web), and North CDR definition based on the affinity propagation clustering using a large number of crystal structures.

The following are the regional ranges of the CDRs defined using the Kabat, AbM, Chothia, Contact, and IMGT schemes.

| CDR | **Kabat** scheme | AbM scheme | Chothia scheme | Contact scheme | IMGT scheme |
|---|---|---|---|---|---|
| LCDR1 (Kabat and Chothia numbering systems) | L24-L34 | L24-L34 | L26-L32 | L30-L36 | L27-L32 |
| LCDR2 (Kabat and Chothia numbering systems) | L50-L56 | L50-L56 | L50-L52 | L46-L55 | L50-L51 |
| LCDR3 (Kabat and Chothia numbering systems) | L89-L97 | L89-L97 | L91-L96 | L89-L96 | L89-L97 |
| HCDR1 (Kabat numbering system) | H31-H35B | H26-H35B | H26-H32 | H30-H35B | H26-H35B |
| HCDR1 (Chothia numbering system) | H31-H35 | H26-H35 | H26-H32 | H30-H35 | H26-H33 |
| HCDR2 (Kabat and Chothia numbering systems) | H50-H65 | H50-H58 | H52-H56 | H47-H58 | H51-H56 |
| HCDR3 (Kabat and Chothia numbering systems) | H95-H102 | H95-H102 | H96-H101 | H93-H101 | H93-H102 |

Unless otherwise stated, the term "CDR" or "CDR sequence" used in the present disclosure encompasses CDR sequences determined by any one of the schemes described above. CDRs may also be determined based on having the same Kabat numbering positions as a reference CDR sequence (e.g., any one of the exemplary CDRs of the present disclosure). Unless otherwise stated, in the present disclosure, residue positions of an antibody variable region (including heavy chain variable region residues and light chain variable region residues) are determined according to the Kabat numbering system (Kabat et al., Sequences of Proteins of Immunological Interest, 5th ed., Public Health Service, National Institutes of Health, Bethesda, Md. (1991)).

In some embodiments, the HCDRs in the VHH antibody of the present disclosure are determined according to the IMGT scheme.

In some embodiments, the HCDRs and/or LCDRs in the antibody of the present disclosure are determined according to the Kabat scheme.

The term "Fc domain", "Fc region", or "Fc fragment" is used herein to define a C-terminal region of an immunoglobulin heavy chain, which comprises at least a portion of the constant region. The term includes Fc regions of native sequences and variant Fc regions. A native immunoglobulin "Fc domain" comprises two or three constant domains, i.e., a CH2 domain, a CH3 domain, and optionally a CH4 domain. For example, in native antibodies, an immunoglobulin Fc domain comprises the second and the third constant domains (CH2 domain and CH3 domain) derived from two heavy chains of IgG, IgA, and IgD antibodies; or comprises the second, the third, and the fourth constant domains (CH2 domain, CH3 domain, and CH4 domain) derived from two heavy chains of IgM and IgE antibodies.

Unless otherwise stated herein, amino acid residues in the Fc region or the heavy chain constant region are numbered according to the EU numbering system (also known as the EU index) as described in, for example, Edelman, G.M. et al., Proc. Natl. Acad. USA, 63, 78-85 (1969) (https://pubmed.ncbi.nlm.nih.gov/5257969/), see also http://www.imgt.org/IMGTScientificChart/Numbering/Hu_IGHGnber.html. Herein, the terms "Fc domain", "Fc region", or "Fc fragment" does not comprise a heavy chain variable region VH and a light chain variable region VL as well as a heavy chain constant region CH1 and a light chain constant region CL of an immunoglobulin, but in some cases, it may comprise a hinge region at the N-terminus of the heavy chain constant region, such as EPKSS or EPKSC. In some embodiments, the heavy chain constant region Fc suitable for use in the present disclosure is from an antibody heavy chain constant region, e.g., a constant region of human IgG1, IgG2, IgG3, or IgG4, preferably from a constant region of IgG1. In some embodiments, the Fc region comprises a mutation(s) that reduces binding to an Fcy receptor, e.g., an LALA mutation, a D265A mutation, and/or a P329A mutation, preferably an LALA mutation, a D265A mutation, and a P329A mutation. In some embodiments, the Fc comprises or consists of an amino acid sequence set forth in any one of SEQ ID NOs: 53-55 or SEQ ID NO: 68, or an amino acid sequence having at least 85%, 90%, 91%, 92%, 93%, 94%, 95%, 96%, 97%, 98%, or 99% identity thereto. In some embodiments of the present disclosure, the Fc fragments form an Fc dimer by dimerization. In some embodiments, the Fc fragments form an Fc heterodimer by heterodimerization. In the case of heterodimerization of the Fc fragments into a heterodimer, the Fc fragments may comprise a mutation(s) for heterodimerization, such as a knob-into-hole mutation.

The term "CH1 region" refers to a portion of an antibody heavy chain polypeptide that extends from EU position 118 to EU position 216 (the EU numbering system). In some cases, e.g., when constructing a Fab, the CH1 region may also comprise a portion of the hinge region, e.g., comprising EPKSC. Thus, in some embodiments, the term "CH1" region refers to the portion of an antibody heavy chain polypeptide that extends from EU position 118 to EU position 220 (the EU numbering system). In one embodiment, the CH1 domain comprises an amino acid sequence of ASTKGPSVFPLAPSSKSTSGGTAALGCLVKDYFPEPVTVSWNSGALTSGVHTFPAVLQSSGL YSLSSVVTVPSSSLGTQTYICNVNHKPSNTKVDKKV (SEQ ID NO: 51). In some embodiments, the CH1 may further comprise a portion of the hinge region, e.g., EPKSC or EPKSS. For example, in a Fab fragment of an antibody, the CH1 may comprise a hinge region EPKSC.

Examples of "effector functions" of immunoglobulins include: C1q binding and complement-dependent cytotoxicity (CDC), Fc receptor binding, antibody-dependent cell-mediated cytotoxicity (ADCC), antibody-dependent cellular phagocytosis (ADCP), cytokine secretion, immune complex-mediated antigen uptake in antigen-presenting cells, down-regulation of cell surface receptors (e.g., B-cell receptors), and B-cell activation.

The term "chimeric antibody" is an antibody molecule in which: (a) a constant region or a portion thereof is modified, substituted, or exchanged such that antigen-binding sites are linked to constant regions of different or modified classes, effector functions, and/or species, or disparate molecules imparting new properties (e.g., enzymes, toxins, hormones, growth factors, and drugs) to chimeric antibodies, etc.; or (b) a variable region or a portion thereof is modified, substituted, or exchanged by variable regions with different or modified antigen-binding specificities. For example, a mouse antibody can be modified by substituting its constant region with a constant region from a human immunoglobulin. Due to the substitution with a human constant region, the chimeric antibody may retain its specificity for recognizing antigens, while having reduced immunogenicity in humans as compared to the original mouse antibody.

"Humanized antibody" is an antibody that retains the antigen-specific reactivity of a non-human antibody (such as a camelid-derived VHH antibody) and has lower immunogenicity when administered to humans as a therapeutic agent. This can be achieved, for example, by retaining non-human antigen-binding sites and substituting the remainder of the antibodies with their human counterparts (i.e., the portions of the variable regions not involved in binding are substituted with the corresponding parts of human antibodies).

As used herein, the term "anti", "binding", or "specific binding" means that the binding effect is selective for targets or antigens and may be distinguished from undesired or non-specific interactions. The ability of a binding site to bind to a particular target or an antigen may be determined by flow cytometry, enzyme-linked immunosorbent assay (ELISA), or conventional binding assays known in the art, such as radioimmunoassay (RIA), biolayer interferometry, MSD assay, or surface plasmon resonance (SPR).

The "affinity" or "binding affinity" refers to the inherent binding affinity that reflects the interaction between members of a binding pair. The affinity of molecule X for its partner Y may be generally represented by the dissociation constant (K_{D}), which is a ratio of the dissociation rate constant (K_{dis}) to the association rate constant (Kₒₙ). Affinity can be measured by common methods known in the art. One specific method for measuring affinity is the ForteBio kinetic binding assay described herein.

The "percent identity (%)" of an amino acid sequence refers to the percentage of amino acid residues in a candidate sequence that are the same as those of a specific amino acid sequence shown in this description when aligning the candidate sequence with the specific amino acid sequence shown in this description, with gaps introduced if necessary to achieve maximum percent sequence identity and without considering any conservative substitutions as part of sequence identity. In some embodiments, the present disclosure considers variants of the antibody molecule of the present disclosure that have a considerable degree of identity to the antibody molecule and the sequence thereof specifically disclosed herein. For example, the identity is at least 80%, 85%, 90%, 95%, 97%, 98%, 99%, or higher. The variants may comprise conservative modifications, or be conservatively modified variants.

For polypeptide sequences, the "conservative modifications" include substitutions of, deletions of, or additions to a polypeptide sequence but do not substantially change the desired functional activity of the polypeptide sequence. For example, conservative substitutions often result in the substitution of an amino acid with a chemically similar amino acid. Conservative substitution tables providing functionally similar amino acids are well known in the art. 8 groups comprising amino acids that are conservatively substituted with each other are listed as follows: 1) alanine (A) and glycine (G); 2) aspartic acid (D) and glutamic acid (E); 3) asparagine (N) and glutamine (Q); 4) arginine (R) and lysine (K); 5) isoleucine (I), leucine (L), methionine (M), and valine (V); 6) phenylalanine (F), tyrosine (Y), and tryptophan (W); 7) serine (S) and threonine (T); and 8) cysteine (C) and methionine (M). In some embodiments, the term "conservative sequence alteration" is used to refer to an amino acid modification that does not significantly affect or change the binding characteristics for an antigen of interest of the antibody molecule or the binding protein molecule of the present disclosure comprising the amino acid sequence. For example, conservatively modified variants retain at least 80%, 85%, 90%, 95%, 98%, 99%, or higher, such as 100%-110% or higher, binding affinity for an antigen of interest relative to the parent antibody or binding protein.

A "knob-in-hole" mutation is used herein to refer to the introduction of mutations in a first Fc polypeptide and a second Fc polypeptide, respectively, using the "knob-in-hole" technique to form a protuberance ("knob") and a complementary cavity ("hole") at the interface of the first Fc polypeptide and the interface of the second Fc polypeptide. It is known in the art that the "knob-in-hole" technique enables the engineering of the interface between different chains of an antibody molecule to promote the correct association of the chains of the antibody molecule. Generally, this technique involves introducing a "protuberance/knob" at the interface of one chain, and introducing a corresponding "cavity/hole" at the interface of the other chain to be paired with, such that the protuberance can be placed in the cavity. A preferred interface comprises the CH3 domain from the heavy chain constant domain of one chain and the CH3 domain from the heavy chain constant domain of the other chain to be paired with. The protuberance can be constructed by substituting small amino acid side chains at an interface of the CH3 domain from the heavy chain constant domain of one chain with relatively large side chains, such as tyrosine or tryptophan. The compensating cavity of the same size as, or a similar size to, the protuberance is constructed at an interface of the CH3 domain from the heavy chain constant domain of the other chain to be paired with, by substituting large amino acid side chains with relatively small side chains, such as alanine or threonine. Another optional interface comprises a light chain CL domain and a heavy chain CH1 domain of the Fab fragment described above, and the correct heterodimerization between the two chains of the Fab fragment is promoted by constructing a protuberance-cavity interaction.

The "single-chain variable fragment" or "scFv" is used herein to refer to a single-chain antibody fragment comprising a heavy chain variable domain VH and a light chain variable domain VL linked with a linker, wherein the VH and the VL are paired to form an antigen-binding site.

Herein, antibody constant regions or antibody constant domains, including CH1, CL, and Fc domains as well as CH2, CH3, and optionally CH4 domains that constitute the Fc domain, may be selected according to the expected function of the antibody molecule. For example, the constant region may be an IgA, IgD, IgE, IgG, or IgM region, particularly an immunoglobulin constant domain of human IgG, e.g., the constant domain of human IgG1, IgG2, IgG3, or IgG4, preferably the constant domain of human IgG1. The immunoglobulin constant region may have a native sequence or a variant sequence.

The term "linker" as used herein refers to any molecule that enables direct connection of different portions of a bispecific binding molecule. Examples of linkers to establish covalent linkages between different portions of a molecule include peptide linkers and non-protein polymers including, but not limited to, polyethylene glycol (PEG), polypropylene glycol, polyalkylene oxide, or copolymers of polyethylene glycol and polypropylene glycol. In some embodiments, the linker is a peptide linker (also referred to as a "linker peptide") and refers to a short amino acid sequence consisting of amino acids, such as glycine (G) and/or serine (S) and/or threonine (T) residues used alone or in combination, or a hinge region from an immunoglobulin, which is used for linking the amino acid sequence of a first portion of a binding molecule to a second portion of the binding molecule. For example, the peptide linker may link a first target-binding region of a binding molecule to a second target-binding region. For example, the peptide linker may also link one portion of an antibody to another portion of the antibody, e.g., a light chain variable region to a heavy chain variable region. Preferably, the peptide linker has a length sufficient to link two entities in a manner that maintains their conformation relative to each other without interference with the desired activities. In one embodiment, the linker peptide has a length of 5-50 amino acids, such as 10, 15, 20, 25, or 30 amino acids. In one embodiment, the linker peptide comprises amino acid sequences (GS)n (SEQ ID NO: 80), (GGS)n (SEQ ID NO: 81), (GSGGS)n (SEQ ID NO: 82), (GGGGS)n (SEQ ID NO: 83), (GGGS)n (SEQ ID NO: 84), and (GGGGS)nG (SEQ ID NO: 85), wherein n is an integer equal to or greater than 1, for example, n is an integer of 2, 3, 4, 5, 6, 7, 8, 9, or 10. Useful linkers further include glycine-alanine polymers, alanine-serine polymers, and other flexible linkers. In some embodiments, the peptide linker is (GGGGS)n (SEQ ID NO: 86), wherein n = 1, 2, 3, 4, or 5, preferably n = 3 or 4, e.g., the sequence set forth in SEQ ID NO: 60 or 70.

In yet another embodiment, the linker peptide is a hinge region or a portion of a hinge region from an immunoglobulin, including a native hinge region or a portion thereof, or a mutated hinge region or a portion thereof. In one embodiment, the linker peptide is, for example, a hinge region or a portion thereof (e.g., EPKSC (SEQ ID NO: 67)) of an immunoglobulin (e.g., IgG, such as IgG1, IgG2, IgG3, or IgG4), or a mutated hinge region or a portion thereof (e.g., EPKSS (SEQ ID NO: 59)). Alternatively, the computer program simulation for three-dimensional structures of proteins and peptides or a phage display method can be used to rationally design a suitable flexible linker peptide.

The term "host cell" refers to a cell into which an exogenous polynucleotide has been introduced, including the progeny of such cells. Host cells include "transformants" and "transformed cells", which include primary transformed cells and progeny derived therefrom. Host cells are any type of cell system that may be used to produce the antibody molecule of the present disclosure, including eukaryotic cells, e.g., mammalian cells, insect cells, and yeast cells, and prokaryotic cells, e.g., *Escherichia coli* cells. Host cells include cultured cells, as well as cells within a transgenic animal, a transgenic plant, or a cultured plant tissue or animal tissue.

The term "vector" as used herein refers to a nucleic acid molecule capable of proliferating another nucleic acid to which it is linked. The term includes vectors that serve as self-replicating nucleic acid structures as well as vectors integrated into the genome of a host cell into which they have been introduced. The term "expression vector" refers to a vector comprising a recombinant polynucleotide, which comprises an expression control sequence effectively linked to a nucleotide sequence to be expressed. Expression vectors contain sufficient cis-regulatory elements for expression, and other elements for expression may be provided by a host cell or in an *in vitro* expression system. Expression vectors include all those known in the art, including cosmids, plasmids (e.g., naked or contained in liposomes), and viruses (e.g., lentiviruses, retroviruses, adenoviruses, and adeno-associated viruses) into which recombinant polynucleotides are incorporated.

The terms "individual" and "subject" are used interchangeably and refer to a mammal. The mammals include, but are not limited to, domesticated animals (e.g., cows, sheep, cats, dogs, and horses), primates (e.g., human and non-human primates such as monkeys), rabbits, and rodents (e.g., mice and rats). In particular, the individual is a human.

The term "treatment" (or "treat" or "treating") refers to slowing, interrupting, arresting, alleviating, stopping, lowering, or reversing the onset of symptoms, complications, or biochemical indications of a disease, or alleviating symptoms, or arresting or inhibiting the further progression of the disease, symptom, or disorder.

The term "prevention" (or "prevent" or "preventing") includes the inhibition of the development or progression of a disease or disorder, or symptoms of a specific disease or disorder. In some embodiments, subjects with a family history of cancer are candidates for preventive regimens. Generally, in the context of cancer, the term "prevention" refers to the administration of a drug prior to the appearance of signs or symptoms of cancer, particularly in subjects at risk of cancer.

The term "therapeutic agent" described herein encompasses any substance that is effective in preventing or treating a tumor, e.g., cancer, including a chemotherapeutic agent, a cytokine, a cytotoxic agent, an additional antibody, a small molecule drug, or an immunomodulatory agent (e.g., an immunosuppressant).

The term "cytotoxic agent" used in the present disclosure refers to a substance that inhibits or prevents cell functions and/or causes cell death or cell destruction.

"Chemotherapeutic agent" includes chemical compounds useful in the treatment of cancers or immune system diseases.

The term "small molecule drug" refers to a low-molecular-weight organic compound capable of regulating biological processes. The "small molecule" is defined as a molecule with a molecular weight of less than 10 kD, usually less than 2 kD, and preferably less than 1 kD. The small molecule includes but is not limited to inorganic molecules, organic molecules, organic molecules containing inorganic components, molecules containing radioactive atoms, synthetic molecules, peptide mimetics, and antibody mimetics. As therapeutic agents, small molecules penetrate cells better, are less susceptible to degradation, and are less likely to induce an immune response compared to large molecules.

The term "immunomodulatory agent" used herein refers to a natural or synthetic active agent or drug that suppresses or modulates an immune response. The immune response may be a humoral response or a cellular response. The immunomodulatory agent includes an immunosuppressant. In some embodiments, the immunomodulatory agent of the present disclosure includes an immune checkpoint inhibitor or an immune checkpoint agonist.

The term "effective amount" refers to an amount or dose of the antibody, fragment, composition, or combination of the present disclosure which generates expected effects in a patient in need of treatment or prevention after being administered to the patient in a single dose or multiple doses.

The term "therapeutically effective amount" refers to an amount effective to achieve a desired therapeutic result at a necessary dose for a necessary period of time. The therapeutically effective amount is also such an amount that any toxic or adverse effect of the antibody, fragment thereof, composition, or combination is inferior to the therapeutically beneficial effect. The "therapeutically effective amount" preferably inhibits a measurable parameter or improves a measurable parameter by at least about 40%, and even more preferably by at least about 50%, 55%, 60%, 65%, 70%, 75%, 80%, 85%, 90%, or even 100%, relative to untreated subjects.

The term "prophylactically effective amount" refers to an amount effective to achieve a desired prophylactic result at a necessary dose for a necessary period of time. Generally, since a prophylactic dose is administered to a subject before or at an earlier stage of a disease, a prophylactically effective amount will be less than a therapeutically effective amount.

The term "tumor" refers to all neoplastic cell growth and proliferation, whether malignant or benign, and all pre-cancerous and cancerous cells and tissues. The terms "cancer", "carcinoma", and "tumor" are not mutually exclusive when referred to herein. The term "tumor" encompasses both solid tumors and hematological tumors.

The term "anti-tumor effect" or "tumor inhibitory effect" refers to a biological effect that can be demonstrated by a variety of means, including but not limited to, for example, decrease in tumor volume, decrease in the number of tumor cells, decrease in tumor cell proliferation, or decrease in tumor cell viability.

The term "pharmaceutical supplementary material" refers to a diluent, an adjuvant (e.g., Freund's adjuvant (complete and incomplete)), an excipient, a carrier, or a stabilizer, etc., that is administered with an active substance.

The term "pharmaceutical composition" refers to a composition that is present in a form allowing the effective biological activity of an active ingredient contained therein and does not contain additional ingredients having unacceptable toxicity to a subject to which the composition is administered.

The term "pharmaceutical combination or combination product" refers to a non-fixed combination product or a fixed combination product, including but not limited to, a kit and a pharmaceutical composition. The term "non-fixed combination" means that the active ingredients (e.g., (i) the antibody or the bispecific antibody of the present disclosure, and (ii) an additional therapeutic agent) are administered to a patient as separate entities either simultaneously without specific time limitation or sequentially at identical or different time intervals, wherein such administration provides two or more active agents at prophylactically or therapeutically effective levels in the patient. The term "fixed combination" means that two or more active agents are administered to a patient simultaneously in the form of a single entity. The dose and/or time intervals of two or more active agents are preferably selected such that the combined use of the ingredients can result in a therapeutic effect on the disease or disorder greater than that achieved by the use of any one of the ingredients alone. The ingredients may each take a separate formulation form and the formulation forms may be identical or different.

The term "combination therapy" refers to the administration of two or more therapeutic agents or modalities (e.g., radiotherapy or surgery) to treat the diseases as described herein. Such administration includes co-administration of these therapeutic agents in a substantially simultaneous manner, for example, in a single capsule with a fixed proportion of active ingredients. Alternatively, such administration includes co-administration of the active ingredients in a variety of or separate containers (e.g., tablets, capsules, powders, and liquids). The powder and/or liquid may be reconstituted or diluted to a desired dose before administration. In addition, such administration further includes using each type of the therapeutic agents at approximately the same time or in a sequential manner at different times. In any case, the therapeutic regimen will provide the beneficial effect of the pharmaceutical combination in the treatment of disorders or symptoms described herein.

The "subject/patient/individual sample" refers to a collection of cells or fluids obtained from a patient or a subject. The source of tissue or cell samples can be solid tissues, e.g., from fresh, frozen, and/or preserved organ or tissue samples or biopsy samples or puncture samples; blood or any blood component; body fluids such as tears, vitreous humors, cerebrospinal fluids, amniotic fluids, peritoneal fluids, or interstitial fluids; and cells from a subject at any time during pregnancy or development. In some embodiments, the tissue sample is a tumor tissue. Tissue samples may comprise compounds that are naturally not mixed with tissues, such as preservatives, anticoagulants, buffers, fixatives, nutrients, and antibiotics.

### II. Anti-LILRB4 Antibody

In one aspect, the present disclosure provides an LILRB4 antibody, which has higher binding affinity for LILRB4. In some embodiments, the LILRB4 antibody of the present disclosure is suitable for use in the construction of an antigen-binding region in a bispecific antibody molecule.

In some embodiments, the anti-LILRB4 antibody or the antigen-binding fragment thereof of the present disclosure binds to LILRB4 (e.g., human LILRB4 or monkey LILRB4, such as cynomolgus monkey LILRB4) with high affinity. In some embodiments, the anti-LILRB4 antibody or the antigen-binding fragment thereof of the present disclosure is capable of binding to both human LILRB4 and monkey LILRB4 (e.g., cynomolgus monkey LILRB4).

In some embodiments, the anti-LILRB4 antibody or the antigen-binding fragment thereof of the present disclosure binds to LILRB4 expressed by a cell. In some embodiments, the affinity of the anti-LILRB4 antibody for LILRB4 expressed by a cell is determined by flow cytometry.

### Single-domain antibody

In some embodiments, the anti-LILRB4 antibody of the present disclosure is a single-domain antibody, particularly a VHH antibody.

The single-domain antibody or the VHH antibody has a molecular weight of about one tenth of a human IgG molecule, and a physical diameter of only a few nanometers. Due to the small molecular size, a single-domain monoclonal antibody has the following advantages over conventional four-chain antibodies: high stability and solubility, and the ability to recognize hidden antigenic sites. In addition, the single-domain antibody is also cheaper to prepare than conventional four-chain antibodies. In addition to the use thereof as an individual molecule, the single-domain antibody is also a suitable component for the construction of multispecific molecules.

In some embodiments, the anti-LILRB4 single-domain antibody of the present disclosure is a VHH antibody comprising or consisting of a heavy chain variable region typically having the following structure: FR1-VHH CDR1-FR2-VHH CDR2-FR3-VHH CDR3-FR4, wherein FR1 to FR4 refer to framework regions 1-4; VHH CDR1 to VHH CDR3 refer to complementarity determining regions 1-3. The CDR sequences in the VHH variable region may be determined according to any of the CDR definition schemes described in the "Definition" section, and preferably, the boundaries of the three CDRs in the VHH sequence may be defined according to IMGT.

In some embodiments, the anti-LILRB4 VHH antibody of the present disclosure comprises
(i) three complementarity determining regions (CDRs) contained in a VH set forth in any one of SEQ ID NOs: 11, 16, 21, 25, 30, 32, 36, 39, and 42, or
(ii) a sequence comprising a total of at least one and no more than 5, 4, 3, 2, or 1 amino acid modifications (preferably amino acid substitutions, and preferably conservative substitutions) in the three CDRs relative to the sequence of (i);
preferably, sequences of the CDRs are defined according to IMGT.

In some embodiments, the anti-LILRB4 VHH antibody of the present disclosure comprises or consists of a heavy chain variable region comprising
(i) three complementarity determining regions (CDRs) contained in a VH set forth in any one of SEQ ID NOs: 11, 16, 21, 25, 30, 32, 36, 39, and 42, or
(ii) a sequence comprising a total of at least one and no more than 5, 4, 3, 2, or 1 amino acid modifications (preferably amino acid substitutions, and preferably conservative substitutions) in the three CDRs relative to the sequence of (i);
preferably, sequences of the CDRs are defined according to IMGT.

In some embodiments, the anti-LILRB4 VHH antibody of the present disclosure comprises the complementarity determining regions (CDRs) VHH CDR1, VHH CDR2, and VHH CDR3. In some embodiments, the anti-LILRB4 VHH of the present disclosure comprises or consists of a heavy chain variable region comprising complementarity determining regions (CDRs) VHH CDR1, VHH CDR2, and VHH CDR3.

In some embodiments, the VHH CDR1 comprises or consists of an amino acid sequence selected from SEQ ID NOs: 12, 17, 26, and 75, or the VHH CDR1 comprises an amino acid sequence having one, two, or three modifications (preferably amino acid substitutions, and preferably conservative substitutions) compared to an amino acid sequence selected from SEQ ID NOs: 12, 17, 26, and 75.

In some embodiments, the VHH CDR2 comprises or consists of an amino acid sequence of SEQ ID NO: 13, 18, 22, 27, 33, or 37, or the VHH CDR2 comprises an amino acid sequence having one, two, or three modifications (preferably amino acid substitutions, and preferably conservative substitutions) compared to an amino acid sequence of SEQ ID NO: 13, 18, 22, 27, 33, or 37.

In some embodiments, the VHH CDR3 comprises or consists of an amino acid sequence selected from SEQ ID NOs: 14, 19, 23, 28, 34, and 40, or the VHH CDR3 comprises an amino acid sequence having one, two, or three modifications (preferably amino acid substitutions, and preferably conservative substitutions) compared to an amino acid sequence selected from SEQ ID NOs: 14, 19, 23, 28, 34, and 40.

In one embodiment, the anti-LILRB4 VHH antibody of the present disclosure comprises complementarity determining regions (CDRs) VHH CDR1, VHH CDR2, and VHH CDR3, wherein
(i) the VHH CDR1 comprises or consists of the amino acid sequence set forth in SEQ ID NO: 12, the VHH CDR2 comprises or consists of the amino acid sequence set forth in SEQ ID NO: 13, and the VHH CDR3 comprises or consists of the amino acid sequence set forth in SEQ ID NO: 14; or
(ii) the VHH CDR1 comprises or consists of the amino acid sequence set forth in SEQ ID NO: 17, the VHH CDR2 comprises or consists of the amino acid sequence set forth in SEQ ID NO: 18, and the VHH CDR3 comprises or consists of the amino acid sequence set forth in SEQ ID NO: 19;
(iii) the VHH CDR1 comprises or consists of the amino acid sequence set forth in SEQ ID NO: 75, the VHH CDR2 comprises or consists of the amino acid sequence set forth in SEQ ID NO: 22, and the VHH CDR3 comprises or consists of the amino acid sequence set forth in SEQ ID NO: 23;
(iv) the VHH CDR1 comprises or consists of the amino acid sequence set forth in SEQ ID NO: 26, the VHH CDR2 comprises or consists of the amino acid sequence set forth in SEQ ID NO: 27, and the VHH CDR3 comprises or consists of the amino acid sequence set forth in SEQ ID NO: 28;
(v) the VHH CDR1 comprises or consists of the amino acid sequence set forth in SEQ ID NO: 17, the VHH CDR2 comprises or consists of an amino acid sequence set forth in SEQ ID NO: 33 or 37, and the VHH CDR3 comprises or consists of an amino acid sequence set forth in SEQ ID NO: 34 or 40;
(vi) the VHH CDR1 comprises or consists of the amino acid sequence set forth in SEQ ID NO: 17, the VHH CDR2 comprises or consists of an amino acid sequence set forth in SEQ ID NO: 33 or 37, and the VHH CDR3 comprises or consists of the amino acid sequence set forth in SEQ ID NO: 34; or
(vii) the VHH CDR1 comprises or consists of the amino acid sequence set forth in SEQ ID NO: 17, the VHH CDR2 comprises or consists of an amino acid sequence set forth in SEQ ID NO: 33 or 37, and the VHH CDR3 comprises or consists of the amino acid sequence set forth in SEQ ID NO: 40.

In one embodiment, the anti-LILRB4 VHH antibody of the present disclosure comprises or consists of a heavy chain variable region comprising complementarity determining regions (CDRs) VHH CDR1, VHH CDR2, and VHH CDR3, wherein
(i) the VHH CDR1 comprises or consists of the amino acid sequence set forth in SEQ ID NO: 12, the VHH CDR2 comprises or consists of the amino acid sequence set forth in SEQ ID NO: 13, and the VHH CDR3 comprises or consists of the amino acid sequence set forth in SEQ ID NO: 14; or
(ii) the VHH CDR1 comprises or consists of the amino acid sequence set forth in SEQ ID NO: 17, the VHH CDR2 comprises or consists of the amino acid sequence set forth in SEQ ID NO: 18, and the VHH CDR3 comprises or consists of the amino acid sequence set forth in SEQ ID NO: 19;
(iii) the VHH CDR1 comprises or consists of the amino acid sequence set forth in SEQ ID NO: 75, the VHH CDR2 comprises or consists of the amino acid sequence set forth in SEQ ID NO: 22, and the VHH CDR3 comprises or consists of the amino acid sequence set forth in SEQ ID NO: 23;
(iv) the VHH CDR1 comprises or consists of the amino acid sequence set forth in SEQ ID NO: 26, the VHH CDR2 comprises or consists of the amino acid sequence set forth in SEQ ID NO: 27, and the VHH CDR3 comprises or consists of the amino acid sequence set forth in SEQ ID NO: 28;
(v) the VHH CDR1 comprises or consists of the amino acid sequence set forth in SEQ ID NO: 17, the VHH CDR2 comprises or consists of an amino acid sequence set forth in SEQ ID NO: 33 or 37, and the VHH CDR3 comprises or consists of an amino acid sequence set forth in SEQ ID NO: 34 or 40;
(vi) the VHH CDR1 comprises or consists of the amino acid sequence set forth in SEQ ID NO: 17, the VHH CDR2 comprises or consists of an amino acid sequence set forth in SEQ ID NO: 33 or 37, and the VHH CDR3 comprises or consists of the amino acid sequence set forth in SEQ ID NO: 34; or
(vii) the VHH CDR1 comprises or consists of the amino acid sequence set forth in SEQ ID NO: 17, the VHH CDR2 comprises or consists of an amino acid sequence set forth in SEQ ID NO: 33 or 37, and the VHH CDR3 comprises or consists of the amino acid sequence set forth in SEQ ID NO: 40.

In some embodiments, the anti-LILRB4 VHH antibody of the present disclosure comprises or consists of a heavy chain variable region
(i) comprising or consisting of an amino acid sequence having at least 90%, 91%, 92%, 93%, 94%, 95%, 96%, 97%, 98%, or 99% identity to an amino acid sequence selected from any one of SEQ ID NOs: 11, 16, 21, 25, 30, 32, 36, 39, and 42; or
(ii) comprising or consisting of an amino acid sequence selected from any one of SEQ ID NOs: 11, 16, 21, 25, 30, 32, 36, 39, and 42; or
(iii) comprising an amino acid sequence having one or more (preferably no more than 10, and more preferably no more than 5, 4, 3, 2, or 1) amino acid modifications (preferably amino acid substitutions, and more preferably conservative amino acid substitutions) compared to an amino acid sequence selected from any one of SEQ ID NOs: 11, 16, 21, 25, 30, 32, 36, 39, and 42, wherein preferably, the amino acid modifications do not occur in the CDRs.

In some embodiments, the anti-LILRB4 VHH antibody of the present disclosure comprises or consists of an amino acid sequence selected from any one of SEQ ID NOs: 11, 16, 21, 25, 30, 32, 36, 39, and 42.

In some embodiments, the VHH antibody of the present disclosure comprises CDR amino acid sequences and/or framework (FR) amino acid sequences derived from a Camelidae heavy-chain antibody produced by immunizing animals of the Camelidae family (e.g., an alpaca). In some embodiments, a VHH monoclonal antibody of the present disclosure derived from the Camelidae heavy-chain antibody may be engineered, for example, to comprise framework region sequences derived from human amino acid sequences (i.e., human antibodies) or other non-Camelidae mammalian species. In one embodiment, to further improve the properties (e.g., affinity) of the engineered antibody, camelid-derived amino acid residues at corresponding positions in the parent camelid-derived antibody may be introduced into the engineered antibody by a back-mutation(s) at one or more positions (e.g., framework regions).

In one embodiment, the VHH antibody of the present disclosure is a humanized antibody. The humanization may be achieved by the following method: substituting one or more amino acid residues, particularly framework region sequences, of a native VHH sequence of a non-human origin (e.g., a VHH sequence derived from animals of the Camelidae family or the alpaca after immunization) with residues from the heavy chain VH of the conventional human antibody at the corresponding positions. Methods for humanizing VHHs are well known in the art, for example, as described in Example 5. Generally, the humanization substitutions are made in a manner that preserves the favorable binding properties of the single-domain antibody. Assays for determining biological properties of the humanized single-domain antibody, such as binding affinity, are well known in the art to determine and select a suitable humanized residue mutation or a combination of mutations.

In some embodiments, the humanized single-domain antibody of the present disclosure may be obtained by a method comprising the following steps:
determining a CDR loop structure of a parent single-domain antibody (e.g., a camelid-derived VHH antibody screened from a phage display library);
finding the closest homologous sequence for each V/J region in a human germline sequence database as a template, for example, by comparing with the IMGT human antibody heavy chain variable region germline gene database (http://www.imgt.org/3Dstructure-DB/cgi/DomainGapAlign.cgi), and selecting a heavy chain variable region germline gene with high homology to the VHH antibody as a template;
grafting the CDRs of the VHH antibody separately into the selected corresponding humanized templates to form a variable region sequence of FR1-CDR1-FR2-CDR2-FR3-CR3-FR4, wherein preferably, a framework sequence for substitution is structurally similar to a framework sequence of an antibody to be humanized, e.g., a framework sequence having at least 80%, 85%, 90%, or 95%, 96%, 97%, 98%, 99% or more sequence identity; and
back-mutating key amino acids in the FR region to amino acids corresponding to the VHH antibody as needed to ensure the original affinity, so as to obtain the humanized anti-LILRB4 VHH antibody, and optionally sequencing the VHH antibody.

In some embodiments, the back-mutation site is selected from one or more of the frameworks (FRs).

In some embodiments, the heavy chain variable region germline gene suitable for humanizing the VHH antibody of the present disclosure is IGHV3-7*01.

In some embodiments, the present disclosure further provides a functional variant of the single-domain antibody of the present disclosure (in particular the VHH antibody). The functional variant may be obtained by the methods well known in the present disclosure, for example, by introducing a mutation(s) into the encoding nucleic acid sequences of exemplary single-domain antibodies of the present disclosure, e.g., into CDR sequences and/or FR sequences, and then screening (e.g., by phage display library screening) variants that retain the desired properties, e.g., via random or site-directed mutagenesis. Generally, the functional variant retains significant sequence identity to the parent single-domain antibody (or the VHH). Preferably, the functional variant retains the desired biological properties of the parent single-domain antibody (or the VHH). For example, the variant has comparable (e.g., at least 50%, 60%, 70%, or 80%, preferably 90% or more) or improved biological activity (e.g., 110%-150% or higher) relative to the biological activity of the parent. The desired biological properties include, for example, but are not limited to, binding affinity for an antigen of interest (e.g., LILRB4) (e.g., as measured by KD values), blocking activity against the binding of the antigen of interest to a receptor (e.g., as measured by IC50 values), activation activity on T cells in *in-vitro* or *in-vivo* assays (e.g., as measured by release amount of cytokines), and inhibition of tumor growth/survival in *in-vitro* or *in-vivo* assays.

### Heavy-chain antibody

In another aspect of the present disclosure, the present disclosure further provides a heavy-chain antibody comprising the heavy chain variable region of the VHH antibody of the present disclosure.

In some embodiments, the single-domain antibody or the VHH (e.g., a camelid-derived VHH or a humanized form thereof) of the present disclosure may be linked to a constant region of a human antibody or a portion thereof (e.g., an Fc region) to produce a heavy-chain antibody comprising a VHH-constant region or a VHH-CH1-Fc or a VHH-Fc. In one embodiment, the heavy-chain antibody comprises the VHH antibody of the present disclosure and the Fc region at the C-terminus thereof. In some embodiments, the VHH is linked to the Fc via a hinge region or a portion thereof, for example, a hinge region from an IgG (e.g., a hinge region from IgG1, IgG2, IgG3, or IgG4) or a portion thereof.

In some embodiments, the anti-LILRB4 heavy-chain antibody of the present disclosure comprises the VHH or the heavy chain variable region therein as defined herein, and a heavy chain constant region, or an Fc region of the heavy chain constant region. In some embodiments, a linker peptide, such as an antibody hinge region or a portion thereof, e.g., a hinge region or a portion thereof from an IgG (comprising a native or mutated IgG hinge region or a portion thereof), is contained between the VHH or the heavy chain variable region thereof and the heavy chain constant region or the Fc region.

In some embodiments, the linker peptide is a hinge region from human IgG1, human IgG2, human IgG3, or human IgG4 or a portion thereof, comprising a native or mutated hinge region or a portion thereof, such as a hinge region from human IgG1. For example, the linker peptide is EPKSS (SEQ ID NO: 59) or EPKSC (SEQ ID NO: 67).

In one embodiment, the heavy-chain antibody comprises an Fc region or a portion thereof from animals of the Camelidae family (e.g., an alpaca). In one embodiment, the heavy-chain antibody is produced and isolated by immunizing animals of the Camelidae family, e.g., an alpaca. A variety of methods are known in the art for immunizing animals of the Camelidae family and isolating the VHH antibody or the heavy-chain antibody produced against the antigen of interest.

In some embodiments, the heavy-chain antibody comprises a constant region from a human or non-human primate (e.g., cynomolgus monkey) antibody, e.g., a constant region from human IgG1, human IgG2, human IgG3, or human IgG4.

In some embodiments, the heavy-chain antibody comprises an Fc region or a portion thereof from a human or non-human primate (e.g., cynomolgus monkey). In yet another embodiment, the heavy-chain antibody comprises a human IgG Fc region, e.g., a human IgG1, human IgG2, human IgG3, or human IgG4 Fc region, preferably a human IgG 1 or human IgG4 Fc region, such as a human IgG 1 Fc region.

In one embodiment, the heavy-chain antibody according to the present disclosure may be dimerized with another polypeptide chain comprising an Fc region (e.g., another heavy-chain antibody, which is the same or different) via the Fc region. Thus, in one embodiment, the present disclosure further provides a homo- or heteromultimeric protein comprising the heavy-chain antibody of the present disclosure. In one preferred embodiment, the protein preferably comprises a heavy-chain antibody formed by pairing two identical heavy-chain antibody chains.

In some embodiments, the anti-LILRB4 heavy-chain antibody of the present disclosure is a chimeric antibody. In some embodiments, the anti-LILRB4 antibody of the present disclosure is a humanized antibody.

The Fc region of the present disclosure may be mutated to obtain desired properties. The mutation(s) for the Fc region is known in the art, as defined below.

In some embodiments, the anti-LILRB4 antibody or the antigen-binding fragment thereof of the present disclosure comprises a heavy chain comprising a heavy chain variable region, an Fc region, and a linker peptide linking the heavy chain variable region and the Fc region. Preferably, the linker peptide comprises or consists of an amino acid sequence set forth in SEQ ID NO: 59 or 67.

In some embodiments, the anti-LILRB4 antibody or the antigen-binding fragment thereof of the present disclosure comprises or consists of a heavy chain comprising or consisting of a heavy chain variable region of the VHH of the present disclosure, a linker peptide, and an Fc region, wherein the heavy chain
(i) comprises or consists of an amino acid sequence having at least 85%, 90%, 91%, 92%, 93%, 94%, 95%, 96%, 97%, 98%, or 99% identity to an amino acid sequence selected from any one of SEQ ID NOs: 15, 20, 24, 29, 31, 35, 38, 41, and 43; or
(ii) comprises or consists of an amino acid sequence selected from any one of SEQ ID NOs: 15, 20, 24, 29, 31, 35, 38, 41, and 43; or
(iii) comprises an amino acid sequence having one or more (preferably no more than 10, and more preferably no more than 5, 4, 3, 2, or 1) amino acid modifications (preferably amino acid substitutions, and more preferably conservative amino acid substitutions) compared to an amino acid sequence selected from any one of SEQ ID NOs: 15, 20, 24, 29, 31, 35, 38, 41, and 43, wherein preferably, the amino acid modifications do not occur in the CDRs.

### III. Humanized CD3 Antibody

In one aspect, the present disclosure provides a humanized CD3 antibody, which has better binding affinity for CD3 and is therefore more suitable for use in the construction of an antigen-binding region in a bispecific antibody molecule.

In some embodiments, the anti-CD3 antibody or the antigen-binding fragment thereof of the present disclosure binds to CD3 (e.g., human CD3 or monkey CD3, such as cynomolgus monkey CD3) with desired affinity. In some embodiments, the anti-CD3 antibody or the antigen-binding fragment thereof of the present disclosure is capable of binding to both human CD3 and monkey CD3 (e.g., cynomolgus monkey CD3). |In some embodiments, the affinity of the antibody is determined by biolayer interferometry or surface plasmon resonance.

In some embodiments, the anti-CD3 antibody of the present disclosure binds to human CD3 or monkey CD3 (e.g., cynomolgus monkey CD3) with an equilibrium dissociation constant (K_{D}) of about 1-1000 nM. In some embodiments, the anti-CD3 antibody of the present disclosure binds to monkey CD3 (e.g., cynomolgus monkey CD3) with a K_{D} of about 10-100 nM, or 20-100 nM, or 50-100 nM. In some embodiments, the anti-CD3 antibody of the present disclosure binds to human CD3 with a K_{D} of about 100-1000 nM (e.g., about 200-1000 nM, 300-1000 nM, 400-1000 nM, or 500-1000 nM).

In some embodiments, the anti-CD3 antibody or the antigen-binding fragment thereof of the present disclosure binds to CD3 on the effector cell surface. In some embodiments, the anti-CD3 antibody or the antigen-binding fragment thereof of the present disclosure is capable of activating effector cells. In some embodiments, the effector cell is a T cell. In some embodiments, the binding is determined by flow cytometry. In some embodiments, the activation of the CD3 antibody is determined using a reporter gene assay system (e.g., a Jurkat/NFAT-luc reporter gene system).

In some embodiments, the anti-CD3 antibody or the antigen-binding fragment thereof of the present disclosure is capable of activating effector cells to induce the killing of tumor cells.

In some embodiments, the anti-CD3 antibody or the antigen-binding fragment thereof of the present disclosure comprises 3 complementarity determining regions from a heavy chain variable region (HCDRs): HCDR1, HCDR2, and HCDR3.

In some embodiments, the anti-CD3 antibody or the antigen-binding fragment thereof of the present disclosure comprises 3 complementarity determining regions from a light chain variable region (LCDRs): LCDR1, LCDR2, and LCDR3.

In some embodiments, the anti-CD3 antibody or the antigen-binding fragment thereof of the present disclosure comprises 3 complementarity determining regions from a heavy chain variable region (HCDRs) and 3 complementarity determining regions from a light chain variable region (LCDRs).

In some aspects, the anti-CD3 antibody or the antigen-binding fragment thereof of the present disclosure comprises a heavy chain variable region (VH). In some aspects, the anti-CD3 antibody or the antigen-binding fragment thereof of the present disclosure comprises a light chain variable region (VH). In some aspects, the anti-CD3 antibody or the antigen-binding fragment thereof of the present disclosure comprises a heavy chain variable region (VH) and a light chain variable region (VL). In some embodiments, the heavy chain variable region comprises 3 complementarity determining regions (CDRs) from a heavy chain variable region: HCDR1, HCDR2, and HCDR3. In some embodiments, the light chain variable region comprises 3 complementarity determining regions (CDRs) from a light chain variable region: LCDR1, LCDR2, and LCDR3.

In some embodiments, the anti-CD3 antibody or the antigen-binding fragment thereof of the present disclosure further comprises an antibody heavy chain constant region HC. In some embodiments, the anti-CD3 antibody or the antigen-binding fragment thereof of the present disclosure further comprises an antibody light chain constant region LC. In some embodiments, the anti-CD3 antibody or the antigen-binding fragment thereof of the present disclosure further comprises a heavy chain constant region HC and a light chain constant region LC.

In some embodiments, the heavy chain variable region VH of the anti-CD3 antibody of the present disclosure
(i) comprises or consists of an amino acid sequence having at least 90%, 91%, 92%, 93%, 94%, 95%, 96%, 97%, 98%, or 99% identity to the amino acid sequence of SEQ ID NO: 3; or
(ii) comprises or consists of the amino acid sequence of SEQ ID NO: 3; or
(iii) comprises or consists of an amino acid sequence having one or more (preferably no more than 10, and more preferably no more than 5, 4, 3, 2, or 1) amino acid modifications (preferably amino acid substitutions, and more preferably conservative amino acid substitutions) compared to the amino acid sequence of SEQ ID NO: 3, wherein preferably, the amino acid modifications do not occur in the CDRs.

In some embodiments, the light chain variable region VL of the anti-CD3 antibody of the present disclosure
(i) comprises or consists of an amino acid sequence having at least 90%, 91%, 92%, 93%, 94%, 95%, 96%, 97%, 98%, or 99% identity to the amino acid sequence of SEQ ID NO: 4; or
(ii) comprises or consists of the amino acid sequence of SEQ ID NO: 4; or
(iii) comprises or consists of an amino acid sequence having one or more (preferably no more than 10, and more preferably no more than 5, 4, 3, 2, or 1) amino acid modifications (preferably amino acid substitutions, and more preferably conservative amino acid substitutions) compared to the amino acid sequence of SEQ ID NO: 4, wherein preferably, the amino acid modifications do not occur in the CDRs.

In some embodiments, the 3 complementarity determining regions from the heavy chain variable region (HCDRs) of the anti-CD3 antibody of the present disclosure, HCDR1, HCDR2, and HCDR3, are
(i) three complementarity determining regions HCDR1, HCDR2, and HCDR3 contained in the VH set forth in SEQ ID NO: 3; or
(ii) a sequence comprising at least one and no more than 5, 4, 3, 2, or 1 amino acid modifications (preferably amino acid substitutions, and preferably conservative substitutions) in the three HCDRs relative to any sequence of (i);
preferably, the HCDRs are determined according to Kabat.

In some embodiments, the 3 complementarity determining regions from the light chain variable region (LCDRs) of the anti-CD3 antibody of the present disclosure, LCDR1, LCDR2, and LCDR3, are
(i) three complementarity determining regions LCDR1, LCDR2, and LCDR3 contained in the VL set forth in SEQ ID NO: 4; or
(ii) a sequence comprising at least one and no more than 5, 4, 3, 2, or 1 amino acid modifications (preferably amino acid substitutions, and preferably conservative substitutions) in the three LCDRs relative to any sequence of (i);
preferably, the LCDRs are determined according to Kabat.

In some embodiments, in the anti-CD3 antibody of the present disclosure,
the HCDR1 comprises or consists of the amino acid sequence of SEQ ID NO: 5; the HCDR2 comprises or consists of the amino acid sequence of SEQ ID NO: 6; the HCDR3 comprises or consists of the amino acid sequence of SEQ ID NO: 7; the LCDR1 comprises or consists of the amino acid sequence of SEQ ID NO: 8; the LCDR2 comprises or consists of the amino acid sequence of SEQ ID NO: 9; and/or the LCDR3 comprises or consists of the amino acid sequence of SEQ ID NO: 10.

In some specific embodiments of the present disclosure, the anti-CD3 antibody or the antigen-binding fragment thereof of the present disclosure comprises a VH and a VL, wherein
the VH comprises or consists of the amino acid sequence set forth in SEQ ID NO: 3 or an amino acid sequence having at least 90%, 91%, 92%, 93%, 94%, 95%, 96%, 97%, 98%, or 99% identity thereto, and the VL comprises or consists of the amino acid sequence set forth in SEQ ID NO: 4 or an amino acid sequence having at least 90%, 91%, 92%, 93%, 94%, 95%, 96%, 97%, 98%, or 99% identity thereto.

In some specific embodiments of the present disclosure, the anti-CD3 antibody or the antigen-binding fragment thereof comprises three complementarity determining regions HCDR1, HCDR2, and HCDR3 contained in the VH set forth in SEQ ID NO: 3, and three complementarity determining regions LCDR1, LCDR2, and LCDR3 contained in the VL set forth in SEQ ID NO: 4.

In some specific embodiments of the present disclosure, the anti-CD3 antibody or the antigen-binding fragment thereof of the present disclosure comprises: the HCDR1 set forth in SEQ ID NO: 5, the HCDR2 set forth in SEQ ID NO: 6, the HCDR3 set forth in SEQ ID NO: 7, the LCDR1 set forth in SEQ ID NO: 8, the LCDR2 set forth in SEQ ID NO: 9, and the LCDR3 set forth in SEQ ID NO: 10.

In one embodiment of the present disclosure, the amino acid modifications described herein include amino acid substitutions, insertions, or deletions. In a preferred embodiment, the amino acid modification described in the present disclosure occurs in a region outside CDRs (e.g., in an FR). More preferably, the amino acid modification described in the present disclosure occurs in a region outside the heavy chain variable regions and/or outside the light chain variable regions. Preferably, the amino acid modification described herein is an amino acid substitution, preferably a conservative substitution.

In some embodiments, the anti-CD3 antibody or the antigen-binding fragment thereof of the present disclosure has one or more of the following properties:
(i) showing the same or similar binding affinity and/or specificity for CD3 as the antibody of the present disclosure;
(ii) inhibiting (e.g., competitively inhibiting) the binding of the antibody of the present disclosure to CD3;
(iii) binding to the same or overlapping epitope as the antibody of the present disclosure;
(iv) competing with the antibody of the present disclosure for binding to CD3;
(v) having one or more biological properties of the antibody of the present disclosure.

In some embodiments, the anti-CD3 antibody of the present disclosure is an antibody in the form of IgG1, IgG2, IgG3, or IgG4, for example, an antibody in the form of IgG1. In some embodiments, the heavy chain constant region of the anti-CD3 antibody of the present disclosure is a constant region of IgG1, IgG2, IgG3, or IgG4. In some embodiments, the light chain constant region of the anti-CD3 antibody of the present disclosure is a kappa or lambda light chain constant region, e.g., a lambda light chain constant region.

In some embodiments, the anti-CD3 antibody is a monoclonal antibody.

In some embodiments, the anti-CD3 antibody is humanized.

In one embodiment, the anti-CD3 antibody of the present disclosure also encompasses an antibody fragment (e.g., an antigen-binding fragment) thereof, preferably an antibody fragment selected from: a Fab, a Fab', a Fab'-SH, an Fv, a single-chain antibody (e.g., scFv), a (Fab')₂, a single-domain antibody (e.g., VHH), a domain antibody (dAb), or a linear antibody.

In one embodiment, the anti-CD3 antibody fragment of the present disclosure is a Fab comprising the VH and VL, and the CH1 and CL described herein. In one embodiment, an anti-CD3 antibody fragment suitable for use in the construction of the bispecific antibody of the present disclosure is a Fab.

In one embodiment, the Fab comprises the heavy chain variable region VH and the light chain variable region VL described in this section.

In some embodiments, the Fab heavy chain comprises a VH and a CH1 (and optionally comprises a portion of the hinge region, EPKSS or EPKSC, at the C-terminus of the CH1), wherein the VH is the VH of an anti-CD3 antibody.

In some embodiments, the Fab light chain comprises a VL-CL, wherein the VL is the VL of an anti-CD3 antibody. In some embodiments, the CL is a light chain constant region from an antibody kappa or lambda light chain, preferably a light chain constant region from a kappa light chain.

In some embodiments, the Fab heavy chain
(i) comprises or consists of an amino acid sequence having at least 85%, 90%, 91%, 92%, 93%, 94%, 95%, 96%, 97%, 98%, or 99% identity to the amino acid sequence selected from SEQ ID NO: 87;
(ii) comprises or consists of the amino acid sequence selected from SEQ ID NO: 87; or
(iii) comprises or consists of an amino acid sequence having one or more (preferably no more than 10 or 10, and more preferably no more than 5, 4, 3, 2, or 1) amino acid modifications (preferably amino acid substitutions, and more preferably conservative amino acid substitutions) compared to the amino acid sequence selected from SEQ ID NO: 87.

In some embodiments, the Fab light chain
(i) comprises or consists of an amino acid sequence having at least 85%, 90%, 91%, 92%, 93%, 94%, 95%, 96%, 97%, 98%, or 99% identity to the amino acid sequence selected from SEQ ID NO: 2;
(ii) comprises or consists of the amino acid sequence selected from SEQ ID NO: 2; or
(iii) comprises or consists of an amino acid sequence having one or more (preferably no more than 10 or 10, and more preferably no more than 5, 4, 3, 2, or 1) amino acid modifications (preferably amino acid substitutions, and more preferably conservative amino acid substitutions) compared to the amino acid sequence selected from SEQ ID NO: 2.

In one embodiment, the anti-CD3 antibody fragment of the present disclosure is an scFv comprising the VH and the VL described herein as well as an optional linker peptide, wherein, for example, the linker peptide is (GGGGS)ₙ, wherein n = 1, 2, 3, 4, or 5, e.g., n = 3 or 4; for example, the linker peptide is the linker set forth in SEQ ID NO: 70. In some embodiments, the scFv comprises or consists of the amino acid sequence set forth in SEQ ID NO: 70 or an amino acid sequence having at least 85%, 90%, 91%, 92%, 93%, 94%, 95%, 96%, 97%, 98%, or 99% identity thereto (preferably having the same 1-6 CDRs therewith). In one embodiment, an anti-CD3 antibody fragment suitable for use in the construction of the bispecific antibody of the present disclosure is an scFv.

### IV. Multispecific Antibody

In some embodiments, the anti-LILRB4 antibody of the present disclosure is a multispecific antibody, e.g., a bispecific antibody, and, for example, comprises one binding specificity for LILRB4 and other binding specificities for one or more molecules (e.g., CD3).

Therefore, one aspect of the present disclosure relates to a bispecific antibody, which comprises
a first antigen-binding region and a second antigen-binding region, wherein the first antigen-binding region specifically binds to CD3, and/or the second antigen-binding region specifically binds to LILRB4.

In some embodiments, the first antigen-binding region is from the anti-CD3 antibody, e.g., an SP34 antibody or a humanized antibody thereof, e.g., an anti-CD3 humanized antibody described herein, e.g., a Fab fragment or an scFv fragment of the anti-CD3 antibody.

In some embodiments, the second antigen-binding region is from the anti-LILRB4 antibody or the antigen-binding fragment thereof described herein, e.g., an anti-LILRB4 VHH.

The first antigen-binding region suitable for use in the bispecific antibody of the present disclosure can comprise or consist of an anti-CD3 full-length antibody or an antigen-binding fragment thereof as long as it is capable of specifically binding to CD3, including, but not limited to, for example, a full-length antibody, a single-chain Fv (scFv), a Fab, a Fab', an (Fab)2, a single-domain antibody, a VHH, or a heavy-chain antibody specifically binding to CD3.

The second antigen-binding region suitable for use in the bispecific antibody of the present disclosure can comprise or consist of an anti-LILRB4 full-length antibody or an antigen-binding fragment thereof as long as it is capable of specifically binding to CD3, including, but not limited to, for example, a full-length antibody, a single-chain Fv (scFv), a Fab, a Fab', an (Fab)2, or a single-domain antibody specifically binding to LILRB4.

The second antigen-binding region suitable for use in the bispecific antibody of the present disclosure can comprise or consist of the anti-LILRB4 VHH or the heavy-chain antibody of the present disclosure as long as it is capable of specifically binding to LILRB4, including, but not limited to, for example, a VHH or a heavy-chain antibody specifically binding to LILRB4.

In some embodiments, the multispecific antibody such as the bispecific antibody of the present disclosure is an IgG-like bispecific antibody. The term "IgG-like bispecific antibody" described herein refers to a bispecific antibody comprising an Fc dimer. Therefore, in some embodiments, the bispecific antibody of the present disclosure comprises an Fc dimer.

In some embodiments, the bispecific antibody of the present disclosure is an IgG-like bispecific antibody comprising a Fab fragment as an antigen-binding region that specifically binds to one antigen, and a VHH as an antigen-binding region that specifically binds to another antigen. In some embodiments, the IgG-like bispecific antibody comprises a Fab specifically binding to CD3 as a first antigen-binding region, and a VHH specifically binding to LILRB4 as a second antigen-binding region.

In some embodiments, the bispecific antibody of the present disclosure is an IgG-like bispecific antibody comprising an scFv fragment as an antigen-binding region that specifically binds to one antigen, and a VHH as an antigen-binding region that specifically binds to another antigen. In some embodiments, the IgG-like bispecific antibody comprises an scFv specifically binding to CD3 as a first antigen-binding region, and a VHH specifically binding to LILRB4 as a second antigen-binding region.

In some embodiments, the first antigen-binding region specifically binding to CD3 is selected from an scFv and a Fab, and/or the second antigen-binding region specifically binding to LILRB4 is a VHH.

In one embodiment, the bispecific antibody may comprise one or more first antigen-binding regions. In one embodiment, the bispecific antibody may comprise one or more second antigen-binding regions. In one embodiment, the bispecific antibody comprises one first antigen-binding region and one or more (e.g., two) second antigen-binding regions.

### ➢ Fab fragment suitable for use in bispecific antibody of the present disclosure

In some embodiments, the Fab fragment that is one of the antigen-binding regions of the bispecific antibody consists of two polypeptide chains comprising VH, CH1, VL, and CL domains of the antibody, wherein the VH is paired with the VL, and the CH1 is paired with the CL to form the antigen-binding region. In some embodiments, in the Fab, one chain comprises, from N-terminus to C-terminus, a VH and a CH1 (i.e., a VH-CH1), and the other chain comprises, from N-terminus to C-terminus, a VL and a CL (i.e., a VL-CL).

In some embodiments, in the bispecific antibody, the Fab may be fused to the N-terminus of the Fc domain of the antibody via the C-terminus of the chain comprising the VH, wherein the Fc domain may or may not comprise a hinge region (e.g., EPKSS or EPKSC). Preferably, the Fab comprises a VH-CH1 chain and a VL-CL chain and is fused to the Fc domain of the antibody via the C-terminus of the CH1 of the VH-CH1 chain, wherein the Fc domain may or may not comprise a hinge region (e.g., EPKSS or EPKSC). Herein, a Fab chain linked to an Fc dimer is also referred to as a Fab heavy chain, and a Fab chain not linked to an Fc dimer is also referred to as a Fab light chain. In some embodiments, the fusion is direct fusion or fusion via a linker such as a linker peptide.

In some embodiments, the CH1 in the Fab may also comprise a portion of the hinge region, e.g., EPKSS or EPKSC, to facilitate the formation of a stable structure, such as to facilitate the production of a multispecific antibody. Where the Fc fused to the heavy chain of the Fab does not comprise the portion of the hinge region, or where the Fab heavy chain is fused to a non-Fc domain, the portion of the hinge region may be comprised at the C-terminus of the CH1 to facilitate the formation of a stable structure. Where the Fc fused to the heavy chain of the Fab comprises the portion of the hinge region, the C-terminus of the CH1 of the Fab heavy chain may not comprise the portion of the hinge region.

In some embodiments, the Fab fragment contained in the bispecific antibody of the present disclosure specifically binds to CD3. In some embodiments, the Fab fragment contained in the bispecific antibody of the present disclosure is from the anti-CD3 antibody of the present disclosure and comprises the heavy chain variable region VH and the light chain variable region VL of the anti-CD3 antibody of the present disclosure.

In some embodiments, the Fab heavy chain comprises a VH and a CH1 (and optionally comprises a portion of a hinge region, EPKSS or EPKSC, at the C-terminus of the CH1), wherein the VH is the VH of the anti-CD3 antibody of the present disclosure. In some embodiments, the CH1 is a CH1 from IgG1, IgG2, IgG3, or IgG4, preferably a CH1 from IgG1. In some embodiments, the CH1
(i) comprises or consists of an amino acid sequence having at least 85%, 90%, 91%, 92%, 93%, 94%, 95%, 96%, 97%, 98%, or 99% identity to the amino acid sequence selected from SEQ ID NO: 51;
(ii) comprises or consists of the amino acid sequence selected from SEQ ID NO: 51; or
(iii) comprises or consists of an amino acid sequence having one or more (preferably no more than 10 or 10, and more preferably no more than 5, 4, 3, 2, or 1) amino acid modifications (preferably amino acid substitutions, and more preferably conservative amino acid substitutions) compared to the amino acid sequence selected from SEQ ID NO: 51.

In some embodiments, the Fab light chain comprises a VL-CL, wherein the VL is the VL of the anti-CD3 antibody of the present disclosure. In some embodiments, the CL is a light chain constant region from an antibody kappa or lambda light chain, preferably a light chain constant region from a kappa light chain. In some embodiments, the CL is the light chain constant region of the anti-CD3 antibody of the present disclosure. In some embodiments, the Fab light chain is the light chain of the anti-CD3 antibody of the present disclosure.

### ➢ scFv suitable for use in bispecific antibody of the present disclosure

**In** some embodiments, the scFv fragment that is one of the binding regions of the bispecific antibody consists of one polypeptide chain comprising VH and VL domains of the antibody, wherein the VH and the VL are linked (e.g., via a linker) to be paired to form the antigen-binding site. In some embodiments, the scFv is a trans-configuration comprising, from N-terminus to C-terminus: a VH, a linker, and a VL (VH-linker-VL). In some other embodiments, the scFv is a cis-configuration comprising, from N-terminus to C-terminus: a VL, a linker, and a VH (VL-linker-VH). In some embodiments, the linker is a peptide linker consisting of amino acid residues. Suitable peptide linkers are known to those skilled in the art. In one embodiment, the linker has a length of 5-50 amino acids, such as 5-30 amino acids, for example, 15 amino acids or 20 amino acids. In one embodiment, the linker comprises an amino acid sequence (G4S)n, where n = 1, 2, 3, 4, or 5, preferably n = 3 or 4, and more preferably n = 3.

In some preferred embodiments, the scFv antigen-binding region contained in the antibody molecule of the present disclosure is a disulfide-stabilized scFv.

In some embodiments, in the bispecific antibody, the scFv may be fused via the C-terminus of the chain to the N-terminus of the Fc domain of the antibody. In some embodiments, in the bispecific antibody, the scFv is fused at the N-terminus of the chain to the C-terminus of another antigen-binding region (e.g., the Fab heavy chain) and fused at the C-terminus of the chain to the N-terminus of the Fc domain of the antibody.

In some embodiments, in the bispecific antibody, the scFv may be fused via the C-terminus of the chain comprising the VL to the N-terminus of the Fc domain of the antibody. In some embodiments, in the bispecific antibody, the scFv is fused at the N-terminus of the VH chain to the C-terminus of another antigen-binding region (e.g., the Fab heavy chain) and fused at the C-terminus of the VL chain to the N-terminus of the Fc domain of the antibody.

In some embodiments, in the bispecific antibody, the scFv may be fused via the C-terminus of the chain comprising the VH to the N-terminus of the Fc domain of the antibody. In some embodiments, in the bispecific antibody, the scFv is fused at the N-terminus of the VL chain to the C-terminus of another antigen-binding region (e.g., the Fab heavy chain) and fused at the C-terminus of the VH chain to the N-terminus of the Fc domain of the antibody.

In some embodiments, the scFv contained in the bispecific antibody of the present disclosure specifically binds to CD3. In some embodiments, the scFv contained in the bispecific antibody of the present disclosure is from the anti-CD3 antibody of the present disclosure and comprises the heavy chain variable region VH and the light chain variable region VL of the anti-CD3 antibody of the present disclosure.

### ➢ Fc dimer suitable for use in bispecific antibody of the present disclosure

In one embodiment, two Fc regions in the bispecific antibody of the present disclosure are dimerized to form a dimeric Fc. Preferably, the two Fc regions form a heterodimeric Fc by heterodimerization.

In some embodiments, the first and second Fc regions are identical. In some other embodiments, the first Fc region and the second Fc region are different, and the two are paired and heterodimerized.

The Fc region fragment suitable for use in the antibody molecule of the present disclosure may be any antibody Fc region. The Fc region may include a native sequence Fc region and a variant Fc region. The native sequence Fc domain encompasses naturally occurring Fc sequences of various immunoglobulins, such as Fc regions of various Ig subtypes and allotypes thereof (Gestur Vidarsson et al., IgG subclasses and allotypes: from structure to effector functions, 20 October 2014, doi: 10.3389/fimmu.2014.00520). For example, the Fc region of the antibody of the present disclosure may comprise two or three constant domains, i.e., a CH2 domain, a CH3 domain, and optionally a CH4 domain. In some embodiments, the antibody Fc region may also bear an IgG hinge region or a portion of the IgG hinge region, e.g., an IgG1 hinge region or a portion of the IgG1 hinge region, at the N-terminus. The hinge region may comprise a mutation(s). In some embodiments, the hinge region may be EPKSS or EPKSC.

Preferably, the Fc region of the antibody of the present disclosure comprises CH2-CH3 from the N-terminus to the C-terminus, or comprises hinge region-CH2-CH3 from the N-terminus to the C-terminus. In some embodiments, the Fc region suitable for use in the antibody or the bispecific antibody of the present disclosure is a human IgG Fc, such as a human IgG1 Fc, a human IgG2 Fc, a human IgG3 Fc, or a human IgG4 Fc. In one embodiment, the Fc region derives from a human IgG1 Fc, e.g., comprises or consists of an amino acid sequence set forth in SEQ ID NO: 55 or 68, or an amino acid sequence having at least 90% identity, e.g., 95%, 96%, 97%, 99%, or higher identity thereto.

The Fc region in the antibody or the bispecific antibody of the present disclosure may be mutated to obtain desired properties. The mutation(s) for the Fc region is known in the art.

In one embodiment, the Fc region is modified in properties of the effector function of the Fc region (e.g., complement activation function of the Fc region). In one embodiment, the effector function has been reduced or eliminated relative to a wild-type isotype Fc region. In one embodiment, the effector function is reduced or eliminated by using a method selected from: using an Fc isotype that naturally has a reduced or an eliminated effector function, and performing Fc region modification.

In one preferred embodiment, the Fc region has a reduced effector function mediated by the Fc region, such as a reduced or an eliminated ADCC or ADCP or CDC effector function, for example, comprising a mutation(s) for achieving the above function.

As understood by those skilled in the art, according to the expected use of the antibody molecule of the present disclosure, the antibody molecule of the present disclosure may further comprise a modification in the Fc domain that alters the binding affinity to one or more Fc receptors. In one embodiment, the Fc receptor is an Fcy receptor, in particular a human Fcy receptor. In some embodiments, the Fc region comprises a mutation(s) that reduces binding to the Fcy receptor. For example, in some embodiments, the Fc region used in the present disclosure has one or more of an L234A/L235A mutation, a D265A mutation, and a P329A mutation, which reduce binding to the Fcy receptor. In some embodiments, the Fc region used in the present disclosure has an L234A/L235A mutation, a D265A mutation, and a P329A mutation, which reduce binding to the Fcy receptor. In yet another preferred embodiment, the Fc fragment may have a mutation(s) that leads to an increased serum half-life, e.g., a mutation(s) that improves the binding of the Fc fragment to FcRn. In some embodiments, the Fc region comprising a mutation(s) that reduces binding to the Fcy receptor comprises or consists of the amino acid sequence of SEQ ID NO: 53 or 54, or an amino acid sequence having at least 90% identity, e.g., 95%, 96%, 97%, 99%, or higher identity thereto. In some embodiments, the Fc region comprises an amino acid sequence having at least 90% identity, e.g., 95%, 96%, 97%, 99%, or higher identity, to SEQ ID NO: 53 or 54 and comprises an L234A/L235A mutation, a D265A mutation, and a P329A mutation.

As understood by those skilled in the art, to facilitate the formation of the bispecific antibody of the present disclosure as a heterodimer, the Fc region contained in the bispecific antibody of the present disclosure may comprise a mutation(s) that favors the heterodimerization. In one embodiment, mutations are introduced into the CH3 regions of the two Fc regions.

Methods for facilitating the heterodimerization of the Fc regions are known in the art. For example, the CH3 region of the first Fc region and the CH3 region of the second Fc region are engineered in a complementary manner, such that each CH3 region (or the heavy chain comprising the same) can be no longer homodimerized with itself but forced to be heterodimerized with other CH3 regions that are complementarily engineered (such that heterodimerization occurs between the first and second CH3 regions and no homodimers are formed between the two first CH3 regions or the two second CH3 regions).

Preferably, based on the knob-in-hole technique, a corresponding knob mutation(s) and a corresponding hole mutation(s) are introduced into the first monomeric Fc region and the second monomeric Fc region, respectively. For this technique, see, e.g., Merchant, A. M., et al., (1998). "An efficient route to human bispecific IgG." Nat Biotechnol 16(7): 677-681.

In a particular embodiment, in the CH3 region of one Fc region, the threonine residue at position 366 is substituted with a tryptophan residue (T366W) (knob mutation); while in the CH3 region of the other Fc region, the tyrosine residue at position 407 is substituted with a valine residue (Y407V) (hole mutation). Optionally, the threonine residue at position 366 is substituted with a serine residue (T366S), and the leucine residue at position 368 is substituted with an alanine residue (L368A) (numbering according to the EU index).

In yet another embodiment, in the CH3 region of one Fc region, the knob mutations comprise or consist of: a substitution of the threonine residue at position 366 with a tryptophan residue (T366W) and a substitution of the serine residue at position 354 with a cysteine residue (S354C) or a substitution of the glutamic acid residue at position 356 with a cysteine residue (E356C) (in particular, a substitution of the serine residue at position 354 with a cysteine residue); while in the CH3 region of the other Fc region, the hole mutations comprise or consist of: a substitution of the tyrosine residue at position 407 with a valine residue (Y407V), optionally a substitution of the threonine residue at position 366 with a serine residue (T366S) and a substitution of the leucine residue at position 368 with an alanine residue (L368A) (numbering according to the EU index), and optionally a substitution of the tyrosine residue at position 349 with a cysteine residue (Y349C) (numbering according to the EU index).

In one specific embodiment, one Fc region comprises amino acid substitutions S354C and T366W (knob mutations), and the other Fc region comprises amino acid substitutions Y349C, T366S, L368A, and Y407V (hole mutations) (numbering according to the EU index).

Thus, in one specific embodiment, the two Fc regions contained in the bispecific antibody of the present disclosure are heterodimerized, wherein
a) one Fc-region polypeptide comprises a mutation T366W, while the other Fc-region polypeptide comprises mutations T366S, L368A, and Y407V, or
b) one Fc-region polypeptide comprises mutations T366W and Y349C, while the other Fc-region polypeptide comprises mutations T366S, L368A, Y407V, and S354C, or
c) one Fc-region polypeptide comprises mutations T366W and S354C, while the other Fc-region polypeptide comprises mutations T366S, L368A, Y407V, and Y349C;
optionally, the Fc region further comprises a mutation(s) that reduces binding to an Fcy receptor, e.g., one or more of an L234A/L235A mutation, a D265A mutation, or a P329A mutation, for example, an L234A/L235A mutation, a D265A mutation, and a P329A mutation.

In some embodiments, the Fc region further comprises additional mutations that favor the purification of the heterodimer.

In one specific embodiment, the two Fc regions of the bispecific antibody of the present disclosure are heterodimerized, wherein
the first Fc region comprises a knob mutation, and comprises or consists of an amino acid sequence set forth in SEQ ID NO: 49, 50, or 73, or an amino acid sequence having at least 90% identity, e.g., 95%, 96%, 97%, 99%, or higher identity thereto; in some embodiments, the Fc region comprises an amino acid sequence having at least 90% identity, e.g., 95%, 96%, 97%, 99%, or higher identity to SEQ ID NO: 49, 50, or 73 and comprises a knob mutation(s) (e.g., S354C and T366W); in some embodiments, the Fc region comprises or does not comprise a hinge region EPKSS or EPKSC;
the second Fc region comprises a hole mutation, and comprises or consists of an amino acid sequence set forth in SEQ ID NO: 47, 48, or 74, or an amino acid sequence having at least 90% identity, e.g., 95%, 96%, 97%, 99%, or higher identity thereto. In some embodiments, the Fc region comprises an amino acid sequence having at least 90% identity, e.g., 95%, 96%, 97%, 99%, or higher identity to SEQ ID NO: 47, 48, or 74 and comprises a hole mutation(s) (T366S, L368A, Y407V, and Y349C); in some embodiments, the Fc region comprises or does not comprise a hinge region EPKSS or EPKSC.

### ➢ Exemplary bispecific antibody molecule

In some preferred embodiments, the present disclosure provides a bispecific antibody comprising a VHH specifically binding to LILRB4 and an antigen-binding region specifically binding to CD3, and optionally an Fc region.

In some embodiments, the present disclosure provides a bispecific antibody comprising a first antigen-binding region, a second antigen-binding region, and an Fc dimer, wherein the first antigen-binding region is a Fab fragment or an scFv specifically binding to CD3, and the second antigen-binding region is a VHH specifically binding to LILRB4.

In some embodiments, the bispecific antibody of the present disclosure comprises one first antigen-binding region and one second antigen-binding region. In some embodiments, the bispecific antibody of the present disclosure comprises one first antigen-binding region and two second antigen-binding regions. In some embodiments, the two second antigen-binding regions are the same or different; for example, two different antigen-binding regions bind to different antigenic epitopes or have different sequences.

In some embodiments, the present disclosure relates to a bispecific antibody which is an IgG-like bispecific antibody comprising at least one anti-LILRB4 VHH, one Fab fragment of an anti-CD3 antibody, and an Fc heterodimer. In some embodiments, the bispecific antibody comprises one anti-LILRB4 VHH. In some embodiments, the bispecific antibody comprises 2 anti-LILRB4 VHHs. In some embodiments, the two VHHs are the same or different, e.g., two different VHHs bind to different anti-LILRB4 epitopes or have different sequences.

In some embodiments, in the bispecific antibody,
the Fab comprises a VH-CH1 and a VL-CL, and the VHH comprises a heavy chain variable region,
wherein the C-terminus of the CH1 of the Fab is fused to a CH2 or a hinge region of a first Fc region (e.g., comprising a knob mutation(s)) to form a heavy chain, and
said one VHH is fused to a second Fc region (e.g., comprising a hole mutation(s)) (e.g., the C-terminus of the VHH is fused to a CH2 or a hinge region of the second Fc region) to form the other heavy chain;
   and
the VL-CL of the Fab constitutes a light chain;
optionally, the additional VHH (e.g., the other VHH) is fused to the N-terminus of the VH of the Fab fragment.

In some embodiments, the present disclosure relates to a bispecific antibody which is an IgG-like bispecific antibody comprising at least one anti-LILRB4 VHH, one scFv of an anti-CD3 antibody, and an Fc heterodimer. In some embodiments, the bispecific antibody comprises one anti-LILRB4 VHH. In some embodiments, the bispecific antibody comprises 2 anti-LILRB4 VHHs. In some embodiments, the two VHHs are the same or different, e.g., two different VHHs bind to different anti-LILRB4 epitopes or have different sequences.

In some embodiments, in the bispecific antibody,
the VHH comprises or consists of a heavy chain variable region, and the scFv comprises a VH and a VL,
wherein the C-terminus of the scFv is fused to a CH2 or a hinge region of a first Fc region (e.g., comprising a knob mutation(s)), and
said one VHH is fused to a second Fc region (e.g., comprising a hole mutation(s)) (e.g., the C-terminus of the VHH is fused to a CH2 or a hinge region of the second Fc region);
optionally, the additional VHH (e.g., the other VHH) is fused to the N-terminus of the scFv.

In some embodiments, the scFv comprises, from N-terminus to C-terminus, a VH and a VL in sequence.

In some embodiments, the fusion is direct fusion or fusion via a linker or a linker peptide. In some embodiments, the VH and the VL in the scFv are linked via a linker or a linker peptide. In some embodiments, the linker or the linker peptide is selected from (GGGGS)n, wherein n =1, 2, 3, or 4. In some embodiments, the linker or the linker peptide is GGGGS. In one embodiment, the linker (e.g., a linker comprised in an scFv) comprises or consists of the amino acid sequence set forth in SEQ ID NO: 70. In some embodiments, the Fc region comprises or does not comprise a hinge region. In some embodiments, the hinge region is selected from EPKSS (SEQ ID NO: 59) and EPKSC (SEQ ID NO: 67).

Thus, in some embodiments, the present disclosure relates to a bispecific antibody which is an IgG-like bispecific antibody comprising one anti-LILRB4 VHH, one Fab fragment of an anti-CD3 antibody, and an Fc heterodimer, wherein
the Fab fragment comprises a VH-CH1 and a VL-CL, and the VHH comprises a heavy chain variable region,
wherein the C-terminus of the CH1 of the Fab fragment is fused to a CH2 or a hinge region of a first Fc region (e.g., comprising a knob mutation(s)) to form a first heavy chain;
the VHH is fused to a second Fc region (e.g., comprising a hole mutation(s)) to form a second heavy chain (e.g., the C-terminus of the VHH is fused to a CH2 or a hinge region of the second Fc region); and
the VL-CL of the Fab fragment constitutes a light chain.

In one embodiment, the present disclosure relates to a bispecific antibody which is an IgG-like bispecific antibody comprising one anti-LILRB4 VHH, one Fab fragment of an anti-CD3 antibody, and an Fc heterodimer, wherein
the Fab fragment comprises a VH-CH1 and a VL-CL, and the VHH comprises a heavy chain variable region,
wherein the C-terminus of the CH1 of the Fab fragment is fused to a CH2 or a hinge region of a first Fc region comprising a knob mutation(s) to form a first heavy chain;
the C-terminus of the VHH is fused to a second Fc region comprising a hole mutation(s) to form a second heavy chain;
   and
the VL-CL of the Fab fragment constitutes a light chain.

In some embodiments, the first heavy chain comprises or consists of the amino acid sequence set forth in SEQ ID NO: 44, or an amino acid sequence having at least 85%, 90%, 91%, 92%, 93%, 94%, 95%, 96%, 97%, 98%, or 99% identity thereto;
the second heavy chain comprises or consists of the amino acid sequence set forth in SEQ ID NO: 45, or an amino acid sequence having at least 85%, 90%, 91%, 92%, 93%, 94%, 95%, 96%, 97%, 98%, or 99% identity thereto; and/or
the light chain comprises or consists of the amino acid sequence set forth in SEQ ID NO: 2, or an amino acid sequence having at least 85%, 90%, 91%, 92%, 93%, 94%, 95%, 96%, 97%, 98%, or 99% identity thereto.

Thus, in some embodiments, the present disclosure relates to a bispecific antibody which is an IgG-like bispecific antibody comprising two anti-LILRB4 VHH, one Fab fragment of an anti-CD3 antibody, and an Fc heterodimer, wherein
the Fab fragment comprises a VH-CH1 and a VL-CL, and the VHH comprises a heavy chain variable region,
wherein the C-terminus of a first VHH is fused to the N-terminus of the VH of the Fab fragment, and the C-terminus of the CH1 of the Fab fragment is fused to a CH2 or a hinge region of an Fc region (e.g., comprising a knob mutation(s)) to form a first heavy chain;
a second VHH is fused to an Fc region (e.g., comprising a hole mutation(s)) to form a second heavy chain (e.g., the C-terminus of the VHH is fused to a CH2 or a hinge region of a second Fc region); and
the VL-CL of the Fab fragment constitutes a light chain.

In one embodiment, the present disclosure relates to a bispecific antibody which is an IgG-like bispecific antibody comprising two anti-LILRB4 VHH, one Fab fragment of an anti-CD3 antibody, and an Fc heterodimer, wherein
the Fab fragment comprises a VH-CH1 and a VL-CL, and the VHH comprises a heavy chain variable region,
wherein the C-terminus of a first VHH is fused to the N-terminus of the VH of the Fab fragment, and the C-terminus of the CH1 of the Fab fragment is fused to a CH2 or a hinge region of an Fc region comprising a knob mutation(s) to form a first heavy chain;
the C-terminus of a second VHH is fused to an Fc region comprising a hole mutation(s) to form a second heavy chain;
   and
the VL-CL of the Fab fragment constitutes a light chain.

In some embodiments, the first VHH and the second VHH may be the same. In some embodiments, the first VHH and the second VHH may be different, for example, the first VHH and the second VHH may be different humanized antibodies of the same camelid-derived VHH antibody, or the first VHH and the second VHH may be different humanized antibodies derived from different camelid-derived antibodies.

In some embodiments, the first heavy chain comprises or consists of the amino acid sequence set forth in SEQ ID NO: 46, or an amino acid sequence having at least 85%, 90%, 91%, 92%, 93%, 94%, 95%, 96%, 97%, 98%, or 99% identity thereto;
the second heavy chain comprises or consists of the amino acid sequence set forth in SEQ ID NO: 45, or an amino acid sequence having at least 85%, 90%, 91%, 92%, 93%, 94%, 95%, 96%, 97%, 98%, or 99% identity thereto; and/or
the light chain comprises or consists of the amino acid sequence set forth in SEQ ID NO: 2, or an amino acid sequence having at least 85%, 90%, 91%, 92%, 93%, 94%, 95%, 96%, 97%, 98%, or 99% identity thereto.

Thus, in some embodiments, the present disclosure relates to a bispecific antibody which is an IgG-like bispecific antibody comprising two anti-LILRB4 VHH, one scFv of an anti-CD3 antibody, and an Fc heterodimer, wherein
the scFv comprises, from N-terminus to C-terminus, a VH and a VL in sequence (wherein the VH and the VL are linked via a linker or a linker peptide; for example, the linker or the linker peptide comprises or consists of the amino acid sequence set forth in SEQ ID NO: 70), and the VHH comprises a heavy chain variable region,
wherein the C-terminus of a first VHH is fused to the N-terminus of the VH of the scFv, and the C-terminus of the VL of the scFv is fused to a CH2 or a hinge region of an Fc region (e.g., comprising a knob mutation(s)) to form a first heavy chain; and
a second VHH is fused to an Fc region (e.g., comprising a hole mutation(s)) to form a second heavy chain (e.g., the C-terminus of the VHH is fused to a CH2 or a hinge region of a second Fc region).

In one embodiment, the present disclosure relates to a bispecific antibody which is an IgG-like bispecific antibody comprising two anti-LILRB4 VHH, one scFv of an anti-CD3 antibody, and an Fc heterodimer, wherein
the scFv comprises, from N-terminus to C-terminus, a VH and a VL in sequence (wherein the VH and the VL are linked via a linker or a linker peptide; for example, the linker or the linker peptide comprises or consists of the amino acid sequence set forth in SEQ ID NO: 70), and the VHH comprises a heavy chain variable region,
wherein the C-terminus of a first VHH is fused to the N-terminus of the VH of the scFv, and the C-terminus of the VL of the scFv is fused to a CH2 or a hinge region of an Fc region comprising a knob mutation(s) to form a first heavy chain; and
the C-terminus of a second VHH is fused to an Fc region comprising a hole mutation(s) to form a second heavy chain.

In some embodiments, the first VHH and the second VHH may be the same. In some embodiments, the first VHH and the second VHH may be different, for example, the first VHH and the second VHH may be different humanized antibodies of the same camelid-derived VHH antibody, or the first VHH and the second VHH may be different humanized antibodies derived from different camelid-derived antibodies.

In some embodiments, the first heavy chain comprises or consists of the amino acid sequence set forth in SEQ ID NO: 72, or an amino acid sequence having at least 85%, 90%, 91%, 92%, 93%, 94%, 95%, 96%, 97%, 98%, or 99% identity thereto; and/or
the second heavy chain comprises or consists of the amino acid sequence set forth in SEQ ID NO: 45, or an amino acid sequence having at least 85%, 90%, 91%, 92%, 93%, 94%, 95%, 96%, 97%, 98%, or 99% identity thereto.

### V. Nucleic Acid Encoding Antibody and Host Cell Comprising the Same

In one aspect, the present disclosure provides a nucleic acid encoding any of the anti-LILRB4 antibodies or the antigen-binding fragments thereof or the multispecific antibodies such as the bispecific antibodies or any one of chains thereof above.

For example, the nucleic acid of the present disclosure comprises a nucleic acid encoding an amino acid sequence selected from any one of SEQ ID NOs: 11, 15, 16, 20, 21, 24, 25, 29, 30, 31, 32, 35, 36, 38, 39, 41, 42, 43, 44, 45, 46, and 72, or a nucleic acid encoding an amino acid sequence having at least 85%, 90%, 91%, 92%, 93%, 94%, 95%, 96%, 97%, 98%, or 99% identity to an amino acid sequence selected from any one of SEQ ID NOs: 11, 15, 16, 20, 21, 24, 25, 29, 30, 31, 32, 35, 36, 38, 39, 41, 42, 43, 44, 45, 46, and 72.

As will be appreciated by those skilled in the art, each antibody or polypeptide amino acid sequence may be encoded by a variety of nucleic acid sequences because of codon degeneracy. Nucleic acid sequences encoding the molecule of the present disclosure may be generated using methods well known in the art, e.g., *de novo* solid-phase DNA synthesis, or PCR amplification.

In one aspect, the present disclosure provides a nucleic acid encoding any of the above antibodies or antigen-binding fragments thereof or any of the above antibody chains. When expressed in a suitable expression vector, a polypeptide encoded by the nucleic acid is capable of exhibiting a binding capacity to human and/or monkey (e.g., cynomolgus monkey) LILRB4 antigens. In one embodiment, the nucleic acids encoding the chains of the antibody may be in the same vector or in different vectors. In yet another embodiment, the nucleic acids encoding the chains of the antibody may be introduced into the same or different host cells for expression. Thus, in some embodiments, the method for producing the antibody of the present disclosure comprises a step of: culturing a host cell comprising nucleic acids encoding the chains of the molecule under a condition suitable for expressing the chains to produce the antibody of the present disclosure.

In yet another aspect, the present disclosure provides a nucleic acid encoding any of the above bispecific antibodies. When expressed in a suitable expression vector, a polypeptide encoded by the nucleic acid is capable of exhibiting a binding capacity to human or monkey (e.g., cynomolgus monkey) CD3 and human or monkey (e.g., cynomolgus monkey) LILRB4 antigens. In one embodiment, the nucleic acids encoding the chains of the bispecific antibody may be in the same vector or in different vectors. In yet another embodiment, the nucleic acids encoding the chains of the bispecific antibody may be introduced into the same or different host cells for expression. Thus, in some embodiments, the method for producing the bispecific antibody of the present disclosure comprises a step of: culturing a host cell comprising nucleic acids encoding the chains of the molecule under a condition suitable for expressing the chains to produce the bispecific antibody of the present disclosure.

In one embodiment, provided is a vector comprising the nucleic acid. In one embodiment, the vector is an expression vector, e.g., a eukaryotic expression vector. The vector includes, but is not limited to, a virus, a plasmid, a cosmid, a λ phage, or a yeast artificial chromosome (YAC). In one embodiment, the vector is, for example, a pcDNA vector, such as pcDNA3.1.

In one embodiment, provided is a host cell comprising the nucleic acid or the vector, e.g., for cloning or expressing a vector encoding the anti-LILRB4 antibody or the antigen-binding fragment thereof or the bispecific antibody. In one embodiment, the host cell is eukaryotic. In another embodiment, the host cell is selected from a yeast cell, a mammalian cell (e.g., a CHO cell (e.g., CHO-S, such as ExpiCHO-S) or 293 cell (e.g., 293F or HEK293 cell)), and other cells suitable for preparing an antibody or a fragment thereof. In one embodiment, the host cell is prokaryotic, e.g., a bacterium, such as *Escherichia coli.*

In one embodiment, the host cell is eukaryotic. In another embodiment, the host cell is selected from a yeast cell, a mammalian cell, and other cells suitable for preparing an antibody or a fragment thereof. For example, eukaryotic microorganisms, such as filamentous fungi or yeast, are suitable cloning or expression hosts for the vector encoding the antibody. For example, fungus and yeast strains in which a glycosylation pathway has been "humanized" produce antibodies having a partial or full human glycosylation pattern. Host cells suitable for expressing a glycosylated antibody are also derived from multicellular organisms (invertebrates and vertebrates). Vertebrate cells may also be used as hosts. For example, a mammalian cell line engineered to be suitable for suspension growth may be used. Other examples of useful mammalian host cell lines are monkey kidney CV1 line (COS-7) transformed with SV40, human embryonic kidney line (HEK293, 293F, or 293T cells), and the like. Other useful mammalian host cell lines include Chinese hamster ovary (CHO) cells, including DHFR-CHO cells, CHO-S cells, ExpiCHO, and the like; and myeloma cell lines such as Y0, NS0, and Sp2/0. Mammalian host cell lines suitable for producing antibodies are known in the art.

### VI. Production and Purification of Anti-LILRB4 Antibody or Antigen-Binding Fragment Thereof or Bispecific Antibody of the Present Disclosure

In one embodiment, provided is a method for preparing the anti-LILRB4 antibody or the antigen-binding fragment thereof or the multispecific antibody such as the bispecific antibody of the present disclosure, wherein the method comprises culturing a host cell comprising a nucleic acid encoding the anti-LILRB4 antibody or the antigen-binding fragment thereof or the multispecific antibody such as the bispecific antibody (e.g., any one and/or more polypeptide chains) or an expression vector comprising the nucleic acid, as provided above, under a condition suitable for expressing the anti-LILRB4 antibody or the antigen-binding fragment thereof or the multispecific antibody such as the bispecific antibody or chains thereof, and optionally recovering the anti-LILRB4 antibody or the antigen-binding fragment thereof or the multispecific antibody such as the bispecific antibody from the host cell (or a host cell culture medium).

Polynucleotides encoding the polypeptide chains of the anti-LILRB4 antibody or the antigen-binding fragment thereof or the multispecific antibody such as the bispecific antibody of the present disclosure may be inserted into one or more vectors for further cloning and/or expression in host cells. Methods known to those skilled in the art can be used to construct expression vectors. Once the expression vector comprising one or more nucleic acid molecules of the present disclosure has been prepared for expression, the expression vector may be transfected or introduced into suitable host cells. Various techniques may be used for this purpose, for example, protoplast fusion, calcium phosphate precipitation, electroporation, retroviral transduction, viral transfection, biolistics, liposome-based transfection, or other conventional techniques.

The anti-LILRB4 antibody or the antigen-binding fragment thereof or the multispecific antibody such as the bispecific antibody prepared as described herein can be purified by known techniques such as high-performance liquid chromatography, ion exchange chromatography, gel electrophoresis, affinity chromatography, and size exclusion chromatography. The actual conditions used for purifying a particular protein also depend on factors such as net charge, hydrophobicity, and hydrophilicity, and these will be apparent to those skilled in the art.

The purity of the molecule of the present disclosure may be determined by any one of a variety of well-known analytical methods including size exclusion chromatography, gel electrophoresis, high-performance liquid chromatography, and the like.

### VII. Assay Method for Anti-LILRB4 Antibody or Antigen-Binding Fragment Thereof or Multispecific Antibody

The anti-LILRB4 antibody or the antigen-binding fragment thereof or the multispecific antibody such as the bispecific antibody provided herein may be identified, screened, or characterized for physical/chemical properties and/or biological activity thereof through a variety of assay methods known in the art.

In one aspect, the anti-LILRB4 antibody or the antigen-binding fragment thereof or the multispecific antibody such as the bispecific antibody of the present disclosure are determined for their binding activity to a target (e.g., an antigen, e.g., a free antigen or an antigen expressed on a cell), for example, by known methods such as biolayer interferometry, ELISA, and flow cytometry. Binding to CD3 and/or LILRB4 (or CD3 and/or LILRB4 expressed on a cell) can be determined using methods known in the art, and exemplary methods are disclosed herein. In some embodiments, the binding is determined by radioimmunoassay (RIA), biolayer interferometry (BLI), electrochemiluminescence (ECL), surface plasmon resonance (SPR), or flow cytometry (FACS).

The present disclosure further provides an assay method for identifying the biological activity of the anti-LILRB4 antibody or the antigen-binding fragment thereof or the multispecific antibody such as the bispecific antibody. The biological activity is selected from the properties of the anti-LILRB4 antibody or the antigen-binding fragment thereof or the multispecific antibody such as the bispecific antibody of the present disclosure.

For example, the binding activity of the antibody molecule of the present disclosure to a cell expressing CD3 and/or LILRB4 may be determined by methods known in the art, such as fluorescent reporter molecule assay and flow cytometry, or by the exemplary methods disclosed in the examples herein. For example, the binding of the antibody molecule of the present disclosure to CD3 and/or LILRB4 expressed on a cell is determined by the methods shown in Example 3, 4, or 5.

For example, the activation activity of the antibody molecule of the present disclosure on T cells or the activation of T cell proliferation may be determined by methods known in the art, such as a T cell activation assay system, e.g., a Jurkat/NFAT-luc (luciferase) reporter gene system or a primary T cell-dependent cytotoxicity assay system, e.g., by the methods shown in Example 4, 6 or 9, by detecting the CD3 signaling pathway in T cells or by detecting the release of cytokines (e.g., interleukins such as IL2; interferons such as IFNy; tumor necrosis factors such as TNFα) following T cell activation. For example, the inhibitory activity or structural safety of the antibody molecule of the present disclosure against a tumor may be determined by methods known in the art, such as a tumor inhibition experiment conducted on a mouse tumor model.

Cells for use in any of the above *in vitro* assay methods are primary cells or cell lines, including cells that naturally express or overexpress CD3 (e.g., human or monkey (e.g., cynomolgus monkey)) or LILRB4 (e.g., human or monkey (e.g., cynomolgus monkey) LILRB4), or cells engineered to express CD3 or LILRB4, e.g., CHO cells or CHO-K1 cells, MV-411, HL-60, or MOLM-13.

It will be understood that any of the assay methods described above may be performed using a combination of the antibody of the present disclosure and other active agents.

### VIII. Immunoconjugate, Pharmaceutical Composition, Pharmaceutical Combination Product, and Kit of Anti-LILRB4 Antibody or Bispecific Antibody of the Present Disclosure

In some embodiments, the present disclosure provides an immunoconjugate comprising any of the anti-LILRB4 antibodies or the antigen-binding fragments thereof or the multispecific antibodies such as the bispecific antibodies described herein. Preferably, the immunoconjugate comprises one or more additional therapeutic agents (e.g., cytotoxins or small molecule compounds) or markers.

In some embodiments, the present disclosure provides a composition or a medicament or a formulation comprising any of the anti-LILRB4 antibodies or the antigen-binding fragments thereof or the multispecific antibodies such as the bispecific antibodies described herein, wherein preferably, the composition is a pharmaceutical composition.

In one embodiment, the composition further comprises a pharmaceutical supplementary material. In one embodiment, the composition, e.g., the pharmaceutical composition, comprises the anti-LILRB4 antibody or the antigen-binding fragment thereof or the multispecific antibody such as the bispecific antibody of the present disclosure, and a combination of one or more additional therapeutic agents.

The composition or the medicament or the formulation of the present disclosure may further comprise a suitable pharmaceutical supplementary material, such as a pharmaceutical carrier and a pharmaceutical excipient known in the art, including buffers.

As used herein, "pharmaceutical carrier" includes any and all solvents, dispersion media, isotonic agents, absorption delaying agents, and the like that are physiologically compatible.

For use and application of the pharmaceutical supplementary materials, see Handbook of Pharmaceutical Excipients, 8th ed., R. C. Rowe, P. J. Seskey and S. C. Owen, Pharmaceutical Press, London, Chicago.

The composition or the medicament or the formulation of the present disclosure may be in a variety of forms. These forms include, for example, liquid, semi-solid, and solid dosage forms, such as liquid solutions (e.g., injectable solutions and eye drops), pulvis or suspensions, liposomes, and suppositories. The preferred form depends on the intended mode of administration and therapeutic use.

The medicament or formulation, e.g., in the form of a lyophilized formulation or an aqueous solution, comprising the anti-LILRB4 antibody or the antigen-binding fragment thereof or the multispecific antibody such as the bispecific antibody described herein can be prepared by mixing the anti-LILRB4 antibody or the antigen-binding fragment thereof or the multispecific antibody such as the bispecific antibody of the present disclosure having the desired purity with one or more optional pharmaceutical supplementary materials.

The composition or the medicament or the formulation of the present disclosure may further comprise more than one active ingredient required by a particular indication treated, preferably those having complementarity activity without adversely affecting one another. For example, it is desirable to further provide additional therapeutic agents.

The present disclosure further provides a pharmaceutical combination or a pharmaceutical combination product comprising the anti-LILRB4 antibody or the antigen-binding fragment thereof or the multispecific antibody such as the bispecific antibody of the present disclosure, and one or more additional therapeutic agents.

The present disclosure further provides a kit of parts, comprising the pharmaceutical combination, wherein, for example, the kit of parts comprises in the same package:
- a first container containing a pharmaceutical composition comprising the anti-LILRB4 antibody or the antigen-binding fragment thereof or the multispecific antibody such as the bispecific antibody of the present disclosure;
- a second container containing a pharmaceutical composition comprising an additional therapeutic agent.

In some embodiments, the additional therapeutic agent is, for example, a chemotherapeutic agent, a cytokine, a cytotoxic agent, an additional antibody, a small molecule drug, or an immunomodulatory agent (e.g., an immunosuppressive agent).

### IX. Use of Anti-LILRB4 Antibody or Antigen-Binding Fragment Thereof or Multispecific Antibody and Method for Using Anti-LILRB4 Antibody or Antigen-Binding Fragment Thereof or Multispecific Antibody

In one aspect, the present disclosure provides a method for preventing or treating a disease in a subject, the method comprising administering to the subject the anti-LILRB4 antibody or the antigen-binding fragment thereof or the multispecific antibody such as the bispecific antibody, or the immunoconjugate or the composition or the medicament or the formulation comprising the same of the present disclosure. In some embodiments, the present disclosure provides a method for specifically activating a T cell in a subject, the method comprising administering to the subject the anti-LILRB4 antibody or the antigen-binding fragment thereof or the multispecific antibody such as the bispecific antibody, or the immunoconjugate or the composition or the medicament or the formulation comprising the same of the present disclosure.

In some embodiments, the disease is, for example, a tumor, e.g., a cancer. The cancer may be at an early, intermediate, or advanced stage, or may be a metastatic cancer. In some embodiments, the cancer may be a solid tumor or a hematological tumor. In some embodiments, the cancer is a hematological tumor, such as a solid tumor, multiple myeloma, or leukemia, e.g., acute myeloid leukemia (AML), e.g., monocytic AML or non-monocytic AML, e.g., a human myelomonocytic leukemia cell, human acute promyelocytic leukemia, or human acute myeloid leukemia.

In some embodiments, the treatment of the disease will benefit from activation of a CD3 signaling pathway and/or activation of T cells.

In some embodiments, the cancer is a cancer characterized by having an LILRB4 protein level and/or nucleic acid level that are present (e.g., having the LILRB4 protein expression or containing the nucleic acid, as compared to the same tissue of a healthy individual without the LILRB4 protein or nucleic acid or the adjacent healthy tissue of the patient) or elevated (e.g., elevated expression, e.g., as compared to the LILRB4 protein level and/or nucleic acid level in the same tissue of a healthy individual or as compared to the LILRB4 protein level and/or nucleic acid level in the adjacent healthy tissue of the patient) in the patient (e.g., in the patient's cancer tissue); for example, tumor cells of the cancer have an LILRB4 protein level and/or nucleic acid level that are present (e.g., having the LILRB4 protein expression or containing the nucleic acid, as compared to the cells of the same tissue of a healthy individual without the LILRB4 protein or nucleic acid or the cells of the adjacent healthy tissue of the patient) or elevated (e.g., elevated expression, e.g., as compared to the LILRB4 protein level and/or nucleic acid level in the cells of the same tissue of a healthy individual or as compared to the LILRB4 protein level and/or nucleic acid level in the healthy cells of the same tissue of the patient or the cells of the adjacent healthy tissue of the patient).

The anti-LILRB4 antibody or the antigen-binding fragment thereof or the multispecific antibody such as the bispecific antibody of the present disclosure (as well as the immunoconjugate, the composition, the pharmaceutical composition, the formulation, the combination product or the like comprising the same) can be administered by any suitable method, including parenteral administration and, if required by local treatment, intralesional administration. Parenteral injection or infusion includes intramuscular, intravenous, intra-arterial, intraperitoneal, or subcutaneous injection or infusion. The administration may be performed by any suitable route, such as injection, e.g., intravenous or subcutaneous injection, to some extent depending on short-term or long-term treatment. Various administration schedules are encompassed herein, including, but not limited to, single administration or multiple administrations at multiple time points, bolus injection, and pulse infusion.

In order to prevent or treat a disease, the appropriate dose of the anti-LILRB4 antibody or the antigen-binding fragment thereof or the multispecific antibody such as the bispecific antibody (as well as the immunoconjugate, the composition, the pharmaceutical composition, the formulation, the combination product or the like comprising the same) of the present disclosure (when used alone or in combination with one or more additional therapeutic agents) will depend on the type of the disease to be treated, the type of the antibody, the severity and progression of the disease, purpose of administration (prophylactic or therapeutic), previous therapies, clinical histories of patients, responses to the antibody, and the discretion of an attending physician. The molecule is suitably administered to a patient through a single treatment or through a series of treatments. In other aspects, the present disclosure provides use of the anti-LILRB4 antibody or the antigen-binding fragment thereof or the multispecific antibody such as the bispecific antibody, or the immunoconjugate, the composition, or the combination product comprising the same of the present disclosure in producing or preparing a medicament for the use described herein, e.g., for use in the prevention or treatment of the related disease or disorder mentioned herein.

In some embodiments, the anti-LILRB4 antibody or the antigen-binding fragment thereof or the multispecific antibody such as the bispecific antibody (as well as the immunoconjugate, the composition, the pharmaceutical composition, the formulation or the like comprising the same) may also be administered in combination with one or more additional therapies, e.g., therapeutic modalities and/or additional therapeutic agents, for the use described herein, e.g., for use in the prevention and/or treatment of the related disease or disorder mentioned herein.

In some embodiments, the therapeutic modality is, for example, radiotherapy.

In some embodiments, the additional therapeutic agent is, for example, a chemotherapeutic agent, a cytokine, a cytotoxic agent, an additional antibody, a small molecule drug, or an immunomodulatory agent (e.g., an immunosuppressive agent).

### X. Diagnosis and Detection

In one aspect, the present disclosure further relates to a method for diagnosing and detecting (e.g., for diagnostic or non-diagnostic purposes) using the anti-LILRB4 antibody or the antigen-binding fragment thereof or the multispecific antibody such as the bispecific antibody, and a composition comprising the anti-LILRB4 antibody or the antigen-binding fragment thereof or the multispecific antibody such as the bispecific antibody for diagnosing and detecting.

In certain embodiments, the anti-LILRB4 antibody or the antigen-binding fragment thereof or the multispecific antibody such as the bispecific antibody provided herein can be used to detect the presence of LILRB4 in a biological sample. In certain embodiments, the bispecific antibody provided herein can be used to detect the presence of CD3 and/or LILRB4 in a biological sample.

The term "detection" used herein includes quantitative and qualitative detections, and exemplary detection methods may involve immunohistochemistry, immunocytochemistry, flow cytometry (e.g., FACS), magnetic beads complexed with antibody molecules, ELISA, and PCR-techniques (e.g., RT-PCR). In certain embodiments, the biological sample is a body fluid, such as bone marrow, blood, serum, or plasma.

In certain embodiments, the method comprises contacting a biological sample with the anti-LILRB4 antibody or the antigen-binding fragment thereof or the multispecific antibody such as the bispecific antibody described herein under a condition that allows the antibody to bind to LILRB4, and detecting whether a complex is formed by the anti-LILRB4 antibody or the antigen-binding fragment thereof or the multispecific antibody such as the bispecific antibody and LILRB4. Formation of the complex indicates the presence of LILRB4. The method may be an *in vitro* or *in vivo* method.

In certain embodiments, provided is a labeled anti-LILRB4 antibody or an antigen-binding fragment thereof or a multispecific antibody such as a bispecific antibody. The label includes, but is not limited to, a label or moiety that is detected directly (such as a fluorescent label, a chromophoric label, an electron-dense label, a chemiluminescent label, and a radioactive label), and a moiety that is detected indirectly, such as an enzyme or a ligand, for example, by enzymatic reaction or molecular interaction. In some embodiments, the label is a marker such as biotin or hFc.

In some embodiments provided herein, the sample is obtained prior to treatment with the anti-LILRB4 antibody or the antigen-binding fragment thereof or the multispecific antibody such as the bispecific antibody of the present disclosure. In some embodiments, the sample is obtained prior to application of additional therapies. In some embodiments, the sample is obtained during treatment with additional therapies, or after treatment with additional therapies.

In some embodiments, LILRB4 is detected prior to treatment, e.g., prior to an initial treatment or prior to a certain treatment after a treatment interval.

### XI. Detailed Description

In some aspects, the present disclosure relates to the following specific embodiments:
1. A VHH antibody specifically binding to LILRB4, comprising
   three complementarity determining regions (CDRs) contained in a VHH set forth in any one of SEQ ID NOs: 11, 16, 21, 25, 30, 32, 36, 39, and 42;
   preferably, sequences of the CDRs are defined according to IMGT.
2. The VHH antibody according to embodiment 1, comprising complementarity determining regions (CDRs) VHH CDR1, VHH CDR2, and VHH CDR3, wherein
   (i) the VHH CDR1 comprises or consists of the amino acid sequence set forth in SEQ ID NO: 12, the VHH CDR2 comprises or consists of the amino acid sequence set forth in SEQ ID NO: 13, and the VHH CDR3 comprises or consists of the amino acid sequence set forth in SEQ ID NO: 14; or
   (ii) the VHH CDR1 comprises or consists of the amino acid sequence set forth in SEQ ID NO: 17, the VHH CDR2 comprises or consists of the amino acid sequence set forth in SEQ ID NO: 18, and the VHH CDR3 comprises or consists of the amino acid sequence set forth in SEQ ID NO: 19;
   (iii) the VHH CDR1 comprises or consists of the amino acid sequence set forth in SEQ ID NO: 75, the VHH CDR2 comprises or consists of the amino acid sequence set forth in SEQ ID NO: 22, and the VHH CDR3 comprises or consists of the amino acid sequence set forth in SEQ ID NO: 23;
   (iv) the VHH CDR1 comprises or consists of the amino acid sequence set forth in SEQ ID NO: 26, the VHH CDR2 comprises or consists of the amino acid sequence set forth in SEQ ID NO: 27, and the VHH CDR3 comprises or consists of the amino acid sequence set forth in SEQ ID NO: 28;
   (v) the VHH CDR1 comprises or consists of the amino acid sequence set forth in SEQ ID NO: 17, the VHH CDR2 comprises or consists of an amino acid sequence set forth in SEQ ID NO: 33 or 37, and the VHH CDR3 comprises or consists of an amino acid sequence set forth in SEQ ID NO: 34 or 40;
   (vi) the VHH CDR1 comprises or consists of the amino acid sequence set forth in SEQ ID NO: 17, the VHH CDR2 comprises or consists of an amino acid sequence set forth in SEQ ID NO: 33 or 37, and the VHH CDR3 comprises or consists of the amino acid sequence set forth in SEQ ID NO: 34; or
   (vii) the VHH CDR1 comprises or consists of the amino acid sequence set forth in SEQ ID NO: 17, the VHH CDR2 comprises or consists of an amino acid sequence set forth in SEQ ID NO: 33 or 37, and the VHH CDR3 comprises or consists of the amino acid sequence set forth in SEQ ID NO: 40.
3. The VHH antibody according to embodiment 1, comprising or consisting of a heavy chain variable region, wherein the heavy chain variable region
   (i) comprises or consists of an amino acid sequence having at least 90%, 91%, 92%, 93%, 94%, 95%, 96%, 97%, 98%, or 99% identity to an amino acid sequence selected from any one of SEQ ID NOs: 11, 16, 21, 25, 30, 32, 36, 39, and 42; or
   (ii) comprises or consists of an amino acid sequence selected from any one of SEQ ID NOs: 11, 16, 21, 25, 30, 32, 36, 39, and 42; or
   (iii) comprises an amino acid sequence having one or more (preferably no more than 10, and more preferably no more than 5, 4, 3, 2, or 1) amino acid modifications (preferably amino acid substitutions, and more preferably conservative amino acid substitutions) compared to an amino acid sequence selected from any one of SEQ ID NOs: 11, 16, 21, 25, 30, 32, 36, 39, and 42, wherein preferably, the amino acid modifications do not occur in the CDRs.
4. A heavy-chain antibody specifically binding to LILRB4, comprising the VHH antibody according to any one of embodiments 1-3.
5. The heavy-chain antibody according to embodiment 4, comprising the VHH antibody according to any one of embodiments 1-3 linked to an antibody constant region or an Fc region, wherein preferably, the antibody constant region or the Fc region is from human IgG1, human IgG2, human IgG3, or human IgG4, optionally, the VHH antibody is linked to the Fc region via a hinge region or a portion thereof, and preferably, an amino acid sequence of the portion of the hinge region is EPKSS (SEQ ID NO: 59) or EPKSC (SEQ ID NO: 67).
6. The heavy-chain antibody according to embodiment 4, comprising the VHH antibody according to any one of embodiments 1-3 linked to an antibody Fc region, wherein the Fc region is an Fc region from human IgG1, IgG2, IgG3, or IgG4, and preferably, the Fc region
   (i) comprises or consists of an amino acid sequence having at least 85%, 90%, 91%, 92%, 93%, 94%, 95%, 96%, 97%, 98%, or 99% identity to an amino acid sequence set forth in SEQ ID NO: 55 or 68; or
   (ii) comprises or consists of an amino acid sequence set forth in SEQ ID NO: 55 or 68; or
   (iii) comprises an amino acid sequence having one or more (preferably no more than 10, and more preferably no more than 5, 4, 3, 2, or 1) amino acid modifications (preferably amino acid substitutions, and more preferably conservative amino acid substitutions) compared to an amino acid sequence set forth in SEQ ID NO: 55 or 68.
7. The heavy-chain antibody according to embodiment 4,
   (i) comprising or consisting of an amino acid sequence having at least 85%, 90%, 91%, 92%, 93%, 94%, 95%, 96%, 97%, 98%, or 99% identity to an amino acid sequence selected from any one of SEQ ID NOs: 15, 20, 24, 29, 31, 35, 38, 41, and 43; or
   (ii) comprising or consisting of an amino acid sequence selected from any one of SEQ ID NOs: 15, 20, 24, 29, 31, 35, 38, 41, and 43; or
   (iii) comprising an amino acid sequence having one or more (preferably no more than 10, and more preferably no more than 5, 4, 3, 2, or 1) amino acid modifications (preferably amino acid substitutions, and more preferably conservative amino acid substitutions) compared to an amino acid sequence selected from any one of SEQ ID NOs: 15, 20, 24, 29, 31, 35, 38, 41, and 43, wherein preferably, the amino acid modifications do not occur in the CDRs.
8. The VHH antibody according to any one of embodiments 1-3 or the heavy-chain antibody according to any one of embodiments 4-7, wherein the antibody is a chimeric antibody or a humanized antibody.
9. A bispecific antibody, comprising a first antigen-binding region and a second antigen-binding region, wherein the second antigen-binding region specifically binds to LILRB4 and comprises the VHH antibody according to any one of embodiments 1-3 and 8, or the heavy-chain antibody according to any one of embodiments 4-8.
10. The bispecific antibody according to embodiment 9, wherein the first antigen-binding region specifically binds to CD3.
11. The bispecific antibody according to embodiment 10, wherein the first antigen-binding region comprises a VH and a VL, wherein the VH comprises 3 complementarity determining regions from a heavy chain variable region (HCDRs): HCDR1, HCDR2, and HCDR3, and the VL comprises 3 complementarity determining regions from a light chain variable region (LCDRs): LCDR1, LCDR2, and LCDR3, wherein
   (i) the HCDR1, the HCDR2, and the HCDR3 are three complementarity determining regions HCDR1, HCDR2, and HCDR3 contained in the VH set forth in SEQ ID NO: 3; and the LCDR1, the LCDR2, and the LCDR3 are three complementarity determining regions LCDR1, LCDR2, and LCDR3 contained in the VL set forth in any one of SEQ ID NO: 4; or
   (ii) the HCDR1 consists of the amino acid sequence set forth in SEQ ID NO: 5, the HCDR2 consists of the amino acid sequence set forth in SEQ ID NO: 6, the HCDR3 consists of the amino acid sequence set forth in SEQ ID NO: 7, the LCDR1 consists of the amino acid sequence set forth in SEQ ID NO: 8, the LCDR2 consists of the amino acid sequence set forth in SEQ ID NO: 9, and the LCDR3 consists of the amino acid sequence set forth in SEQ ID NO: 10.
12. The bispecific antibody according to embodiment 11, wherein the first antigen-binding region comprises a VH and a VL, wherein
   the VH comprises or consists of the amino acid sequence set forth in SEQ ID NO: 3, or an amino acid sequence having at least 90%, 91%, 92%, 93%, 94%, 95%, 96%, 97%, 98%, or 99% identity thereto,
   and/or the VL comprises or consists of the amino acid sequence set forth in SEQ ID NO: 4, or an amino acid sequence having at least 90%, 91%, 92%, 93%, 94%, 95%, 96%, 97%, 98%, or 99% identity thereto.
13. The bispecific antibody according to any one of embodiments 10-12, wherein the first antigen-binding region is a Fab specifically binding to CD3.
14. The bispecific antibody according to embodiment 13, wherein the Fab comprises a CH1, wherein the CH1 is a CH1 from IgG1, IgG2, IgG3, or IgG4, preferably a CH1 from IgG1.
15. The bispecific antibody according to embodiment 14, wherein the CH1
   (i) comprises or consists of an amino acid sequence having at least 85%, 90%, 91%, 92%, 93%, 94%, 95%, 96%, 97%, 98%, or 99% identity to the amino acid sequence selected from SEQ ID NO: 51; or
   (ii) comprises or consists of the amino acid sequence selected from SEQ ID NO: 51.
16. The bispecific antibody according to any one of embodiments 10-12, wherein the first antigen-binding region is an scFv specifically binding to CD3, and the scFv comprises the VH and the VL as defined in embodiment 11 or 12; optionally, the VH and the VL are linked via a linker comprising, for example, an amino acid sequence (G4S)n, wherein n = 1, 2, 3, 4, or 5, preferably n = 3 or 4, and more preferably n = 3.
17. The bispecific antibody according to any one of embodiments 9-16, wherein the second antigen-binding region is the VHH according to any one of embodiments 1-3 and 8.
18. The bispecific antibody according to any one of embodiments 9-17, wherein the bispecific antibody is an IgG-like bispecific antibody comprising an Fc dimer, wherein two Fc regions constituting the Fc dimer are the same or different.
19. The bispecific antibody according to embodiment 18, wherein the two Fc regions are different, and preferably, a corresponding knob mutation(s) and a corresponding hole mutation(s) are introduced into the two Fc regions, respectively.
20. The bispecific antibody according to embodiment 19, wherein
   a) one Fc-region polypeptide comprises a knob mutation T366W, and the other Fc-region polypeptide comprises hole mutations T366S, L368A, and Y407V, or
   b) one Fc-region polypeptide comprises knob mutations T366W and Y349C, and the other Fc-region polypeptide comprises hole mutations T366S, L368A, Y407V, and S354C, or
   c) one Fc-region polypeptide comprises knob mutations T366W and S354C, and the other Fc-region polypeptide comprises hole mutations T366S, L368A, Y407V, and Y349C;
   optionally, the Fc region further comprises a mutation(s) that reduces binding to an Fcy receptor, e.g., one or more of an L234A/L235A mutation, a D265A mutation, or a P329A mutation, for example, an L234A/L235A mutation, a D265A mutation, and a P329A mutation.
21. The bispecific antibody according to any one of embodiments 18-20, wherein one or both of the Fc regions comprise a hinge region, e.g., EPKSS or EPKSC.
22. The bispecific antibody according to embodiment 21, wherein
   (i) the Fc region comprising a knob mutation(s)
      a) comprises or consists of an amino acid sequence set forth in SEQ ID NO: 49, 50, or 73; or
      b) comprises or consists of an amino acid sequence having at least 90% identity, e.g., 95%, 96%, 97%, 99%, or higher identity to SEQ ID NO: 49, 50, or 73 and comprising a knob mutation(s) (e.g., S354C and T366W); and/or
   (ii) the Fc region comprising a hole mutation(s)
      a) comprises or consists of an amino acid sequence set forth in SEQ ID NO: 47, 48, or 74; or
      b) comprises or consists of an amino acid sequence having at least 90% identity, e.g., 95%, 96%, 97%, 99%, or higher identity to SEQ ID NO: 47, 48, or 74 and comprising a hole mutation(s) (e.g., Y349C, T366S, L368A, and Y407V).
23. The bispecific antibody according to any one of embodiments 9-22, comprising a first antigen-binding region, a second antigen-binding region, and an Fc dimer, wherein the first antigen-binding region is a Fab fragment specifically binding to CD3, and the second antigen-binding region is a VHH specifically binding to LILRB4, e.g., the VHH according to any one of embodiments 1-3 and 8; for example, the bispecific antibody comprises one first antigen-binding region and 1 or 2 second antigen-binding regions.
24. The bispecific antibody according to embodiment 23, comprising one anti-LILRB4 VHH, one Fab fragment of an anti-CD3 antibody, and an Fc heterodimer, wherein
   the Fab fragment comprises a VH-CH1 and a VL-CL, and the VHH comprises a heavy chain variable region,
   wherein the C-terminus of the CH1 of the Fab fragment is fused to a CH2 or a hinge region of a first Fc region (e.g., an Fc region comprising a knob mutation(s)) to form a first heavy chain;
   the VHH is fused to a CH2 or a hinge region of a second Fc region (e.g., an Fc region comprising a hole mutation(s)) to form a second heavy chain;
      and
   the VL-CL of the Fab fragment constitutes a light chain.
25. The bispecific antibody according to embodiment 24, wherein
   the first heavy chain comprises or consists of the amino acid sequence set forth in SEQ ID NO: 44, or an amino acid sequence having at least 85%, 90%, 91%, 92%, 93%, 94%, 95%, 96%, 97%, 98%, or 99% identity thereto;
   the second heavy chain comprises or consists of the amino acid sequence set forth in SEQ ID NO: 45, or an amino acid sequence having at least 85%, 90%, 91%, 92%, 93%, 94%, 95%, 96%, 97%, 98%, or 99% identity thereto; and/or
   the light chain comprises or consists of the amino acid sequence set forth in SEQ ID NO: 2, or an amino acid sequence having at least 85%, 90%, 91%, 92%, 93%, 94%, 95%, 96%, 97%, 98%, or 99% identity thereto.
26. The bispecific antibody according to embodiment 23, comprising two anti-LILRB4 VHHs, one Fab fragment of an anti-CD3 antibody, and an Fc heterodimer, wherein
   the Fab fragment comprises a VH-CH1 and a VL-CL, and the VHH comprises a heavy chain variable region,
   wherein the C-terminus of a first VHH fragment is fused to the N-terminus of the VH of the Fab fragment, and the C-terminus of the CH1 of the Fab fragment is fused to a CH2 or a hinge region of an Fc region (e.g., comprising a knob mutation(s)) to form a first heavy chain;
   a second VHH is fused to a CH2 or a hinge region of an Fc region (e.g., comprising a hole mutation(s)) to form a second heavy chain;
      and
   the VL-CL of the Fab fragment constitutes a light chain.
27. The bispecific antibody according to embodiment 26, wherein the first VHH and the second VHH are the same or different.
28. The bispecific antibody according to embodiment 26 or 27, wherein fusion of the first VHH and the Fab is achieved via a linker peptide, such as (GGGGS)n, wherein n = 1, 2, 3, or 4.
29. The bispecific antibody according to embodiment 26, wherein
   the first heavy chain comprises or consists of the amino acid sequence set forth in SEQ ID NO: 46, or an amino acid sequence having at least 85%, 90%, 91%, 92%, 93%, 94%, 95%, 96%, 97%, 98%, or 99% identity thereto;
   the second heavy chain comprises or consists of the amino acid sequence set forth in SEQ ID NO: 45, or an amino acid sequence having at least 85%, 90%, 91%, 92%, 93%, 94%, 95%, 96%, 97%, 98%, or 99% identity thereto; and/or
   the light chain comprises or consists of the amino acid sequence set forth in SEQ ID NO: 2, or an amino acid sequence having at least 85%, 90%, 91%, 92%, 93%, 94%, 95%, 96%, 97%, 98%, or 99% identity thereto.
30. The bispecific antibody according to embodiment 23, comprising two anti-LILRB4 VHHs, one scFv of an anti-CD3 antibody, and an Fc heterodimer, wherein
   the scFv comprises, from N-terminus to C-terminus, a VH and a VL in sequence, and the VHH comprises a heavy chain variable region,
31. The bispecific antibody according to embodiment 30, wherein the VH and the VL of the scFv are linked via a linker or a linker peptide; for example, the linker or the linker peptide comprises or consists of the amino acid sequence set forth in SEQ ID NO: 70.
32. The bispecific antibody according to embodiment 30 or 31, wherein the first VHH and the second VHH are the same or different.
33. The bispecific antibody according to any one of embodiments 30-32, wherein fusion of the first VHH and the scFv is achieved via a linker peptide, such as (GGGGS)n, wherein n = 1, 2, 3, or 4.
34. The bispecific antibody according to embodiment 30, wherein
   the first heavy chain comprises or consists of the amino acid sequence set forth in SEQ ID NO: 72, or an amino acid sequence having at least 85%, 90%, 91%, 92%, 93%, 94%, 95%, 96%, 97%, 98%, or 99% identity thereto; and/or
   the second heavy chain comprises or consists of the amino acid sequence set forth in SEQ ID NO: 45, or an amino acid sequence having at least 85%, 90%, 91%, 92%, 93%, 94%, 95%, 96%, 97%, 98%, or 99% identity thereto.
35. A multispecific antibody, comprising the VHH antibody according to any one of embodiments 1-3 and 8, or the heavy-chain antibody according to any one of embodiments 4-8, wherein, for example, the multispecific antibody is a bispecific antibody.
36. A nucleic acid molecule, wherein the nucleic acid molecule encodes the VHH antibody according to any one of embodiments 1-3 and 8, the heavy-chain antibody according to any one of embodiments 4-8, any one of chains of the bispecific antibody according to any one of embodiments 9-34, or any one of chains of the multispecific antibody according to embodiment 35, or consists of the nucleic acid sequence.
37. An expression vector, comprising the nucleic acid molecule according to embodiment 36.
38. A host cell, comprising the nucleic acid molecule according to embodiment 36 or the expression vector according to embodiment 37, wherein preferably, the host cell is prokaryotic or eukaryotic, e.g., a 293 cell or a CHO cell, such as a 293F cell, a 293T cell, or a CHO-S cell.
39. A method for preparing the VHH antibody according to any one of embodiments 1-3 and 8, the heavy-chain antibody according to any one of embodiments 4-8, the bispecific antibody according to any one of embodiments 9-34, or the multispecific antibody according to embodiment 35, the method comprising culturing a host cell comprising the nucleic acid molecule according to embodiment 36 or the expression vector according to embodiment 37 under a condition suitable for expressing chains of the antibody, and optionally recovering the antibody from the host cell (or a host cell culture medium).
40. An immunoconjugate, comprising the VHH antibody according to any one of embodiments 1-3 and 8, the heavy-chain antibody according to any one of embodiments 4-8, the bispecific antibody according to any one of embodiments 9-34, or the multispecific antibody according to embodiment 35.
41. A pharmaceutical composition or a medicament or a formulation, comprising the VHH antibody according to any one of embodiments 1-3 and 8, the heavy-chain antibody according to any one of embodiments 4-8, the bispecific antibody according to any one of embodiments 9-34, the multispecific antibody according to embodiment 35, or the immunoconjugate according to embodiment 40, and optionally a pharmaceutical supplementary material.
42. A pharmaceutical combination product, comprising the VHH antibody according to any one of embodiments 1-3 and 8, the heavy-chain antibody according to any one of embodiments 4-8, the bispecific antibody according to any one of embodiments 9-34, the multispecific antibody according to embodiment 35, or the immunoconjugate according to embodiment 40, and one or more additional therapeutic agents (e.g., a chemotherapeutic agent, a cytokine, a cytotoxic agent, an additional antibody, a small molecule drug, or an immunomodulatory agent).
43. A method for preventing or treating a cancer in a subject, comprising administering to the subject an effective amount of the VHH antibody according to any one of embodiments 1-3 and 8, the heavy-chain antibody according to any one of embodiments 4-8, the bispecific antibody according to any one of embodiments 9-34, the multispecific antibody according to embodiment 35, the immunoconjugate according to embodiment 40, the pharmaceutical composition or the medicament or the formulation according to embodiment 41, or the pharmaceutical combination product according to embodiment 42.
44. The method according to embodiment 43, wherein tumor cells of the cancer have an LILRB4 protein level and/or nucleic acid level that are present or elevated (e.g., elevated expression).
45. The method according to embodiment 43 or 44, wherein the cancer is a solid tumor or a hematological tumor, such as a solid tumor, multiple myeloma, or leukemia, e.g., acute myeloid leukemia (AML), e.g., monocytic AML or non-monocytic AML, e.g., a human myelomonocytic leukemia cell, human acute promyelocytic leukemia, or human acute myeloid leukemia.
46. The method according to any one of embodiments 43-45, wherein the method further comprises administering in combination with an additional therapy such as a therapeutic modality (e.g., radiotherapy) and/or an additional therapeutic agent (e.g., a chemotherapeutic agent, a cytokine, a cytotoxic agent, an additional antibody, a small molecule drug, or an immunomodulatory agent).
47. A method for detecting the presence of LILRB4 in a biological sample, comprising
   (i) contacting a biological sample with the VHH antibody according to any one of embodiments 1-3 and 8, the heavy-chain antibody according to any one of embodiments 4-8, the bispecific antibody according to any one of embodiments 9-34, or the multispecific antibody according to embodiment 35, under a condition that allows the antibody to bind to LILRB4, and
   (ii) detecting whether a complex is formed by the antibody or the bispecific antibody and LILRB4, wherein formation of the complex indicates the presence of LILRB4.

### Examples

### Example 1: Generation of LILRB4 Antibodies by Immunization

Anti-human LILRB4 antibodies of the present disclosure can be prepared by the following method. However, the method for preparing the anti-human LILRB4 antibody of the present disclosure is not limited to this method, and the antibody may also be prepared by other methods known in the art.

### 1.1. Alpaca immune library construction and screening

### 1.1.1. Animal immunization

2 alpacas (Chengdu NBbiolab, Co., Ltd.) were immunized with a human LILRB4-his recombinant protein (ACRO, Cat. No.: LI4-H52H7). The alpacas were subjected to immunization injections four times at an interval of 21 days (Table 1). The alpacas were subjected to venous blood collection on day 10 after the second immunization and day 10 after the third booster immunization. The immune serum of mice was subjected to the ELISA assay using the human LILRB4-his recombinant protein, and one alpaca was selected for blood collection and library construction.

**Table 1. Alpaca immunization procedure**

| Procedure | Route of administration | Dose |
|---|---|---|
| Primary Immunization | Subcutaneous injection | 0.5 mg protein/alpaca |
| 1st Boost | Subcutaneous injection | 0.25 mg protein/alpaca |
| 2nd Boost | Subcutaneous injection | 0.25 mg protein/alpaca |
| 3nd Boost | Subcutaneous injection | 0.25 mg protein/alpaca |
| Final Boost | Subcutaneous injection | 0.25 mg protein/alpaca |

### 1.1.2. Alpaca immune library construction and screening

Total RNA was extracted from the peripheral blood of the alpaca using the Trizol method, and reverse transcription was performed using a Prime Script II 1st Strand cDNA Synthesis Kit (Takara, Cat. No.: 6210A) to obtain cDNA. The cDNA was subjected to nested PCR amplification, and 750 bp products containing nanobodies were recovered. A second round of PCR was performed to amplify and purify the VHHs. The VHHs were ligated into a phage vector using enzyme digestion and ligation techniques, followed by electroporation, transformation, and plating. The next day, the library capacity was determined, and the library capacity of the constructed library was found to be 2 × 10e8 CFU, with an insertion rate of 100%. 2 rounds of solid-phase screening were performed using a human LILRB4 recombinant protein, and the positive clones obtained were sequenced and identified. The partial amino acid sequences obtained are shown in Table 2.

**Table 2. Amino acid sequences of camelid-derived VHHs (anti-LILRB4 antibodies)**

| Antibody name | VHH sequence | HCDR1 | HCDR2 | HCDR3 |
|---|---|---|---|---|
| B1A2-VHH | SEQ ID NO: 11 | 12 | 13 | 14 |
| B1G5-VHH | SEQ ID NO: 16 | 17 | 18 | 19 |
| 1H12-VHH | SEQ ID NO:21 | 75 | 22 | 23 |
| 2A3-VHH | SEQ ID NO:25 | 26 | 27 | 28 |

### Example 2: Characterization of Anti-LILRB4 Antibodies

### 2.1. Synthesis and expression of anti-LILRB4 chimeric antibodies

The amino acid sequences of the camelid-derived antibodies (VHHs) in Table 2 were subjected to codon optimization and
gene synthesis by General Biology System (Anhui) Co., Ltd. The variable regions encoding each VHH antibody were added to the hinge region (SEQ ID NO: 59) and then inserted into an expression vector pcDNA3. 1(+) containing a sequence encoding the human Fc constant region (SEQ ID NO: 55) to obtain plasmids encoding the antibodies.

The specific chimeric antibody sequences obtained were as follows:

**Table 3. Amino acid sequences of anti-LILRB4 chimeric antibodies**

| Antibody name | Heavy chain sequence | Variable region sequence | HCDR1 | HCDR2 | HCDR3 |
|---|---|---|---|---|---|
| B1A2 | SEQ ID NO:15 | SEQ ID NO: 11 | 12 | 13 | 14 |
| B1G5 | SEQ ID NO:20 | SEQ ID NO: 16 | 17 | 18 | 19 |
| 1H12 | SEQ ID NO:24 | SEQ ID NO:21 | 75 | 22 | 23 |
| 2A3 | SEQ ID NO:29 | SEQ ID NO:25 | 26 | 27 | 28 |

The plasmid encoding the anti-LILRB4 antibody was transfected into ExpiCHO-S cells to express camelid-derived chimeric antibodies. h193 (a fully human monoclonal antibody against LILRB4) (with a heavy chain amino acid sequence of SEQ ID NO: 61 and a light chain amino acid sequence of SEQ ID NO: 62) was used as a control antibody, and the sequences are from PCT Publication No. WO2020056077A1. Similarly, the control antibody h193 was expressed, and the antibody was purified accordingly.

Specifically, the plasmids encoding the anti-LILRB4 chimeric antibodies were transfected into ExpiCHO-S cells using an ExpiCHO^{™} expression system (ThermoFisher, Cat# A29133) to similarly express the control antibody h193 monoclonal antibody, according to the manufacturer's product instructions. The cells were cultured for 10-12 days after transfection. When the cell viability decreased to 60% to 70%, the supernatants were collected, and the antibodies expressed and secreted in the supernatants were purified using a MabSelect Sure protein A affinity chromatography system (GE healthcare). The purified antibodies were concentrated and subjected to sterile filtration. The purity of the antibody protein was detected by SDS-PAGE and size exclusion chromatography. The results indicated that the purity of the antibodies was more than 90%, and the antibodies could be used in the next step.

### 2.2. Binding of anti-LILRB4 chimeric antibodies to human and cynomolgus monkey LILRB4 engineered cells

Whether the anti-LILRB4 chimeric antibodies in the present disclosure could bind to the engineered cells expressing the human or cynomolgus monkey LILRB4 protein was determined by a cell binding experiment.

A sequence encoding human LILRB4 (Q8NHJ6, SEQ ID NO: 65) was transfected into CHO-K1 cells (ATCC, Cat. No.: CCL-61^{™}) by electroporation, and then the cells were screened with 24 µg/mL puromycin (Gibco, Cat. No.: A1113802) to obtain CHO-K1 cells highly expressing human LILRB4 (CHO-K1-huLILRB4).

A sequence encoding cynomolgus monkey LILRB4 (XP_015297198.1, SEQ ID NO: 66) was transfected into CHO-K1 cells (ATCC, Cat. No.: CCL-61^{™}) or 293T cells (Cell Bank of Type Culture Collection Committee of the Chinese Academy of Sciences, GNHu17) by electroporation, and then the cells were screened with 24 µg/mL (for CHO-K1 cells) or 0.3 µg/mL (for 293T cells) puromycin to obtain CHO-K1 cells (CHO-K1-cynoLILRB4) and 293T cells (293T-cynoLILRB4) highly expressing cynomolgus monkey LILRB4.

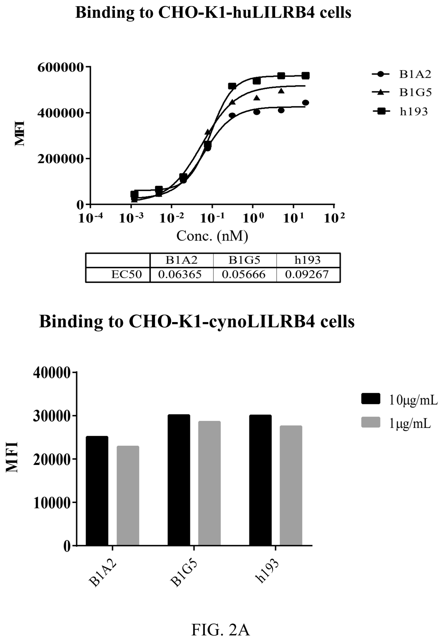

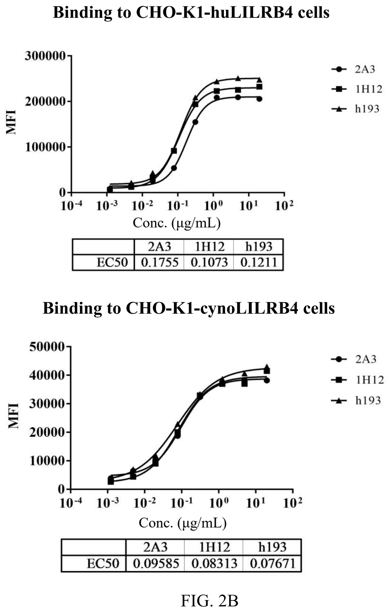

Adherent CHO-K1-huLILRB4, CHO-K1-cynoLILRB4, and 293T-cynoLILRB4 cell lines were subjected to enzymatic digestion to obtain single-cell suspensions. The single-cell suspensions were centrifuged at room temperature at 300× g for 4 min, and then the media were discarded. The resulting cell pellet was resuspended and washed once with a PBS buffer and centrifuged, and the supernatant was discarded to obtain a cell pellet again. The cell pellet was resuspended in the anti-LILRB4 chimeric antibodies obtained by gradient dilution (at an initial concentration of 20 µg/mL, 4-fold gradient dilution, 8 concentrations in total), and the cells were incubated at 4 °C for 30 min. The cells were washed once with PBS, and then fluorescent secondary antibody R-PE-conjugated AffiniPure Goat Anti-Human IgG, Fcγ Fragment Specific (Jackson ImmunoResearch, Cat#: 109-116-098) diluted in a 1:200 ratio was added. The cells were incubated in the dark at 4 °C for 30 min. The cells were washed twice with PBS and then resuspended, and finally, two-channel fluorescence signals were detected using a flow cytometer. The results are shown in FIGs. 2A and 2B.

FIG. 2A shows that the anti-LILRB4 chimeric antibodies (designated as antibody B1A2 and antibody B1G5) and antibody h193 are all capable of binding to engineered cells expressing human/cynomolgus monkey LILRB4. FIG. 2B shows that the chimeric anti-LILRB4 antibodies (designated as 2A3 and antibody 1H12) and antibody h193 are all capable of binding to cells expressing human/cynomolgus monkey LILRB4, and the binding activity was comparable to that of the control antibody h193.

### Example 3: Humanization of Anti-LILRB4 Antibody and Characterization Thereof

### 3.1. Humanization of alpaca-derived anti-LILRB4 antibody

The antibody B1G5 obtained in Example 2.1 was humanized. Specifically, the sequences of the antibody B1G5 were searched and aligned in the IMGT database to obtain a human germline gene sequence IGHV3-7*01 with relatively high homology to the variable region of the antibody B1G5, which was used as a humanized framework of the heavy chain variable region. The CDRs in the heavy chain variable region of the antibody B1G5 were grafted into the corresponding humanized framework to form humanized antibodies of the antibody B1G5. In order to maintain the affinity of the humanized antibodies for LILRB4, the humanized antibody framework regions obtained were subjected to back-mutation.

**Table 4. Heavy chain and variable region sequences of humanized antibodies**

| Heavy chain name and sequence | Heavy chain variable region | HCDR1 | HCDR2 | HCDR3 |
|---|---|---|---|---|
| huB1G5-1 (SEQ ID NO:31) | SEQ ID NO:30 | SEQ ID NO:17 | SEQ ID NO:18 | SEQ ID NO:19 |
| huB1G5-2 (SEQ ID NO: 35) | SEQ ID NO:32 | SEQ ID NO:17 | SEQ ID NO:33 | SEQ ID NO:34 |
| huB1G5-3 (SEQ ID NO: 38) | SEQ ID NO:36 | SEQ ID NO:17 | SEQ ID NO:37 | SEQ ID NO:34 |
| huB1G5-4 (SEQ ID NO: 41) | SEQ ID NO:39 | SEQ ID NO:17 | SEQ ID NO:33 | SEQ ID NO:40 |
| huB1G5-5 (SEQ ID NO: 43) | SEQ ID NO:42 | SEQ ID NO:17 | SEQ ID NO:37 | SEQ ID NO:40 |

### 3.2. Expression and purification of humanized anti-LILRB4 antibody

Similarly as described in Example 2.1, the plasmids encoding the full-length anti-LILRB4 humanized antibodies were transfected into ExpiCHO-S cells to express the anti-LILRB4 humanized antibodies, and the corresponding purification was performed as described in Example 2.1. The purified anti-LILRB4 humanized antibodies were concentrated, and subjected to sterile filtration. The purity of the anti-LILRB4 humanized antibodies was detected by SDS-PAGE and size exclusion chromatography (SEC).

### 3.3. Physicochemical analysis of humanized anti-LILRB4 antibody

For the humanized anti-LILRB4 antibodies obtained, the purity of each antibody was determined by size exclusion chromatography. Specifically, 20 µg of each humanized anti-LILRB4 antibody sample in Table 4 was injected onto a TSK G3000SWXL column using 100 mM sodium phosphate + 100 mM Na₂SO₄ (pH 7.0) as a running buffer for 30 min. The collected effluent was measured using the Agilent 1220 HPLC system, and the data were analyzed using OpenLAB software. The results show that the purity of each humanized anti-LILRB4 antibody was more than 90%, and the humanized anti-LILRB4 antibodies could be used in the next step.

### 3.4. Binding of humanized anti-LILRB4 antibodies to tumor cell line

Whether the anti-LILRB4 humanized antibodies in the present disclosure could bind to tumor cells expressing LILRB4 was determined by a cell binding experiment.

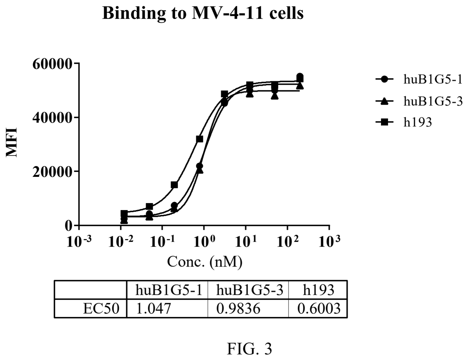

The MV-4-11 (human myelomonocytic leukemia cell, Cell Bank of Chinese Academy of Sciences, CAT# SCSP-5031) cell line was collected, pipetted, and mixed well to obtain a single-cell suspension. The single-cell suspension was centrifuged at room temperature at 300× g for 4 min, and then the medium was discarded. The resulting cell pellet was washed once with PBS. The humanized anti-LILRB4 antibodies obtained by gradient dilution (at an initial concentration of 200 nM, 4-fold gradient dilution, 8 concentrations in total) were used for resuspension, and the cells were incubated at 4 °C for 30 min. The cells were washed once with PBS, and then fluorescent secondary antibody R-PE-conjugated AffiniPure Goat Anti-Human IgG, Fcγ Fragment Specific (Jackson ImmunoResearch, Cat#: 109-116-098) diluted in a 1:200 ratio was added. The cells were incubated in the dark at 4 °C for 30 min. The cells were washed twice with PBS and then resuspended, and finally, two-channel fluorescence signals were detected using a flow cytometer. The results are shown in FIG. 3.

FIG. 3 shows that the humanized anti-LILRB4 antibodies (designated as antibody huB1G5-1 and antibody huB1G5-3) and antibody h193 were all capable of binding to tumor cells expressing LILRB4, and the binding activity was comparable to that of the control antibody h193.

### Example 4: Preparation and Characterization of Humanized CD3 Antibodies

### 4.1. Preparation of CD3 antibody humanization

The SP34 antibody is a murine antibody specifically binding to the CD3 epsilon subunit (CD3ε), and it has species cross-activity with humans and monkeys (Pessano, S., et al. (1985). "The T3/T cell receptor complex: antigenic distinction between the two 20-kd T3 (T3-delta and T3-epsilon) subunits." EMBO J 4(2): 337-344.). In this example, the antibody SP34 was used as a candidate molecule for humanization construction.

The murine antibody SP34 was humanized using CDR grafting technology. Briefly, the SP34 VH and SP34 VL sequences described above were searched and aligned in the IMGT database, thereby obtaining human germline gene sequences IGHV3-73*01 and IGLV7-46*01 which had relatively high homology to the heavy chain variable region and the light chain variable region of SP34, respectively, and then using them as frameworks of humanized antibody variable regions; the CDRs of the murine antibody SP34 were grafted into the corresponding heavy and light chain variable region frameworks of the humanized antibody, respectively, to form humanized anti-CD3 antibody variable regions. In order to maintain the affinity of the murine antibody SP34, the humanized antibody variable regions obtained were back-mutated.

Thus, a humanized heavy chain variable region sequence (hu34H1) and a light chain variable region sequence (hu34L2) of the SP34 antibody were obtained, and a humanized antibody of the SP34 antibody was further obtained. The specific sequences are shown in Table 5.

**Table 5: Sequences of humanized anti-CD3 antibody (CDR sequences are numbered according to the Kabat numbering scheme)**

| Name | SEQ ID NO |
|---|---|
| Heavy chain of humanized SP34 antibody hu34 hu34H 1 | 1 |
| Light chain of humanized SP34 antibody hu34 hu34L2 | 2 |
| Heavy chain variable region VH of humanized SP34 antibody hu34 (hu34H1) | 3 |
| Light chain variable region VL of humanized SP34 antibody hu34 (hu34L2) | 4 |
| HCDR1 | 5 |
| HCDR2 | 6 |
| HCDR3 | 7 |
| LCDR1 | 8 |
| LCDR2 | 9 |
| LCDR3 | 10 |

The sequences of the humanized control antibody (017-Roche-CD3) were from the patent US 2016/0075785 A1; the heavy chain amino acid sequence of the control antibody was set forth in SEQ ID NO: 63, and the light chain was set forth in SEQ ID NO: 64. See the sequence listing for details.

The variable region amino acid sequences in Table 5 and of the control antibody 017-Roche-CD3 were sent to General Biology System (Anhui) Co., Ltd. for codon optimization and gene synthesis. The genes encoding the VH region and the VL region of the 017-Roche-CD3 antibody were inserted into an expression vector pcDNA3.1(+) comprising a coding gene of a human IgG1 heavy chain constant region (with the amino acid sequence set forth in SEQ ID NO: 52) and a coding gene of a lambda light chain constant region (with the amino acid sequence set forth in SEQ ID NO: 56) in sequence to obtain a plasmid expressing the anti-CD3 humanized antibody 017-2 full-length heavy chain hu34H1 (SEQ ID NO: 1) and a plasmid encoding the full-length light chain hu34L2 (SEQ ID NO: 2). Meanwhile, the synthesized VH region (hu34H1) and VL region (hu34L2) were linked via a linker peptide (G4S)3 (SEQ ID NO: 70) to constitute a single-chain antibody 23L2 (23L2ScFv) (SEQ ID NO: 69), and inserted into the expression vector pcDNA3.1(+) comprising a coding gene of a human IgG1 heavy chain constant region Fc region (with the amino acid sequence set forth in SEQ ID NO: 53) to obtain a plasmid expressing DA023AH23L2 (scFv-Fc) (SEQ ID NO: 71). The heavy chain constant regions used all contained mutations at sites L234A, L235A, D265A, and P329A (according to Eu numbering) to eliminate effector functions.

The single-chain antibody (23L2) was named 23L2ScFv, and its amino acid sequence was set forth in SEQ ID NO: 69. The synthesized anti-CD3 single-chain antibody comprising the constant region Fc domain was named DA023AH23L2 (VH-linker peptide-VL-Fc), and its sequence was set forth in SEQ ID NO: 71, wherein the amino acid sequence of the VH-linker peptide-VL was set forth in SEQ ID NO: 69, the amino acid sequence of the linker peptide was set forth in SEQ ID NO: 70, and the amino acid sequence of the Fc region was set forth in SEQ ID NO: 53.

The plasmids obtained above were co-transfected into ExpiCHO-S cells to express the control antibody 017-Roche-CD3, 017-2, and DA023AH23L2 using an ExpiCHO^{™} expression system (ThermoFisher, Cat# A29133) according to the manufacturer's product instructions. The cells were cultured for 10-12 days after transfection. When the cell viability decreased to 60%-70%, the supernatant was collected, and the antibody expressed in the supernatant was purified using a MabSelect Sure protein A affinity chromatography system (GE healthcare). The purified antibodies were concentrated and subjected to sterile filtration. The purity of the antibody protein was detected by SDS-PAGE and size exclusion chromatography. The results indicated that the purity of the antibodies met the requirements, and the antibodies could be used in the next step.

### 4.2. Detection of activating effects of humanized CD3 antibodies

The activating effect of the CD3 antibody was detected using a Jurkat/NFAT-luc (luciferase) reporter gene system. According to the manufacturer's instructions, reporter gene elements in NFAT-luc2P (plasmid: Promega, Cat# E8481) were transfected into Jurkat cells (Cell Bank of Type Culture Collection Committee of the Chinese Academy of Sciences, Cat# SCSP-513) by Gene Pul Ser X Cell TM (BIO-RAD) electroporation, and then the cells were screened with hygromycin B (Invitrogen, Cat# 10687010) to obtain an NFAT-luc2P polyclonal cell strain. The polyclonal cells were subjected to limiting dilution to obtain a stable monoclonal cell strain Jurkat/NFAT-luc highly expressing NFATluc2P.

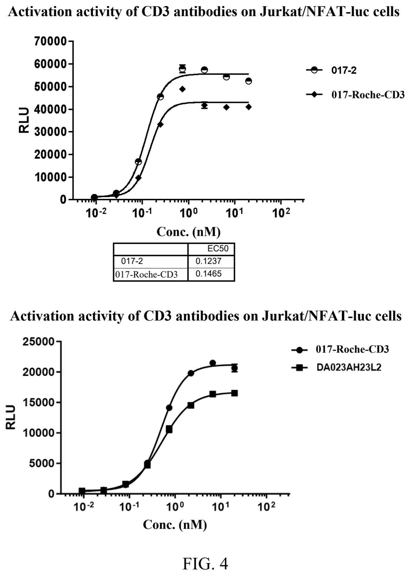

The cells were collected and centrifuged, and the supernatant was removed. The cells were resuspended in an assay buffer (99% 1640 medium + 1% FBS) with a cell density of 5 × 10⁵ cells/mL. The cells were seeded into a 384-well plate (Corning, Cat# 3570) at 40 µL per well. Then, 10 µL of the humanized anti-CD3 antibodies DA023AH23L2, 017-2, and 017-Roche-CD3 obtained by gradient dilution (at a final concentration of 20 nM, 3-fold dilution, 8 gradients) was added per well. The 384-well plate was placed in an incubator at 37 °C. After 6 h, 30 µL of the One-Glo (Promega, Cat# E6130) reagent was added to each well, and finally, luminescence signals were detected using a multifunctional microplate reader (Tecan Infinite F200PRO). The activating effect of the CD3 antibody was directly reflected by the expression level of luciferase. The results are shown in FIG. 4, indicating that 017-2 and DA023AH23L2 have activating effects.

### Example 5: Preparation and Affinity Assay of Anti-LILRB4×CD3 Bispecific Antibodies

### 5.1. Construction of bispecific antibodies

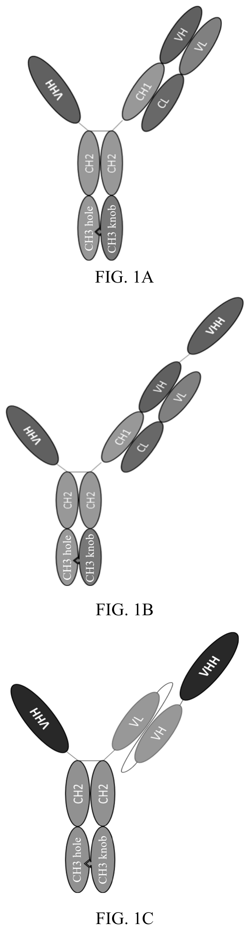

In the present disclosure, three types of bispecific antibodies with different asymmetric structures were constructed as shown in FIG. 1. The anti-LILRB4 moiety of each bispecific antibody was from the humanized huB1G5-3 antibody described above, the anti-CD3 moiety was from the humanized CD3 antibody described above, and the constant regions of the bispecific antibodies each comprised a knob-in-hole (KIH) structure (Merchant, A. M., et al. (1998). "An efficient route to human bispecific IgG." Nat Biotechnol 16(7): 677-681.). Such bispecific antibodies are also referred to herein as "anti-CD3/LILRB4 bispecific antibodies" or "anti-CD3/anti-LILRB4 bispecific antibodies", sometimes simply referred to as "bispecific antibody molecules" or "diabodies". The anti-LILRB4 moiety of the anti-CD3/LILRB4 bispecific antibodies targeted cells expressing LILRB4, while the anti-CD3 moiety activated T cells. The diabodies simultaneously bound to LILRB4 on tumor cells and CD3 on T cells to facilitate targeted killing of the tumor cells by the activated T cells.

In the present application, 3 types of anti-CD3/LILRB4 bispecific antibodies with the structures shown in FIG. 1 were obtained using standard construction methods:
CD3Fab heavy chain-knob: The sequence encoding the heavy chain variable region of the anti-CD3 humanized monoclonal antibody was cloned into a pcDNA3.1(+) vector containing IgG1 heavy chain constant region with Fc (knob) for expression to obtain a CD3Fab heavy chain-knob molecule.
huB1G5-hole: The variable region sequence of the anti-LILRB4 humanized antibody huB1G5-3 was cloned into a pcDNA3.1(+) vector containing Fc (hole) for expression to obtain a huB1G5-hole molecule.
huB1G5-CD3Fab-knob: The sequence encoding the huB1G5-3 variable region and the sequence encoding the heavy chain variable region of the anti-CD3 humanized monoclonal antibody were sequentially ligated in tandem using an overlapping PCR technique (with a linker GGGGS between the two encoding fragments). The ligated sequence was then cloned into a pcDNA3.1(+) vector containing the IgG1 heavy chain constant region with Fc (knob) for expression to obtain a huB1G5-CD3Fab heavy chain-knob molecule.
CD3Fab light chain: The light chain variable region encoding the anti-CD3 humanized monoclonal antibody was cloned into a light chain constant region.
CD3ScFv heavy chain-knob: The nucleotide sequence encoding the anti-CD3 single-chain antibody 23L2 was cloned into a pcDNA3.1(+) vector with Fc (knob) for expression to obtain a 23L2ScFv-knob molecule.
huB1G5-Hu34ScFv-knob: The sequence encoding the huB1G5-3 variable region and the sequence encoding the anti-CD3 humanized ScFv were sequentially ligated in tandem using an overlapping PCR technique (with a linker GGGGS between the two encoding fragments). The ligated sequence was then cloned into a pcDNA3.1(+) vector containing the IgG1 heavy chain constant region with Fc (knob) for expression to obtain a huB1G5-CD3Fab heavy chain-knob molecule.

The bispecific antibodies in FIG. 1 were obtained by accordingly combining the expression vectors described above according to the specific composition shown in Table 6 and expressing them under appropriate conditions.

The construction, expression, purification, and preliminary analysis steps of the bispecific antibodies were the same as those in Example 2.1.

**Table 6. Humanized bispecific antibodies**

| Clone | Chain | Structure | Amino acid sequence |
|---|---|---|---|
| huB1G5-CD3Fab | HC1: CD3Fab heavy chain-knob SEQ ID NO: 44 | hu34H1-VH | SEQ ID NO:3 |
| | | CH1 | SEQ ID NO:51 |
| | | Fc (knob) (hinge region EPKSC-CH2-CH3knob) | SEQ ID NO:49 |
| | LC1: CD3Fab light chain SEQ ID NO: 2 | Hu34L-2 | SEQ ID NO:2 |
| | HC2: huB1G5-hole SEQ ID NO:45 | huB1G5-3 | SEQ ID NO:36 |
| | | Fc (hole) (hinge region EPKSS-CH2-CH3hole) | SEQ ID NO:48 |
| huB1G5-CD3-2VHH | HC1: huB1G5-CD3Fab heavy chain-knob SEQ ID NO: 46 | huB1G5-3 | SEQ ID NO:36 |
| | | Linker peptide | SEQ ID NO:60 |
| | | hu34H1-VH | SEQ ID NO:3 |
| | | CH1 | SEQ ID NO:51 |
| | | Fc (knob) (hinge region EPKSC-CH2-CH3knob) | SEQ ID NO:49 |
| | LC1: CD3Fab light chain SEQ ID NO: 2 | Hu34L-2 | SEQ ID NO:2 |
| | HC2: huB1G5-hole SEQ ID NO:45 | huB1G5-3 | SEQ ID NO:36 |
| | | Fc (hole) (hinge region EPKSS-CH2-CH3hole) | SEQ ID NO:48 |
| huB1G5-ScFv-2VHH | HC1: huB1G5-Hu34ScFv-knob SEQ ID NO:72 | huB1G5-3 | SEQ ID NO:36 |
| | | Linker peptide | SEQ ID NO:60 |
| | | 23L2 ScFv | SEQ ID NO:69 |
| | | Fc (knob) (hinge region EPKSS-CH2-CH3knob) | SEQ ID NO:73 |
| | HC2: huB1G5-hole SEQ ID NO:4 | huB1G5-3 | SEQ ID NO:36 |
| | | Fc (hole) (hinge region EPKSS-CH2-CH3hole) | SEQ ID NO:48 |

The heavy and light chain expression vectors obtained (see Table 6) were co-transfected into ExpiCHO-S cells for expression under appropriate conditions to obtain bispecific antibody proteins, and the expression, purification, and preliminary analysis steps were the same as those in Example 2.1.

In addition, the amino acid sequences (SEQ ID NOs: 76, 77, 78, and 79) of the synthetic control antibody 4ab3-4ab S91A (CN 115551894 A) were subjected to codon optimization and gene synthesis by General Biology System (Anhui) Co., Ltd. The expression and purification methods were as described in CN 115551894 A.

### 5.2. Assay on affinity of bispecific antibodies for human/cynomolgus monkey LILRB4 and CD3

In this experiment, the binding affinity of the huB1G5-CD3Fab, huB1G5-CD3-2VHH, and huB1G5-ScFv-2VHH bispecific antibodies for the human LILRB4 protein (ACRO Biosystems, Cat. No.: LI4-H52H7), the cynomolgus monkey LILRB4 protein (ACRO Biosystems, Cat. No.: CDK-C5227), the human CD3 protein (Sino Biological, Cat. No.: CT038-H2508H), or the cynomolgus monkey CD3 protein (ACRO Biosystems, Cat. No.: CDD-C52W4) was determined using ForteBio Octet RED96e according to the manufacturer's instructions.

Briefly, an AHC sensor (ForteBio, Cat. No.: 18-5060) was pre-equilibrated in a running buffer (1× PBS, XiGene, Cat. No.: XG3650, containing 0.02% Tween 20, 0.1% BSA, pH 7.0) at room temperature for 10 min.

In a 96-well plate, the kinetic assay for LILRB4 was performed according to the following steps:
a) equilibrating the baseline with the running buffer for 180 s;
b) adding each bispecific antibody diluted with the running buffer at a final concentration of 5 µg/mL and immobilizing for 200 s;
c) equilibrating the baseline with the running buffer for 300 s;
d) adding the human LILRB4 protein or the cynomolgus monkey LILRB4 protein diluted with the running buffer to each well at the following concentrations: 100 nM, 50 nM, 25 nM, 12.5 nM, 6.25 nM, 3.13 nM, and 1.56 nM, binding for 200 s, and dissociating for 600 s;
e) applying a regeneration solution (0.01 M Gly-HCl, pH 1.5) for 30 s. The experimental data were fitted and calculated using a 1:1 binding model in the Fortebio Data Analysis software.

In a 96-well plate, the kinetic assay for CD3 was performed according to the following steps:
a) equilibrating the baseline with the running buffer for 300 s;
b) adding each bispecific antibody diluted with the running buffer at a final concentration of 5 µg/mL and immobilizing for 200 s;
c) equilibrating the baseline with the running buffer for 200 s;
d) adding the human CD3 protein or the cynomolgus monkey CD3 protein diluted with the running buffer to each well at the following concentrations: 200 nM, 100 nM, 50 nM, 25 nM, 12.5 nM, 6.25 nM, and 3.13 nM, binding for 120 s, and dissociating for 400 s;
e) applying a regeneration solution (0.01 M Gly-HCl, pH 1.5) for 30 s. The experimental data were fitted and calculated using a 1:1 binding model in the Fortebio Data Analysis software.

Table 7 summarizes the binding affinity of the huB1G5-CD3Fab, huB1G5-CD3-2VHH, and huB1G5-ScFv-2VHH bispecific antibodies for the human LILRB4 protein, the cynomolgus monkey LILRB4 protein, human CD3 protein, or the cynomolgus monkey CD3 protein.

**Table 7. Binding affinity of bispecific antibodies for LILRB4 and CD3 proteins**

| Antigen | Antibody | K_{D} (M) | Kon(1/Ms) | Kdis(1/s) |
|---|---|---|---|---|
| Human LILRB4 protein | huB1G5-CD3Fab | 7.11E-10 | 3.08E+05 | 2.19E-04 |
| Cynomolgus monkey LILRB4 protein | | 1.10E-08 | 3.20E+05 | 3.51E-03 |
| Human CD3 protein | | 2.63E-08 | 3.05E+05 | 8.03E-03 |
| Cynomolgus monkey CD3 protein | | 6.59E-09 | 3.18E+05 | 2.10E-03 |
| Human LILRB4 protein | huB1G5-CD3-2VHH | 6.19E-10 | 1.93E+05 | 1.20E-04 |
| Cynomolgus monkey LILRB4 protein | | 1.06E-08 | 2.18E+05 | 2.30E-03 |
| Human CD3 protein | | 2.26E-08 | 2.42E+05 | 5.49E-03 |
| Cynomolgus monkey CD3 protein | | 6.92E-09 | 2.76E+05 | 1.91E-03 |
| Human LILRB4 protein | huB1G5-ScFv-2VHH | 7.24E-10 | 2.92E+05 | 2.12E-04 |
| Cynomolgus monkey LILRB4 protein | | 1.53E-08 | 2.38E+05 | 3.65E-03 |
| Human CD3 protein | | 3.57E-08 | 3.13E+05 | 1.12E-02 |
| Cynomolgus monkey CD3 protein | | 2.82E-08 | 2.84E+05 | 8.01E-03 |

Table 7 shows that the bispecific antibodies of the present disclosure can specifically bind to the human LILRB4 protein, the cynomolgus monkey LILRB4 protein, the human CD3 protein, or the cynomolgus monkey CD3 protein. Moreover, the K_{D} values of the bispecific antibodies huB1G5-CD3Fab, huB1G5-CD3-2VHH, and huB1G5-ScFv-2VHH for the human LILRB4 protein were two orders of magnitude higher than those for the human CD3 protein. The anti-LILRB4×CD3 bispecific antibodies huB1G5-CD3Fab, huB1G5-CD3-2VHH, and huB1G5-ScFv-2VHH targeting human LILRB4 with high affinity can effectively reduce their side effects due to targeting human CD3.

### Example 6: Killing Activity of Anti-LILRB4×CD3 Bispecific Antibodies Against Tumor Cells

To detect the activity of the anti-LILRB4×CD3 bispecific antibodies in mediating T cells to kill tumor cells, a primary T cell-dependent cytotoxicity assay system was established. In this system, HL-60 cells (human acute promyelocytic leukemia cell line, source: Chinese Academy of Sciences, TCHu 23) that endogenously express human LILRB4 were used as target cells, and T cells isolated from human peripheral blood mononuclear cells (PBMCs) were used as effector cells.

### 6.1. Killing activity of huB1G5-CD3Fab and huB1G5-CD3-2VHH bispecific antibodies against tumor cells

PBMC cells (Shanghai AoNeng Biotechnology Co., Ltd.) were thawed and adjusted to a cell density of 1-2 × 10⁶ cells/mL using a 1640 + 10% FBS complete medium. The cells were activated overnight with IL2 (NMPA Approval Number: S10970058) at a final concentration of 100 IU/mL. The next morning, the PBMC cell suspension was collected, and all T cells were sorted using a T cell isolation kit (Stemcell, Cat# 17951RF) according to the instructions. The cells were centrifuged at 400 g for 5 min, then resuspended in an MEM-α (Gibco, Cat# 41061-029) assay buffer containing 1% FBS (Gibco, Cat# 10099-141) and 100 IU/mL IL-2, and counted. The T cells were adjusted to a density of 2 × 10⁵ cells/mL, and seeded into a 96-well plate (Corning, Cat# 3599) at 100 µL per well (i.e., 20000 cells per well). The cultured target cells HL-60 were collected and centrifuged at 200 g for 5 min to collect the cells, and the supernatant was discarded. The cells were resuspended in PBS and centrifuged at 200 g for 5 min, and the supernatant was discarded. The cells were resuspended in PBS and counted. The CellTrace^{™} Violet cell proliferation reagent dye (Thermo, C34557) was added at a ratio of 1:2000. The mixture was left to stand at 37 °C for 15 min, and 5 volumes of a complete medium were added to stop the staining. The mixture was centrifuged at 200 g for 5 min, and the supernatant was discarded. The cells were washed once with 3 mL of the complete medium, resuspended in an assay buffer, and counted. The target cells were adjusted to a density of 2.67 × 10⁵ cells/mL, and the cells were seeded into the above 96-well plate at 75 µL per well (i.e., 20000 cells per well) (effector cell:target cell = 1:1). The test antibodies were prepared at 8-fold the final detection concentration. The maximum detection concentration of the test antibodies was 1 nM. A 5-fold gradient dilution was performed to obtain a total of 9 concentration points, and 25 µL of the test antibody was added to each well. In this study, the minimal killing (the assay buffer was added to the target cells) and natural killing (the effector cells were added to the target cells) controls were set. The final total volume of each well was 200 µL, and the plate was incubated in a cell incubator for another 22 h. After the incubation was completed, the experimental plate was taken out and centrifuged at 2000 rpm for 3 min to allow all cells to settle to the bottom of the plate. 100 µL of the supernatant was carefully pipetted into a 96-well V-bottom plate (NEST, 701211). Cytokine assays for TNFα (PerkinElmer, 62HTNFAPEH) and IFNγ (PerkinElmer, 62HIFNGPEH) were performed according to the instructions of the cytokine detection kit. The remaining 100 µL of the cell suspension was collected into a new 96-well V-bottom plate and centrifuged at 400 g for 3 min. The supernatant was discarded, and 100 µL of PBS containing a PI live/dead dye (Beyotime, ST511) was added to each well to resuspend the cell pellet. After 5 min of incubation at room temperature, the percentage death of the target cells was determined using a flow cytometer.

The results, shown in FIGs. 5A-C, indicate that the anti-LILRB4×CD3 bispecific antibodies (designated as huB1G5-CD3-2VHH and huB1G5-CD3Fab) were still capable of specifically inducing T cells to kill HL-60 tumor cells at a low effector-to-target ratio, and huB1G5-CD3-2VHH had superior killing to huB1G5-CD3Fab. The detection results of the release of inflammatory cytokines TNFα and IFNγ were consistent with the killing.

### 6.2. Killing activity of huB1G5-CD3-2VHH and huB1G5-ScFv-2VHH bispecific antibodies against tumor cells

PBMC cells (Shanghai AoNeng Biotechnology Co., Ltd.) were thawed and adjusted to a cell density of 1-2 × 10⁶ cells/mL using a 1640 + 10% FBS complete medium. The cells were activated overnight with IL2 at a final concentration of 100 IU/mL. The next morning, the PBMC cell suspension was collected, and all T cells were sorted using a T cell isolation kit (Stemcell, Cat# 17951RF) according to the instructions. The cells were centrifuged at 400 g for 5 min, and the supernatant was discarded. The cells were resuspended in PBS and centrifuged at 200 g for 5 min, and the supernatant was discarded. The cells were resuspended in PBS and counted. The CellTrace^{™} Violet cell proliferation reagent dye (Thermo, C34557) was added at a ratio of 1:2000. The mixture was left to stand at 37 °C for 15 min, and 5 volumes of a complete medium were added to stop the staining. The mixture was centrifuged at 200 g for 5 min, and the cells were washed once with 3 mL of the complete medium, resuspended in an assay buffer containing 10% FBS (Gibco, Cat# 10099-141) and 100 IU/mL IL-2 in an RPMI 1640 medium (Gibco, Cat# 22400071), and counted. The T cells were adjusted to a density of 2 × 10⁵ cells/mL, and seeded into a 96-well plate (Corning, Cat# 3599) at 100 µL per well (i.e., 20000 cells per well). The cultured target cells HL-60 were collected and centrifuged at 200 g for 5 min to collect the cells, and the supernatant was discarded. The cells were resuspended in PBS and centrifuged at 200 g for 5 min, and the supernatant was discarded. The cells were resuspended in PBS and counted. The CytoTell red 650 cell proliferation reagent dye (AAT Bioquest, 22255) was added at a ratio of 1:2000. The mixture was left to stand at 37 °C for 15 min, and 5 volumes of a complete medium were added to stop the staining. The mixture was centrifuged at 200 g for 5 min, and the supernatant was discarded. The cells were washed once with 3 mL of the complete medium, resuspended in an assay buffer, and counted. The target cells were adjusted to a density of 2.67 × 10⁵ cells/mL, and the cells were seeded into the above 96-well plate at 75 µL per well (i.e., 20000 cells per well) (effector cell:target cell = 1:1). The test antibodies were prepared at 8-fold the final detection concentration. The maximum detection concentration of the test antibodies was 1 nM. A 5-fold gradient dilution was performed to obtain a total of 9 concentration points, and 25 µL of the test antibody was added to each well. In this study, the minimal killing (the assay buffer was added to the target cells) and natural killing (the effector cells were added to the target cells) controls were set. The final total volume of each well was 200 µL, and the plate was incubated in a cell incubator for another 21 h. 100 µL of the cell suspension was collected into a new 96-well V-bottom plate and centrifuged at 400 g for 3 min. The supernatant was discarded, and 100 µL of PBS containing a PI live/dead dye (Beyotime, ST511) was added to each well to resuspend the cell pellet. After 5 min of incubation at room temperature, the number of remaining live target cells was detected using a flow cytometer, and the target cell lysis percentage was calculated.

The results, shown in FIG. 5D, indicate that the anti-LILRB4×CD3 bispecific antibodies (designated as huB1G5-CD3-2VHH and huB1G5-ScFv-2VHH) and the control antibody 4ab3-4ab S91A were still capable of specifically inducing T cells to kill HL-60 tumor cells at a lower effector-to-target ratio, and the killing of the huB1G5-CD3-2VHH and huB1G5-ScFv-2VHH was stronger than that of **the control antibody.**

### Example 7: In Vivo Efficacy of Anti-LILRB4×CD3 Bispecific Antibodies in hPBMC-B-NDG Mouse MOLM-13 Tumor Model

The *in vivo* anti-tumor effect of the anti-LILRB4×CD3 bispecific antibodies was investigated in an hPBMC-B-NDG mouse MOLM-13 tumor model.

### 7.1. In vivo efficacy of huB1G5-CD3Fab and huBIG5-CD3-2VHH bispecific antibodies

The severe immunodeficiency B-NDG mice were purchased from Biocytogen Pharmaceuticals (Beijing) Co., Ltd., and the human peripheral blood mononuclear cells (PBMCs) were purchased from Shanghai AoNeng Biotechnology Co., Ltd. Human acute myeloid leukemia MOLM-13 cells were purchased from Nanjing Cobioer Biosciences Co., Ltd. The cells were cultured in an incubator containing 5% CO₂ at 37 °C, and the culture medium was an RPMI 1640 medium containing 20% inactivated fetal bovine serum.

30 severe immunodeficiency B-NDG mice were first inoculated with 5 × 10⁶ human peripheral blood mononuclear cells in 0.2 mL of RPMI 1640 per mouse via the tail vein. After 7 days, the mice were subcutaneously implanted with 5 × 10⁶ MOLM-13 cells in 0.1 mL of RPMI 1640 per mouse on the right anterior side. When the mean tumor volume reached about 182.5 mm³, 20 appropriate mice were selected for study and randomly divided into 3 experimental groups of 5 mice each based on mouse tumor volume and PBMC reconstitution. On days 0, 3, and 7 after grouping, the blank control PBS, the huB1G5-CD3Fab bispecific antibody (0.2 mg/kg), and the huB1G5-CD3-2VHH bispecific antibody (0.2 mg/kg) were administered intraperitoneally to the mice, separately. Tumor volume was monitored twice weekly by vernier caliper measurement from the day of grouping and administration.

Treatment with the anti-LILRB4×CD3 bispecific antibodies huB1G5-CD3Fab and huB1G5-CD3-2VHH resulted in significant inhibition of tumor growth as compared to the PBS group, with TGIs of 49.54% and 96.12%, respectively. The results are shown in FIG. 6A and Table 8 below.

**Table 8. Growth inhibition of MOLM-13 tumors by test substances**

| Group | Dose | Number of animals (N) | Tumor volume (mm³)^{a} (day 9 after first administration) | TGI(%)^{b} | P^{c} |
|---|---|---|---|---|---|
| Blank control (PBS) | / | 5 | 2554.61±334.91 | - | / |
| huB1G5-CD3Fab | 0.2 mg/kg | 5 | 1383.86±183.65 | 49.54 | *0.0271 |
| huB1G5-CD3-2VHH | 0.2 mg/kg | 5 | 268.70±131.54 | 96.12 | ***0.000 1 |

| | | | | | |
|---|---|---|---|---|---|
| Note: a. Mean ± standard error; b. TGI = (1 - change in tumor volume of administration group/change in tumor volume of control group) × 100%; c. tumor volume in the administration groups and the PBS control group were statistically analyzed (One-way ANOVA) on day 9 after grouping and administration, *: *P* < 0.05 and ***: *P* < 0.001. | | | | | |

Throughout the course of the experiment, the animals maintained good mobility and feeding status during the administration period, and the body weight of the animals in the G1-G3 groups increased to some extent, indicating that the animals had good tolerance to the test substances. The body weight changes of all animals are shown in FIG. 6B and Table 9.

**Table 9. Effect of test substances on mouse body weight**

| Group | Body weight (g) ^{a} | | | Body weight change (%) after 9 days of administration |
|---|---|---|---|---|
| | Before administration | Day 9 after grouping and administration | p^{b} | |
| Blank control (PBS) | 19.5±0.5 | 21.4±0.7 | | +10.2 |
| huB1G5-CD3Fab | 19.0±0.4 | 20.8±0.5 | 0.49 51 | +9.3 |
| huB1G5-CD3-2VHH | 19.4±0.5 | 19.8±0.5 | 0.01 90 | +2.4 |

| | | | | |
|---|---|---|---|---|
| Note: a. Mean ± standard error; b. body weight in the administration groups and the PBS control group was statistically analyzed (One-way ANOVA) on day 9 after grouping and administration. | | | | |

### 7.2. In vivo efficacy of huB1G5-CD3-2VHH and huB1G5-ScFv-2VHH bispecific antibodies

The severe immunodeficiency B-NDG mice were purchased from Biocytogen Pharmaceuticals (Beijing) Co., Ltd., and the human peripheral blood mononuclear cells (PBMCs) were purchased from Milecell Biotechnology Inc. Human acute myeloid leukemia MOLM-13 cells were purchased from Nanjing Cobioer Biosciences Co., Ltd. The cells were cultured in an incubator containing 5% CO2 at 37 °C, and the culture medium was an RPMI 1640 medium containing 20% inactivated fetal bovine serum.

38 severe immunodeficiency B-NDG mice were first inoculated with 5 × 10⁶ human peripheral blood mononuclear cells in 0.2 mL of RPMI 1640 per mouse via the tail vein. After 7 days, the mice were subcutaneously implanted with 5 × 10⁶ MOLM-13 cells in 0.1 mL of RPMI 1640 per mouse on the right anterior side. When the mean tumor volume reached about 166.64 mm3, 25 appropriate mice were selected for study and randomly divided into 5 experimental groups of 5 mice each based on mouse tumor volume and PBMC reconstitution (hCD 45% proportion). On days 0, 3, and 6 after grouping, the blank control PBS, the huB1G5-ScFv-2VHH (0.2 mg/kg or 2 mg/kg) bispecific antibody, and the huB1G5-CD3-2VHH(0.2 mg/kg or 2 mg/kg) bispecific antibody were administered intraperitoneally to the mice, separately. During the experiment, the tumor volume was monitored twice weekly by vernier caliper measurement.

Treatment with the anti-LILRB4×CD3 bispecific antibodies huB1G5-ScFv-2VHH (0.2 mg/kg and 2 mg/kg) and huB1G5-CD3-2VHH (0.2 mg/kg and 2 mg/kg) resulted in significant inhibition of tumor growth as compared to the PBS group, with TGIs of 72.0% and 63.3%, respectively, for huB1G5-ScFv-2VHH, and 54.2% and 87.6%, respectively, for huB1G5-CD3-2VHH (0.2 mg/kg and 2 mg/kg). The results are shown in FIG. 6C and Table 10 below.

**Table 10. Growth inhibition of MOLM-13 tumors by test substances**

| Group | Dose | Number of animals (N) | Tumor volume (mm³)^{a} (on day 9 after first administration) | TGI(%)^{b} | P^{c} |
|---|---|---|---|---|---|
| Blank control (PBS) | / | 5 | 2452.77±182.33 | | / |
| huB1G5-ScFv-2VHH | 0.2 mg/kg | 5 | 805.35±214.20 | 72.0 | ***0.0003 |
| huB1G5-ScFv-2VHH | 2 mg/kg | 5 | 1016.00±83.44 | 63.3 | **0.0011 |
| huB1G5-CD3-2VHH | 0.2 mg/kg | 5 | 1222.63±384.61 | 54.2 | **0.0046 |
| huB1G5-CD3-2VHH | 2 mg/kg | 5 | 443.43±191.16 | 87.6 | ****<0.0001 |

| | | | | | |
|---|---|---|---|---|---|
| **Note:** a. Mean + standard error; b. TGI = (1 - change in tumor volume of administration group/change in tumor volume of control group) × 100%; c. tumor volume in the administration groups and the PBS control group were statistically analyzed (One-way ANOVA) on day 9 after grouping and administration,**: P < 0.01, ***: *P <* 0.001, and ****: *P* < 0.0001. | | | | | |

Throughout the course of the experiment, the animals maintained good mobility and feeding status during the administration period, the body weight of the animals in the G1-G4 groups increased to some extent, and the body weight of the animals in the G5 group did not significantly decrease, indicating that the animals had good tolerance to the test substances. The body weight changes of all animals are shown in FIG. 6D and Table 11.

**Table 11. Effect of test substances on mouse body weight**

| Group | Body weight (g) ^{a} | | | Body weight change (%) after 9 days of administration |
|---|---|---|---|---|
| | Before administration | Day 9 after grouping and administration | p^{b} | / |
| Blank control (PBS) | 20.2±0.6 | 20.5±0.6 | - | +1.7 |
| huB1G5-ScFv-2VHH | 21.4±0.1 | 21.4±0.4 | 0.6814 | +0.3 |
| huB1G5-ScFv-2VHH | 20.7±0.7 | 21.5±0.6 | 0.6369 | +4.3 |
| huB1G5-CD3-2VHH | 21.1±0.6 | 21.1±0.6 | 0.8799 | +0.3 |
| huB1G5-CD3-2VHH | 19.9±0.4 | 19.8±0.9 | 0.8799 | -0.5 |

| | | | | |
|---|---|---|---|---|
| Note: a. Mean ± standard error; b. body weight in the administration groups and the PBS control group was statistically analyzed (One-way ANOVA) on day 9 after grouping and administration. | | | | |

### Example 8: In Vivo Efficacy of Bispecific Antibodies in MOLM-13-luc Hematological Tumor Model

The *in vivo* anti-tumor effect of the anti-LILRB4×CD3 bispecific antibodies was investigated in an hPBMC-B-NDG mouse MOLM-13-luc hematological tumor model.

The severe immunodeficiency B-NDG mice were purchased from Biocytogen Pharmaceuticals (Beijing) Co., Ltd., and the human peripheral blood mononuclear cells (PBMCs) were purchased from Milecell Biotechnology Inc. Human acute myeloid leukemia MOLM-13-luc cells were purchased from Nanjing Cobioer Biosciences Co., Ltd. The cells were cultured in an incubator containing 5% CO₂ at 37 °C, and the culture medium was an RPMI 1640 medium containing 20% inactivated fetal bovine serum and 1 µg/mL puromycin.

60 severe immunodeficiency B-NDG mice were first inoculated with 5 × 10⁶ human peripheral blood mononuclear cells in 0.2 mL of RPMI 1640 per mouse via the tail vein. After 7 days, the mice were inoculated with 2 × 10⁶ MOLM-13-luc cells in 0.2 mL of RPMI 1640 per mouse via the tail vein. 40 appropriate mice were selected for study and randomly divided into 8 experimental groups of 5 mice each based on the *in vivo* imaging results of the mice and the PBMC reconstruction (hCD 45% proportion). On days 0, 3, 7, 10, and 14 after grouping, the blank control PBS, the 4ab3-4ab S91A (0.2 mg/kg) bispecific antibody, the huB1G5-ScFv-2VHH (0.02 mg/kg, 0.2 mg/kg, or 2 mg/kg) bispecific antibody, and the huB1G5-CD3-2VHH (0.02 mg/kg, 0.2 mg/kg, or 2 mg/kg) bispecific antibody were administered intraperitoneally to the mice, separately. The animal status was observed daily during the experiment, and the animals were subjected to *in vivo* imaging with the abdomen facing up on day 8 and day 13.

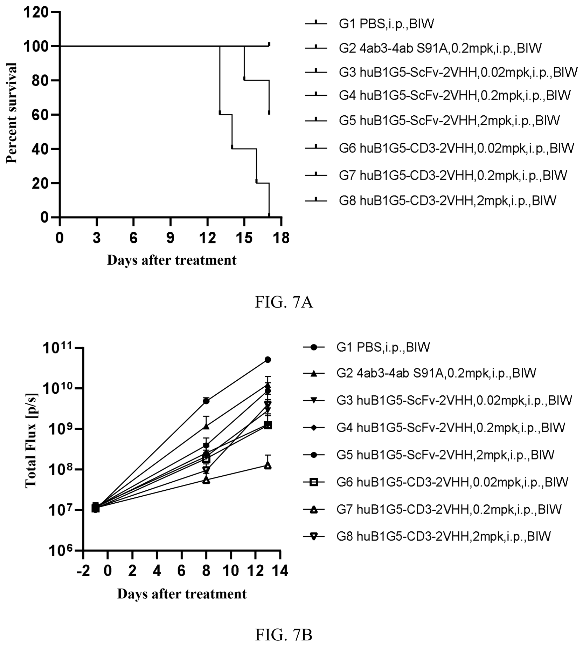

Treatment with the anti-LILRB4×CD3 bispecific antibodies 4ab3-4ab S91A (0.2 mg/kg), huB1G5-ScFv-2VHH (0.02 mg/kg, 0.2 mg/kg, and 2 mg/kg), and huB1G5-CD3-2VHH(0.02 mg/kg, 0.2 mg/kg, and 2 mg/kg) resulted in prolonged survival of the animals as compared to the PBS group. The results are shown in FIG. 7A and Table 12 below.

**Table 12. Effect of test substances on survival of animals**

| Group | Dose | Number of animals (N) | Number of surviving animals (on day 17 after first administration) |
|---|---|---|---|
| Blank control (PBS) | / | 5 | 0 |
| 4ab3-4ab S91A | 0.2 mg/kg | 5 | 3 |
| huB1G5-ScFv-2VHH | 0.02 mg/kg | 5 | 5 |
| huB1G5-ScFv-2VHH | 0.2 mg/kg | 5 | 5 |
| huB1G5-ScFv-2VHH | 2 mg/kg | 5 | 5 |
| huB1G5-CD3-2VHH | 0.02 mg/kg | 5 | 5 |
| huB1G5-CD3-2VHH | 0.2 mg/kg | 5 | 5 |
| huB1G5-CD3-2VHH | 2 mg/kg | 5 | 5 |

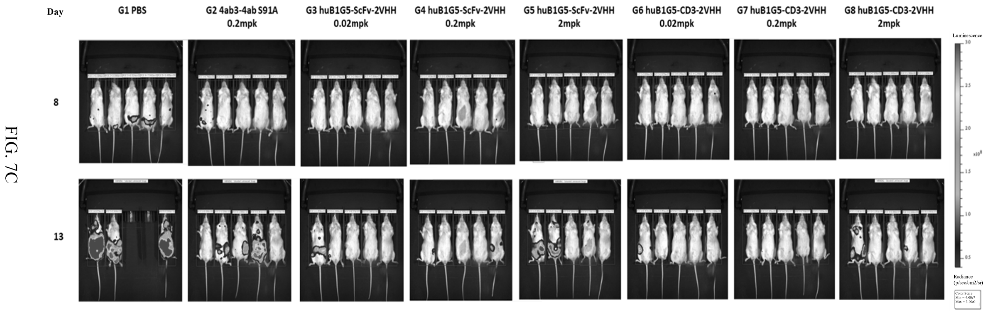

The groups treated with the anti-LILRB4×CD3 bispecific antibodies 4ab3-4ab S91A (0.2 mg/kg), huB1G5-ScFv-2VHH (0.02 mg/kg, 0.2 mg/kg, or 2 mg/kg), and huB1G5-CD3-2VHH (0.02 mg/kg, 0.2 mg/kg, or 2 mg/kg) showed a significant reduction in fluorescence signals as compared to the PBS group. The *in vivo* imaging fluorescence signals of all animals are shown in FIGs. 7B and 7C and Table 13.

**Table 13. Effect of test substances on in vivo imaging fluorescence signals of mice**

| Group | Fluorescence signal (p/s) ^{a} | | |
|---|---|---|---|
| | Before administration | Day 13 after grouping and administration | p^{b} |
| Blank control (PBS) | 1.02E7±2.10E6 | 5.14E10±7.20E9 | - |
| 4ab3-4ab S91A | 1.15E7±2.27E6 | 1.26E10±7.09E9 | ****<0.0001 |
| huB1G5-ScFv-2VHH | 1.22E7±3.13E6 | 2.87E9±2.50E9 | ****<0.0001 |
| huB1G5-ScFv-2VHH | 1.17E7±2.73E6 | 1.30E9±8.52E8 | ****<0.0001 |
| huB1G5-ScFv-2VHH | 1.18E7±2.36E6 | 8.77E9±4.99E9 | ****<0.0001 |
| huB1G5-CD3-2VHH | 1.10E7±2.44E6 | 1.23E9±1.13E9 | ****<0.0001 |
| huB1G5-CD3-2VHH | 1.15E7±3.15E6 | 1.29E8±9.74E7 | ****<0.0001 |
| huB1G5-CD3-2VHH | 1.15E7±1.97E6 | 4.00E9±3.17E9 | ****<0.0001 |

| | | | |
|---|---|---|---|
| Note: a. Mean ± standard error; b. fluorescence signals in the administration groups and the PBS control group were statistically analyzed (One-way ANOVA) on day 13 after grouping and administration. | | | |

### Example 9: MLR Function of Bispecific Antibodies

Whether the anti-LILRB4×CD3 bispecific antibodies in the present disclosure could activate T cells was determined by detecting the release of IL-2 and IFN-γ in the mixed lymphocyte system and the proliferation of T cells.

Cryopreserved human PBMCs (purchased from Shanghai AoNeng Biotechnology Co., Ltd., Cat. No.: PBMNC050C, batch: 2108040345) were taken out from a liquid nitrogen tank, rapidly thawed in a water bath at 37 °C, and centrifuged to obtain a cell pellet. Pure monocytes were isolated using a human total monocyte isolation kit (Miltenyi, Cat. No.: 130-096-537) according to the instructions and cultured in a 1640 complete medium containing 10% fetal bovine serum, 100 ng/mL recombinant human GM-CSF (PeproTech, Cat. No.: 00-03-100UG), and 10 ng/mL recombinant human IL-4 (PeproTech, Cat. No.: 200-04-100UG) for 5 days to induce the differentiation of monocytes into imDCs. The induced imDCs were collected and centrifuged at room temperature at 400× g for 5 min, and the supernatant was discarded. The cell pellet was cultured in a complete medium containing 100 ng/mL lipopolysaccharide (LPS, Sigma, Cat. No.: LPS25) for 2 days to promote DC maturation. The DCs were collected, resuspended in a complete medium, counted, adjusted to a density of 2 × 10⁵ cells/mL, and seeded into a 96-well U-bottom plate (Corning, Cat. No.: 3799) at 1 × 10⁴ cells/50 µL/well. Cryopreserved human PBMCs (purchased from Shanghai Schbio Biotech Co. Ltd., Cat. No.: PBMNC050C, batch: 2301310011) were taken out from a liquid nitrogen tank, rapidly thawed in a water bath at 37 °C, and centrifuged to obtain a cell pellet. Pure T cells were obtained by isolation using an EasySep^{™} human T cell sorting kit (Stem, Cat. No.: 17951) according to the instructions. T cells were stained with CFSE (BioTracker 488 green CSFE cell proliferation kit, Sigma, Cat. No.: C34557), then resuspended in a 1640 complete medium containing 10% fetal bovine serum, adjusted to a density of 1 × 10⁶ cells/mL, seeded into a 96-well U-bottom plate at 1 × 10⁵ cells/100 µL/well, and well mixed with induced DCs (effector cell:target cell = 10:1).

The anti-LILRB4×CD3 bispecific antibodies of the present disclosure were subjected to gradient dilution with a 1640 complete medium (at an initial concentration of 1000 nM, and 5-fold gradient dilution was performed for a total of 8 concentration points) and inoculated at 50 µL/well. The mixture was mixed well and then incubated at 37 °C. The IL-2 content in the supernatant after 2 days of incubation was detected using a human IL2 detection kit @HTRF (Cisbio, Cat. No.: 62HIL02PEH) according to the instructions. The IFNγ content in the supernatant after 5 days of incubation was detected using a human IFNγ detection kit @HTRF (Cisbio, Cat. No.: 62HIFNGPEH) according to the instructions. The cell pellet was collected, and the change in the number of CFSE-positive cells was detected by absolute counting by flow cytometry.

The results shown in FIG. 8C show that the anti-LILRB4×CD3 bispecific antibodies of the present disclosure (designated as huB1G5-CD3-2VHH and huB1G5-ScFv-2VHH) can promote the proliferation of T cells in a DC-T mixed lymphocyte reaction system, and the amount of cell proliferation was in a dose-dependent relationship with the antibody concentration. The difference between the two bispecific antibodies of the present disclosure was not significant. The results shown in FIGs. 8A and 8B show that the anti-LILRB4×CD3 bispecific antibodies of the present disclosure (designated as huB1G5-CD3-2VHH and huB1G5-ScFv-2VHH) can promote the release of IL-2 and IFN-γ in a DC-T mixed lymphocyte reaction system, and the release amount is in a dose-dependent relationship with the antibody concentration.

### Example 10: Reduction of IL-10 Release in PBMCs by Anti-LILRB4 Antibodies

Whether the anti-LILRB4 humanized antibodies in the present disclosure could promote the differentiation of myeloid cells in PBMCs to a pro-inflammatory phenotype was determined by measuring the release level of IL-10 in the PBMC system.

Cryopreserved human PBMCs (purchased from Shanghai Saily & Xuanfeng Biotechnology Co., Ltd., Cat. No.: HFB-HP050B, batch: W20210078) were taken out from a liquid nitrogen tank, rapidly thawed in a water bath at 37 °C, and centrifuged to obtain a cell pellet. The cells were resuspended in a 1640 (Gibco, Cat. No.: 22400089) complete medium containing 10% fetal bovine serum (Gibco, Cat. No.: 10099-141), sampled, counted, and seeded into the experimental wells of a 96-well culture plate (Corning, Cat. No.: 3599) at 1.5 × 10⁵ cells/100 µL. The CD3 monoclonal antibody (OKT3, Invitrogen, Cat. No.: 16-0037-85) was diluted with a complete medium and added at 50 µL/well to a final concentration of 10 ng/mL, followed by activation at 37 °C for 6 h. The anti-human LILRB4 humanized antibody, the control antibody h193, and a negative control antibody (IgG4 control, wherein IgG1 control was purchased from SinoBiological, Cat. No.: HG1K) in the present disclosure were diluted with a complete medium to 400 nM, 40 nM, and 4 nM, respectively, and added at 50 µL/well, with the final concentrations of the system being 100 nM, 10 nM, and 1 nM. After the mixture was mixed well, the culture plate was incubated in an incubator at 37 °C for 3 days. The IL-10 content in the supernatant was detected using an IL-10 detection kit (R&D, Cat. No.: DY217B).

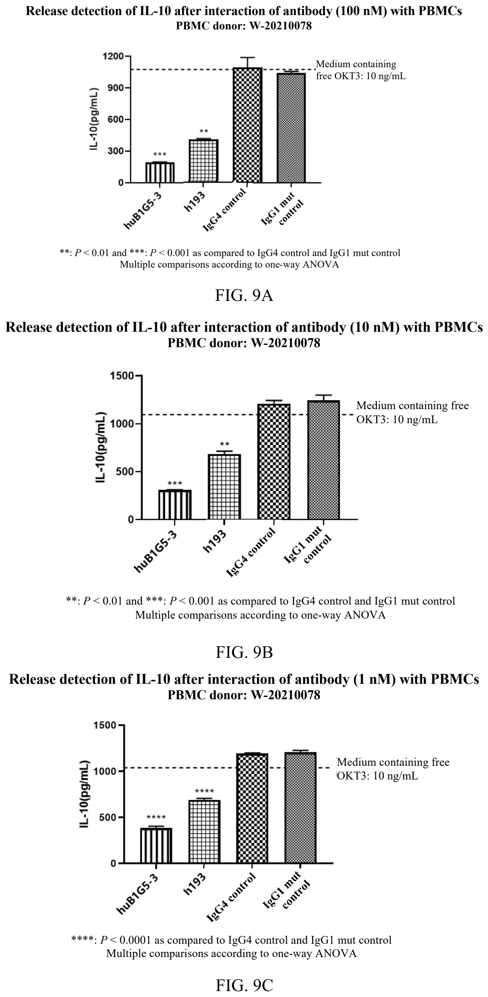

The results shown in FIGs. 9A, 9B, and 9C show that the anti-human LILRB4 humanized antibody (designated as huB1G5-3) and the antibody h193 of the present disclosure were both capable of extremely significantly reducing the IL-10 secretion in the system at three assay concentrations as compared to the two negative controls. It is shown that the anti-human LILRB4 humanized antibody in the present disclosure can promote the differentiation of myeloid cells in PBMCs to a pro-inflammatory phenotype.

All publications, patent applications, patents, and other references mentioned herein are incorporated by reference in their entirety. Any or all of the features described above and throughout the present application may be combined in various embodiments of the present disclosure. In addition, the materials, methods, and examples described herein are illustrative only and are not intended to be limiting. Other features, objectives, and advantages of the present disclosure will be apparent from this description and drawings and from the appended claims.

### Sequence information:

| SEQ ID NO | | (CDRs of the CD3 antibody were determined using Kabat, and CDRs of the LILRB4 antibody were determined using IMGT; the gray portions are sequences in the constant regions) |
|---|---|---|
| 1 | SP34 antibody humanized antibody heavy chain hu34H1 | EVQLVESGGGLVQPGGSLKLSCAASGFTFSTYAMNWVRQAPGKGLEWVGRIRSKYNNYAT YYADSVKDRFTISRDDSKNTLYLQMNSLKTEDTAVYYCTRHGNFGNSYVSWFAYWGQGTT VTVSSASTKGPSVFPLAPSSKSTSGGTAALGCLVKDYFPEPVTVSWNSGALTSGVHTFPAVL QSSGLYSLSSVVTVPSSSLGTQTYICNVNHKPSNTKVDKKVEPKSCDKTHTCPPCPAPEAAG GPSVFLFPPKPKDTLMISRTPEVTCVVVAVSHEDPEVKFNWYVDGVEVHNAKTKPREEQYN STYRVVSVLTVLHQDWLNGKEYKCKVSNKALAAPIEKTISKAKGQPREPQVYTLPPSRDEL TKNQVSLTCLVKGFYPSDIAVEWESNGQPENNYKTTPPVLDSDGSFFLYSKLTVDKSRWQQ GNVFSCSVMHEALHNHYTQKSLSLSPGK |
| 2 | SP34 antibody humanized antibody light chain hu34L2 | QAVVTQEPSLTVSPGGTVTLTCRSSTGAVTTSNYANWVQQKPGQAPRGLIGGTNKRAPGVP ARFSGSLLGGKAALTLSGAQPEDEAEYFCALWYSNLWVFGGGTKLTVLGQPKAAPSVTLF PPSSEELQANKATLVCLISDFYPGAVTVAWKADSSPVKAGVETTTPSKQSNNKYAASSYLSL TPEQWKSHRSYSCQVTHEGSTVEKTVAPTEC |
| 3 | SP34 antibody humanized antibody heavy chain variable region hu34H1-VH | EVQLVESGGGLVQPGGSLKLSCAASGFTFSTYAMNWVRQAPGKGLEWVGRIRSKYNNYAT YYADSVKDRFTISRDDSKNTLYLQMNSLKTEDTAVYYCTRHGNFGNSYVSWFAYWGQGTT VTVSS |
| 4 | SP34 antibody humanized antibody light chain variable region hu34L2-VL | QAVVTQEPSLTVSPGGTVTLTCRSSTGAVTTSNYANWVQQKPGQAPRGLIGGTNKRAPGVP ARFSGSLLGGKAALTLSGAQPEDEAEYFCALWYSNLWVFGGGTKLTVL |
| 5 | SP34 antibody humanized antibody HCDR1 | TYAMN |
| 6 | SP34 antibody humanized antibody HCDR2 | RIRSKYNNYATYYADSVKD |
| 7 | SP34 antibody humanized antibody HCDR3 | HGNFGNSYVSWFAY |
| 8 | SP34 antibody humanized antibody LCDRl | RSSTGAVTTSNYAN |
| 9 | SP34 antibody humanized antibody LCDR2 | GTNKRAP |
| 10 | SP34 antibody humanized antibody LCDR3 | ALWYSNLWV |
| 11 | Camelid-derived anti-LILRB4 VHH B1A2 | QVQLVESGGGLVQPGGSLRLSCAASGFTFSSYTMSWVRQAPGKGLEWISDINNDGGKTYY AFSVEGRFTISRDNAKNTLYLQMNSLKPEDTAVYYCVRDHQYTSRNGDRGQGTQVTVSS |
| 12 | Camelid-derived anti-LILRB4 VHH B1A2-HCDR1 | GFTFSSYT |
| 13 | Camelid-derived anti-LILRB4 VHH B1A2-HCDR2 | INNDGGKT |
| 14 | Camelid-derived anti-LILRB4 VHH B1A2-HCDR3 | VRDHQYTSRNGD |
| 15 | Camelid-derived anti-LILRB4 VHH B1A2-Fc (camelid-derived anti-LILRB4 HC) | QVQLVESGGGLVQPGGSLRLSCAASGFTFSSYTMSWVRQAPGKGLEWISDINNDGGKTYY AFSVEGRFTISRDNAKNTLYLQMNSLKPEDTAVYYCVRDHQYTSRNGDRGQGTQVTVSS |
| | | DKTHTCPPCPAPELLGGPSVFLFPPKPKDTLMISRTPEVTCVVVDVSHEDPEVKFNWYVDG VEVHNAKTKPREEQYNSTYRVVSVLTVLHQDWLNGKEYKCKVSNKALPAPIEKTISKAKG QPREPQVYTLPPSRDELTKNQVSLTCLVKGFYPSDIAVEWESNGQPENNYKTTPPVLDSDGS FFLYSKLTVDKSRWQQGNVFSCSVMHEALHNHYTQKSLSLSPGK |
| 16 | Camelid-derived anti-LILRB4 VHH B1G5 | EVQLVESGGGLVQAGGSLRLSCAASESIFSSNVMGWYRQSPGHQRELVAAVGINGLTKIGDS VKGRFTISRDNAKNTVYLQMNSLKVEDTAVYYCAAGAYVRFLQNSPDYWGQGTQVTVSS |
| 17 | Camelid-derived anti-LILRB4 VHH B1G5-HCDR1 | ESIFSSNV |
| 18 | Camelid-derived anti-LILRB4 VHH B1G5-HCDR2 | VGINGLT |
| 19 | Camelid-derived anti-LILRB4 VHH B1G5-HCDR3 | AAGAYVRFLQNSPDY |
| 20 | Camelid-derived anti-LILRB4 VHH B1G5-Fc (camelid-derived anti-LILRB4 HC) | EVQLVESGGGLVQAGGSLRLSCAASESIFSSNVMGWYRQSPGHQRELVAAVGINGLTKIGDS VKGRFTISRDNAKNTVYLQMNSLKVEDTAVYYCAAGAYVRFLQNSPDYWGQGTQVTVSS |
| | | DKTHTCPPCPAPELLGGPSVFLFPPKPKDTLMISRTPEVTCVVVDVSHEDPEVKFNWYVDG VEVHNAKTKPREEQYNSTYRVVSVLTVLHQDWLNGKEYKCKVSNKALPAPIEKTISKAKG QPREPQVYTLPPSRDELTKNQVSLTCLVKGFYPSDIAVEWESNGQPENNYKTTPPVLDSDGS FFLYSKLTVDKSRWQQGNVFSCSVMHEALHNHYTQKSLSLSPGK |
| 21 | Camelid-derived anti-LILRB4 VHH 1H12 | EVQVVESGGGLVQAGGSLRLSCAASETIFSSNVMGWYRQAHGKQRELVAATGINGVTKYA DSVKGRFTISRDNAKNTVYLQMNSLNVEDTAVYYCAAGVWRNFVMRSPDYWGQGTQVT VSS |
| 75 | Camelid-derived anti-LILRB4 VHH 1H12-HCDR1 | ETlFSSNV |
| 22 | Camelid-derived anti-LILRB4 VHH 1H12-HCDR2 | TGINGVT |
| 23 | Camelid-derived anti-LILRB4 VHH 1H12-HCDR3 | AAGVWRNFVMRSPDY |
| 24 | Camelid-derived anti-LILRB4 VHH 1H12-Fc (camelid-derived anti-LILRB4 HC) | EVQVVESGGGLVQAGGSLRLSCAASETIFSSNVMGWYRQAHGKQRELVAATGINGVTKYA DSVKGRFTISRDNAKNTVYLQMNSLNVEDTAVYYCAAGVWRNFVMRSPDYWGQGTQVT VSS |
| | | DKTHTCPPCPAPELLGGPSVFLFPPKPKDTLMISRTPEVTCVVVDVSHEDPEVKFNWYVDG VEVHNAKTKPREEQYNSTYRVVSVLTVLHQDWLNGKEYKCKVSNKALPAPIEKTISKAKG QPREPQVYTLPPSRDELTKNQVSLTCLVKGFYPSDIAVEWESNGQPENNYKTTPPVLDSDGS FFLYSKLTVDKSRWQQGNVFSCSVMHEALHNHYTQKSLSLSPGK |
| 25 | Camelid-derived anti-LILRB4 VHH 2A3 | EVQVVESGGGSVQTGGSLTLSCAASGRTFRSNGMGWFRQAPGKEREFVAAIRWSGRRTTY TDSVKGRFTISSDSAKNTVYLQMSSLKPEDTAVYYCAVDLLPPYGALDKYDYWGQGTQVT VSS |
| 26 | Camelid-derived anti-LILRB4 VHH 2A3-HCDR1 | GRTFRSNG |
| 27 | Camelid-derived anti-LILRB4 VHH 2A3-HCDR2 | IRWSGRRT |
| 28 | Camelid-derived anti-LILRB4 VHH 2A3-HCDR3 | AVDLLPPYGALDKYDY |
| 29 | Camelid-derived anti-LILRB4 VHH 2A3-Fc (camelid-derived anti-LILRB4 HC) | EVQVVESGGGSVQTGGSLTLSCAASGRTFRSNGMGWFRQAPGKEREFVAAIRWSGRRTTY TDSVKGRFTISSDSAKNTVYLQMSSLKPEDTAVYYCAVDLLPPYGALDKYDYWGQGTQVT VSS |
| | | DKTHTCPPCPAPELLGGPSVFLFPPKPKDTLMISRTPEVTCVVVDVSHEDPEVKFNWYVDG VEVHNAKTKPREEQYNSTYRVVSVLTVLHQDWLNGKEYKCKVSNKALPAPIEKTISKAKG QPREPQVYTLPPSRDELTKNQVSLTCLVKGFYPSDIAVEWESNGQPENNYKTTPPVLDSDGS FFLYSKLTVDKSRWQQGNVFSCSVMHEALHNHYTQKSLSLSPGK |
| 30 | Humanized anti-LILRB4 VHH huB1G5-1 | EVQLVESGGGLVQPGGSLRLSCAASESIFSSNVMGWYRQAPGKQRELVAAVGINGLTKIGDS VKGRFTISRDNAKNSVYLQMNSLRAEDTAVYYCAAGAYVRFLQNSPDYWGQGTQVTVSS |
| 17 | Humanized anti-LILRB4 VHH huB1G5-1-HCDR1 | SEQ ID NO:17 |
| 18 | Humanized anti-LILRB4 VHH huB1G5-1-HCDR2 | SEQ ID NO:18 |
| 19 | Humanized anti-LILRB4 VHH huB1G5-1-HCDR3 | SEQ ID NO:19 |
| 31 | Humanized anti-LILRB4 VHH huB1G5-1-Fc (humanized anti-LILRB4 HC) | EVQLVESGGGLVQPGGSLRLSCAASESIFSSNVMGWYRQAPGKQRELVAAVGINGLTKIGDS VKGRFTISRDNAKNSVYLQMNSLRAEDTAVYYCAAGAYVRFLQNSPDYWGQGTQVTVSS |
| | | DKTHTCPPCPAPELLGGPSVFLFPPKPKDTLMISRTPEVTCVVVDVSHEDPEVKFNWYVDG VEVHNAKTKPREEQYNSTYRVVSVLTVLHQDWLNGKEYKCKVSNKALPAPIEKTISKAKG QPREPQVYTLPPSRDELTKNQVSLTCLVKGFYPSDIAVEWESNGQPENNYKTTPPVLDSDGS FFLYSKLTVDKSRWQQGNVFSCSVMHEALHNHYTQKSLSLSPGK |
| 32 | Humanized anti-LILRB4 VHH huB1G5-2 | EVQLVESGGGLVQPGGSLRLSCAASESIFSSNVMGWYRQAPGKQRELVAAVGINRLTKIGDS VKGRFTISRDNAKNSVYLQMNSLRAEDTAVYYCAAGAYVRFLQQSPDYWGQGTQVTVSS |
| 17 | Humanized anti-LILRB4 VHH huB1G5-2-HCDR1 | SEQ ID NO:17 |
| 33 | Humanized anti-LILRB4 VHH huB1G5-2-HCDR2 | VGINRLT |
| 34 | Humanized anti-LILRB4 VHH huB1G5-2-HCDR3 | AAGAYVRFLQQSPDY |
| 35 | Humanized anti-LILRB4 VHH huB1G5-2-Fc (humanized anti-LILRB4 HC) | EVQLVESGGGLVQPGGSLRLSCAASESIFSSNVMGWYRQAPGKQRELVAAVGINRLTKIGDS VKGRFTISRDNAKNSVYLQMNSLRAEDTAVYYCAAGAYVRFLQQSPDYWGQGTQVTVSS |
| | | DKTHTCPPCPAPELLGGPSVFLFPPKPKDTLMISRTPEVTCVVVDVSHEDPEVKFNWYVDG VEVHNAKTKPREEQYNSTYRVVSVLTVLHQDWLNGKEYKCKVSNKALPAPIEKTISKAKG QPREPQVYTLPPSRDELTKNQVSLTCLVKGFYPSDIAVEWESNGQPENNYKTTPPVLDSDGS FFLYSKLTVDKSRWQQGNVFSCSVMHEALHNHYTQKSLSLSPGK |
| 36 | Humanized anti-LILRB4 VHH huB1G5-3 | EVQLVESGGGLVQPGGSLRLSCAASESIFSSNVMGWYRQAPGKQRELVAAVGIQGLTKIGDS VKGRFTISRDNAKNSVYLQMNSLRAEDTAVYYCAAGAYVRFLQQSPDYWGQGTQVTVSS |
| 17 | Humanized anti-LILRB4 VHH huB1G5-3-HCDR1 | SEQ ID NO:17 |
| 37 | Humanized anti-LILRB4 VHH huB1G5-3-HCDR2 | VGIQGLT |
| 34 | Humanized anti-LILRB4 VHH huB1G5-3-HCDR3 | SEQ ID NO:34 |
| 38 | Humanized anti-LILRB4 VHH huB1G5-3-Fc (humanized anti-LILRB4 HC) | EVQLVESGGGLVQPGGSLRLSCAASESIFSSNVMGWYRQAPGKQRELVAAVGIQGLTKIGDS VKGRFTISRDNAKNSVYLQMNSLRAEDTAVYYCAAGAYVRFLQQSPDYWGQGTQVTVSS |
| | | DKTHTCPPCPAPELLGGPSVFLFPPKPKDTLMISRTPEVTCVVVDVSHEDPEVKFNWYVDG VEVHNAKTKPREEQYNSTYRVVSVLTVLHQDWLNGKEYKCKVSNKALPAPIEKTISKAKG QPREPQVYTLPPSRDELTKNQVSLTCLVKGFYPSDIAVEWESNGQPENNYKTTPPVLDSDGS FFLYSKLTVDKSRWQQGNVFSCSVMHEALHNHYTQKSLSLSPGK |
| 39 | Humanized anti-LILRB4 VHH huB1G5-4 | EVQLVESGGGLVQPGGSLRLSCAASESIFSSNVMGWYRQAPGKQRELVAAVGINRLTKIGDS VKGRFTISRDNAKNSVYLQMNSLRAEDTAVYYCAAGAYVRFLQYSPDYWGQGTQVTVSS |
| 17 | Humanized anti-LILRB4 VHH huB1G5-4-HCDR1 | SEQ ID NO:17 |
| 33 | Humanized anti-LILRB4 VHH huB1G5-2-HCDR2 | SEQ ID NO:33 |
| 40 | Humanized anti-LILRB4 VHH huB1G5-2-HCDR3 | AAGAYVRFLQYSPDY |
| 41 | Humanized anti-LILRB4 VHH huB1G5-4-Fc (humanized anti-LILRB4 HC) | EVQLVESGGGLVQPGGSLRLSCAASESIFSSNVMGWYRQAPGKQRELVAAVGINRLTKIGDS VKGRFTISRDNAKNSVYLQMNSLRAEDTAVYYCAAGAYVRFLQYSPDYWGQGTQVTVSS |
| | | DKTHTCPPCPAPELLGGPSVFLFPPKPKDTLMISRTPEVTCVVVDVSHEDPEVKFNWYVDG VEVHNAKTKPREEQYNSTYRVVSVLTVLHQDWLNGKEYKCKVSNKALPAPIEKTISKAKG QPREPQVYTLPPSRDELTKNQVSLTCLVKGFYPSDIAVEWESNGQPENNYKTTPPVLDSDGS FFLYSKLTVDKSRWQQGNVFSCSVMHEALHNHYTQKSLSLSPGK |
| 42 | Humanized anti-LILRB4 VHH huB1G5-5 | EVQLVESGGGLVQPGGSLRLSCAASESIFSSNVMGWYRQAPGKQRELVAAVGIQGLTKIGDS VKGRFTISRDNAKNSVYLQMNSLRAEDTAVYYCAAGAYVRFLQYSPDYWGQGTQVTVSS |
| 17 | Humanized anti-LILRB4 VHH huB1G5-5 -HCDR1 | SEQ ID NO:17 |
| 37 | Humanized anti-LILRB4 VHH huB1G5-5 -HCDR2 | SEQ ID NO:37 |
| 40 | Humanized anti-LILRB4 VHH huB1G5-5 -HCDR3 | SEQ ID NO:40 |
| 43 | Humanized anti-LILRB4 VHH huB1G5-5 -Fc (humanized anti-LILRB4 HC) | EVQLVESGGGLVQPGGSLRLSCAASESIFSSNVMGWYRQAPGKQRELVAAVGIQGLTKIGDS VKGRFTISRDNAKNSVYLQMNSLRAEDTAVYYCAAGAYVRFLQYSPDYWGQGTQVTVSS DKTHTCPPCPAPELLGGPSVFLFPPKPKDTLMISRTPEVTCVVVDVSHEDPEVKFNWYVDG VEVHNAKTKPREEQYNSTYRVVSVLTVLHQDWLNGKEYKCKVSNKALPAPIEKTISKAKG QPREPQVYTLPPSRDELTKNQVSLTCLVKGFYPSDIAVEWESNGQPENNYKTTPPVLDSDGS FFLYSKLTVDKSRWQQGNVFSCSVMHEALHNHYTQKSLSLSPGK |
| 44 | Bispecific antibody huB1G5-CD3Fab CD3Fab heavy chain-knob | EVQLVESGGGLVQPGGSLKLSCAASGFTFSTYAMNWVRQAPGKGLEWVGRIRSKYNNYAT YYADSVKDRFTISRDDSKNTLYLQMNSLKTEDTAVYYCTRHGNFGNSYVSWFAYWGQGTT VTVSS ASTKGPSVFPLAPSSKSTSGGTAALGCLVKDYFPEPVTVSWNSGALTSGVHTFPAVLQSSGL YSLSSVVTVPSSSLGTQTYICNVNHKPSNTKVDKKVEPKSCDKTHTCPPCPAPEAAGGPSVF LFPPKPKDTLMISRTPEVTCVVVAVSHEDPEVKFNWYVDGVEVHNAKTKPREEQYNSTYR VVSVLTVLHQDWLNGKEYKCKVSNKALAAPIEKTISKAKGQPREPQVYTLPPCRDELTKN QVSLWCLVKGFYPSDIAVEWESNGQPENNYKTTPPVLDSDGSFFLYSKLTVDKSRWQQGN VFSCSVMHEALHNHYTQKSLSLSPGK |
| 2 | Bispecific antibody huB1G5-CD3Fab CD3Fab light chain | SEQ ID NO:2 |
| 45 | Bispecific antibody huB1G5-CD3Fab huB1G5-hole | EVQLVESGGGLVQPGGSLRLSCAASESIFSSNVMGWYRQAPGKQRELVAAVGIQGLTKIGDS VKGRFTISRDNAKNSVYLQMNSLRAEDTAVYYCAAGAYVRFLQQSPDYWGQGTQVTVSS |
| | | DKTHTCPPCPAPEAAGGPSVFLFPPKPKDTLMISRTPEVTCVVVAVSHEDPEVKFNWYVDG VEVHNAKTKPREEQYNSTYRVVSVLTVLHQDWLNGKEYKCKVSNKALAAPIEKTISKAKG QPREPQVCTLPPSRDELTKNQVSLSCAVKGFYPSDIAVEWESNGQPENNYKTTPPVLDSDGS FFLVSKLTVDKSRWQQGNVFSCSVMHEALHNHYTQKSLSLSPGK |
| 46 | Bispecific antibody huB1G5-CD3-2VHH huB1G5-CD3Fab heavy chain-knob | EVQLVESGGGLVQPGGSLRLSCAASESIFSSNVMGWYRQAPGKQRELVAAVGIQGLTKIGDS VKGRFTISRDNAKNSVYLQMNSLRAEDTAVYYCAAGAYVRFLQQSPDYWGQGTQVTVSS GGGGS |
| | | EVQLVESGGGLVQPGGSLKLSCAASGFTFSTYAMNWVRQAPGKGLEWVGRIRSKYNNYAT YYADSVKDRFTISRDDSKNTLYLQMNSLKTEDTAVYYCTRHGNFGNSYVSWFAYWGQGTT VTVSS |
| | | ASTKGPSVFPLAPSSKSTSGGTAALGCLVKDYFPEPVTVSWNSGALTSGVHTFPAVLQSSGL YSLSSVVTVPSSSLGTQTYICNVNHKPSNTKVDKKVEPKSCDKTHTCPPCPAPEAAGGPSVF LFPPKPKDTLMISRTPEVTCVVVAVSHEDPEVKFNWYVDGVEVHNAKTKPREEQYNSTYR VVSVLTVLHQDWLNGKEYKCKVSNKALAAPIEKTISKAKGQPREPQVYTLPPCRDELTKN QVSLWCLVKGFYPSDIAVEWESNGQPENNYKTTPPVLDSDGSFFLYSKLTVDKSRWQQGN VFSCSVMHEALHNHYTQKSLSLSPGK |
| 2 | Bispecific antibody huB1G5-CD3-2VHH CD3Fab light chain | SEQ ID NO:2 |
| 45 | Bispecific antibody huB1G5-CD3-2VHH huB1G5-hole | SEQ ID NO:45 |
| 47 | Bispecific antibody Fc-hole (CH2-CH3hole) | DKTHTCPPCPAPEAAGGPSVFLFPPKPKDTLMISRTPEVTCVVVAVSHEDPEVKFNWYVDG VEVHNAKTKPREEQYNSTYRVVSVLTVLHQDWLNGKEYKCKVSNKALAAPIEKTISKAKG QPREPQVCTLPPSRDELTKNQVSLSCAVKGFYPSDIAVEWESNGQPENNYKTTPPVLDSDGS FFLVSKLTVDKSRWQQGNVFSCSVMHEALHNHYTQKSLSLSPGK |
| 48 | Bispecific antibody Fc-hole (hinge region-CH2-CH3 hole) | DKTHTCPPCPAPEAAGGPSVFLFPPKPKDTLMISRTPEVTCVVVAVSHEDPEVKFNW YVDGVEVHNAKTKPREEQYNSTYRVVSVLTVLHQDWLNGKEYKCKVSNKALAAPIEKTIS KAKGQPREPQVCTLPPSRDELTKNQVSLSCAVKGFYPSDIAVEWESNGQPENNYKTTPPVL DSDGSFFLVSKLTVDKSRWQQGNVFSCSVMHEALHNHYTQKSLSLSPGK |
| 49 | Bispecific antibody Fc-knob (hinge region-CH2-CH3knob) | EPKSCDKTHTCPPCPAPEAAGGPSVFLFPPKPKDTLMISRTPEVTCVVVAVSHEDPEVKFNW YVDGVEVHNAKTKPREEQYNSTYRVVSVLTVLHQDWLNGKEYKCKVSNKALAAPIEKTIS KAKGQPREPQVYTLPPCRDELTKNQVSLWCLVKGFYPSDIAVEWESNGQPENNYKTTPPVL DSDGSFFLYSKLTVDKSRWQQGNVFSCSVMHEALHNHYTQKSLSLSPGK |
| 50 | Bispecific antibody Fc-knob (CH2-CH3knob) | DKTHTCPPCPAPEAAGGPSVFLFPPKPKDTLMISRTPEVTCVVVAVSHEDPEVKFNWYVDG VEVHNAKTKPREEQYNSTYRVVSVLTVLHQDWLNGKEYKCKVSNKALAAPIEKTISKAKG QPREPQVYTLPPCRDELTKNQVSLWCLVKGFYPSDIAVEWESNGQPENNYKTTPPVLDSDG SFFLYSKLTVDKSRWQQGNVFSCSVMHEALHNHYTQKSLSLSPGK |
| 51 | CH1 | ASTKGPSVFPLAPSSKSTSGGTAALGCLVKDYFPEPVTVSWNSGALTSGVHTFPAVLQSSGL YSLSSVVTVPSSSLGTQTYICNVNHKPSNTKVDKKV |
| 52 | IgG1 constant region (LALA mutation + D265A + P329A) | ASTKGPSVFPLAPSSKSTSGGTAALGCLVKDYFPEPVTVSWNSGALTSGVHTFPAVLQSSGL YSLSSVVTVPSSSLGTQTYICNVNHKPSNTKVDKKVEPKSCDKTHTCPPCPAPEAAGGPSVF LFPPKPKDTLMISRTPEVTCVVVAVSHEDPEVKFNWYVDGVEVHNAKTKPREEQYNSTYR VVSVLTVLHQDWLNGKEYKCKVSNKALAAPIEKTISKAKGQPREPQVYTLPPSRDELTKNQ VSLTCLVKGFYPSDIAVEWESNGQPENNYKTTPPVLDSDGSFFLYSKLTVDKSRWQQGNVF SCSVMHEALHNHYTQKSLSLSPGK (the constant region Fc sequence is underlined) |
| 53 | Fc (without hinge region, CH2-CH3 + LALA mutation + D265A + P329A) | DKTHTCPPCPAPEAAGGPSVFLFPPKPKDTLMISRTPEVTCVVVAVSHEDPEVKFNWYVDG VEVHNAKTKPREEQYNSTYRVVSVLTVLHQDWLNGKEYKCKVSNKALAAPIEKTISKAKG QPREPQVYTLPPSRDELTKNQVSLTCLVKGFYPSDIAVEWESNGQPENNYKTTPPVLDSDGS FFLYSKLTVDKSRWQQGNVFSCSVMHEALHNHYTQKSLSLSPGK |
| 54 | Fc (with hinge region, CH2-CH3 + LALA mutation + D265A + P329A) | DKTHTCPPCPAPEAAGGPSVFLFPPKPKDTLMISRTPEVTCVVVAVSHEDPEVKFNW YVDGVEVHNAKTKPREEQYNSTYRVVSVLTVLHQDWLNGKEYKCKVSNKALAAPIEKTIS KAKGQPREPQVYTLPPSRDELTKNQVSLTCLVKGFYPSDIAVEWESNGQPENNYKTTPPVL DSDGSFFLYSKLTVDKSRWQQGNVFSCSVMHEALHNHYTQKSLSLSPGK |
| 55 | IgGl Fc (without hinge region, no mutation, CH2-CH3) | DKTHTCPPCPAPELLGGPSVFLFPPKPKDTLMISRTPEVTCVVVDVSHEDPEVKFNWYVDG VEVHNAKTKPREEQYNSTYRVVSVLTVLHQDWLNGKEYKCKVSNKALPAPIEKTISKAKG QPREPQVYTLPPSRDELTKNQVSLTCLVKGFYPSDIAVEWESNGQPENNYKTTPPVLDSDGS FFLYSKLTVDKSRWQQGNVFSCSVMHEALHNHYTQKSLSLSPGK |
| 56 | Lambda light chain constant region | GQPKAAPSVTLFPPSSEELQANKATLVCLISDFYPGAVTVAWKADSSPVKAGVETTTPSKQS NNKYAASSYLSLTPEQWKSHRSYSCQVTHEGSTVEKTVAPTEC |
| 57 | IgG1 constant region (no mutation) | |
| 58 | Kappa light chain constant region | |
| 59 | Hinge region | |
| 60 | Linker peptide | GGGGS |
| 67 | Hinge region | EPKSC |
| 61 | h193-H (WO2020056077 SEQ ID:231) | EVQLVESGGGLVQPGGSLRLSCAASGFSLSSSYWISWVRQAPGKGLEWIGSIDSGSVGITYY ATWVKGRFTISRDNSKNTLYLQMNSLRAEDTAVYYCARHGDNWALDLWGQGTLVTVSS ASTKGPSVFPLAPSSKSTSGGTAALGCLVKDYFPEPVTVSWNSGALTSGVHTFPAVLQSSGL YSLSSVVTVPSSSLGTQTYICNVNHKPSNTKVDKKVEPKSCDKTHTCPPCPAPELLGGPSVF LFPPKPKDTLMISRTPEVTCVVVDVSHEDPEVKFNWYVDGVEVHNAKTKPREEQYNSTYR VVSVLTVLHQDWLNGKEYKCKVSNKALPAPIEKTISKAKGQPREPQVYTLPPSRDELTKNQ VSLTCLVKGFYPSDIAVEWESNGQPENNYKTTPPVLDSDGSFFLYSKLTVDKSRWQQGNVF SCSVMHEALHNHYTQKSLSLSPGK |
| 62 | h193-L(WO2020056077 SEQ ID:235) | DLVMTQSPSTLSASVGDRVTITCQASQSINSWLAWYQQKPGKPPKLLIYKASTLASGVSSRF SGSGSGTEFTLTISSLQPDDVATYYCQHGYIRGDLDNVFGGGTKVEIK RTVAAPSVFIFPPSDEQLKSGTASVVCLLNNFYPREAKVQWKVDNALQSGNSQESVTEQDS KDSTYSLSSTLTLSKADYEKHKVYACEVTHQGLSSPVTKSFNRGEC |
| 63 | 017-Roche-CD3(US 2016/0075785 A1) Roche-CD3 HC | EVQLLESGGGLVQPGGSLRLSCAASGFTFSTYAMNWVRQAPGKGLEWVSRIRSKYNNYAT YYADSVKGRFTISRDDSKNTLYLQMNSLRAEDTAVYYCVRHGNFGNSYVSWFAYWGQGT LVTVSSASTKGPSVFPLAPSSKSTSGGTAALGCLVKDYFPEPVTVSWNSGALTSGVHTFPAV LQSSGLYSLSSVVTVPSSSLGTQTYICNVNHKPSNTKVDKKVEPKSCDKTHTCPPCPAPEAA GGPSVFLFPPKPKDTLMISRTPEVTCVVVAVSHEDPEVKFNWYVDGVEVHNAKTKPREEQY NSTYRVVSVLTVLHQDWLNGKEYKCKVSNKALAAPIEKTISKAKGQPREPQVYTLPPSRDE LTKNQVSLTCLVKGFYPSDIAVEWESNGQPENNYKTTPPVLDSDGSFFLYSKLTVDKSRWQ QGNVFSCSVMHEALHNHYTQKSLSLSPGK (the sequence of the heavy chain variable region is underlined) |
| 64 | 017-Roche-CD3(US 2016/0075785 A1) Roche-CD3 LC | QAVVTQEPSLTVSPGGTVTLTCGSSTGAVTTSNYANWVQEKPGQAFRGLIGGTNKRAPGTP ARFSGSLLGGKAALTLSGAQPEDEAEYYCALWYSNLWVFGGGTKLTVLGQPKAAPSVTLF PPSSEELQANKATLVCLISDFYPGAVTVAWKADSSPVKAGVETTTPSKQSNNKYAASSYLSL TPEQWKSHRSYSCQVTHEGSTVEKTVAPTEC (the light chain variable region is underlined) |
| 65 | Human LILRB4 extracellular + transmembrane sequence Uniprot: Q8NHJ6 | MIPTFTALLCLGLSLGPRTHMQAGPLPKPTLWAEPGSVISWGNSVTIWCQGTLEAREYRLD KEESPAPWDRQNPLEPKNKARFSIPSMTEDYAGRYRCYYRSPVGWSQPSDPLELVMTGAYS KPTLSALPSPLVTSGKSVTLLCQSRSPMDTFLLIKERAAHPLLHLRSEHGAQQHQAEFPMSP VTSVHGGTYRCFSSHGFSHYLLSHPSDPLELIVSGSLEDPRPSPTRSVSTAAGPEDQPLMPTG SVPHSGLRRHWEVLIGVLVVSILLLSLLLFLLL |
| 66 | Monkey **LILRB4** extracellular + transmembrane sequence NCBI Reference Sequence: XP 015297198.1 | MTPPLTVLFCLGLSLGPRTCVQAGPLPKPTVWAEPGSVISWGSPVTIWCQGTLDAQEYHLD KEGSPAPWDTQNPLEPRNKAKFSIPSMTQHYAGRYRCYYHSHPDWSEDSDPLDLVMTGAY SKPILSVLPSPLVTSGESVTLLCQSQSPMDTFLLFKEGAAHPLPRLRSQHGAQLHWAEFPMG PVTSVHGGTYRCISSRSFSHYLLSRPSDPVELTVLGSLESPSPSPTRSISAAAGPEDQSLMPTG SDPQSGLRRHWEVLIGVLVVSILLLSLVFFLLL |
| 68 | IgG1 Fc (with hinge region, no mutation, CH2-CH3) | EPKSCDKTHTCPPCPAPELLGGPSVFLFPPKPKDTLMISRTPEVTCVVVDVSHEDPEVKFNW YVDGVEVHNAKTKPREEQYNSTYRVVSVLTVLHQDWLNGKEYKCKVSNKALPAPIEKTIS KAKGQPREPQVYTLPPSRDELTKNQVSLTCLVKGFYPSDIAVEWESNGQPENNYKTTPPVL DSDGSFFLYSKLTVDKSRWQQGNVFSCSVMHEALHNHYTQKSLSLSPGK |
| 69 | 23L2 ScFv | EVQLVESGGGLVQPGGSLKLSCAASGFTFSTYAMNWVRQAPGKGLEWVGRIRSKYNNYAT YYADSVKDRFTISRDDSKNTLYLQMNSLKTEDTAVYYCTRHGNFGNSYVSWFAYWGQGTT VTVSSGGGGSGGGGSGGGGSQAVVTQEPSLTVSPGGTVTLTCRSSTGAVTTSNYANWVQQ KPGQAPRGLIGGTNKRAPGVPARFSGSLLGGKAALTLSGAQPEDEAEYFCALWYSNLWVF GGGTKLTVLG |
| 70 | scFv linker peptide | GGGGSGGGGSGGGGS |
| 71 | DA023AH23L2 heavy chain (the linker peptide is in bold and underlined) | EVQLVESGGGLVQPGGSLKLSCAASGFTFSTYAMNWVRQAPGKGLEWVGRIRSKYNNYAT YYADSVKDRFTISRDDSKNTLYLQMNSLKTEDTAVYYCTRHGNFGNSYVSWFAYWGQGTT VTVSSGGGGSGGGGSGGGGSQAVVTQEPSLTVSPGGTVTLTCRSSTGAVTTSNYANWVQQ KPGQAPRGLIGGTNKRAPGVPARFSGSLLGGKAALTLSGAQPEDEAEYFCALWYSNLWVF GGGTKLTVLGDKTHTCPPCPAPEAAGGPSVFLFPPKPKDTLMISRTPEVTCVVVAVSHEDPE VKFNWYVDGVEVHNAKTKPREEQYNSTYRVVSVLTVLHQDWLNGKEYKCKVSNKALAA PIEKTISKAKGQPREPQVYTLPPSRDELTKNQVSLTCLVKGFYPSDIAVEWESNGQPENNYK TTPPVLDSDGSFFLYSKLTVDKSRWQQGNVFSCSVMHEALHNHYTQKSLSLSPGK |
| 72 | huB1G5-ScFv-2VHH Heavy chain | EVQLVESGGGLVQPGGSLRLSCAASESIFSSNVMGWYRQAPGKQRELVAAVGIQGLTKIGDS VKGRFTISRDNAKNSVYLQMNSLRAEDTAVYYCAAGAYVRFLQQSPDYWGQGTQVTVSS GGGGSEVQLVESGGGLVQPGGSLKLSCAASGFTFSTYAMNWVRQAPGKGLEWVGRIRSKY NNYATYYADSVKDRFTISRDDSKNTLYLQMNSLKTEDTAVYYCTRHGNFGNSYVSWFAYW GQGTTVTVSSGGGGSGGGGSGGGGSQAVVTQEPSLTVSPGGTVTLTCRSSTGAVTTSNYAN WVQQKPGQAPRGLIGGTNKRAPGVPARFSGSLLGGKAALTLSGAQPEDEAEYFCALWYSN LWVFGGGTKLTVLGEPKSSDKTHTCPPCPAPEAAGGPSVFLFPPKPKDTLMISRTPEVTCVV VAVSHEDPEVKFNWYVDGVEVHNAKTKPREEQYNSTYRVVSVLTVLHQDWLNGKEYKC KVSNKALAAPIEKTISKAKGQPREPQVYTLPPCRDELTKNQVSLWCLVKGFYPSDIAVEWES NGQPENNYKTTPPVLDSDGSFFLYSKLTVDKSRWQQGNVFSCSVMHEALHNHYTQKSLSL SPGK |
| 73 | Bispecific antibody Fc-knob (hinge region-CH2-CH3knob) | EPKSSDKTHTCPPCPAPEAAGGPSVFLFPPKPKDTLMISRTPEVTCVVVAVSHEDPEVKFNW YVDGVEVHNAKTKPREEQYNSTYRVVSVLTVLHQDWLNGKEYKCKVSNKALAAPIEKTIS KAKGQPREPQVYTLPPCRDELTKNQVSLWCLVKGFYPSDIAVEWESNGQPENNYKTTPPVL DSDGSFFLYSKLTVDKSRWQQGNVFSCSVMHEALHNHYTQKSLSLSPGK |
| 74 | Bispecific antibody Fc-hole (hinge region-CH2-CH3 hole) | DKTHTCPPCPAPEAAGGPSVFLFPPKPKDTLMISRTPEVTCVVVAVSHEDPEVKFNW YVDGVEVHNAKTKPREEQYNSTYRVVSVLTVLHQDWLNGKEYKCKVSNKALAAPIEKTIS KAKGQPREPQVCTLPPSRDELTKNQVSLSCAVKGFYPSDIAVEWESNGQPENNYKTTPPVL DSDGSFFLVSKLTVDKSRWQQGNVFSCSVMHEALHNHYTQKSLSLSPGK |
| 76 | 4ab3-4abS91A-1 | EVQLVESGGGLVQPGGSLRLSCAASGFSLSSSYWISWVRQAPGKGLEWIGSIDSGSVGITYY ATWVKGRFTISRDNSKNTLYLQMNSLRAEDTAVYYCARHGDNWALDLWGQGTLVTVLED LKNVFPPEVAVFEPSEAEISHTQKATLVCLATGFYPDHVELSWWVNGKEVHSGVCTDPQPL KEQPALQDSRYALSSRLRVSATFWQNPRNHFRCQVQFYGLSENDEWTQDRAKPVTQIVSAE AWGRGGGGSGGGGSQVQLVQSGAEVKKPGSSVKVSCKASGFAFTDYYIHWVRQAPGQGL EWMGWISPGNVNTKYNENFKGRVTITADKSTSTAYMELSSLRSEDTAVYYCARDGYSLYYF DYWGQGTLVTVSSASTKGPSVFPLAPSSKSTSGGTAALGCLVKDYFPEPVTVSWNSGALTS GVHTFPAVLQSSGLYSLSSVVTVPSSSLGTQTYICNVNHKPSNTKVDKRVEPKSCDKTHTCP PCPAPEAAGGPSVFLFPPKPKDTLMISRTPEVTCVVVDVSHEDPEVKFNWYVDGVEVHNA KTKPREEQYNSTYRVVSVLTVLHQDWLNGKEYKCKVSNKALPAPIEKTISKAKGQPREPQV YTLPPCREEMTKNQVSLWCLVKGFYPSDIAVEWESNGQPENNYKTTPPVLDSDGSFFLYSK LTVDKSRWQQGNVFSCSVMHEALHNHYTQKSLSLSPG |
| 77 | 4ab3-4abS91A-2 | EVQLVESGGGLVQPGGSLRLSCAASGFSLSSSYWISWVRQAPGKGLEWIGSIDSGSVGITYY ATWVKGRFTISRDNSKNTLYLQMNSLRAEDTAVYYCARHGDNWALDLWGQGTLVTVLED LKNVFPPEVAVFEPSEAEISHTQKATLVCLATGFYPDHVELSWWVNGKEVHSGVCTDPQPL KEQPALQDSRYALSSRLRVSATFWQNPRNHFRCQVQFYGLSENDEWTQDRAKPVTQIVSAE AWGRASDKTHTCPPCPAPEAAGGPSVFLFPPKPKDTLMISRTPEVTCVVVDVSHEDPEVKF NWYVDGVEVHNAKTKPREEQYNSTYRVVSVLTVLHQDWLNGKEYKCKVSNKALPAPIEK TISKAKGQPREPQVCTLPPSREEMTKNQVSLSCAVKGFYPSDIAVEWESNGQPENNYKTTPP VLDSDGSFFLVSKLTVDKSRWQQGNVFSCSVMHEALHNHYTQKSLSLSPG |
| 78 | 4ab3-4abS91A-3 | DIVMTQSPDSLAVSLGERATINCKSSQSLLNSRTRKNYLAWYQQKPGQPPKLLIYWASTRQS GVPDRFSGSGSGTDFTLTISSLQAEDVAVYYCTQSHTLRTFGGGTKVEIKRTVAAPSVFIFPPS DEQLKSGTASVVCLLNNFYPREAKVQWKVDNALQSGNSQESVTEQDSKDSTYSLSSTLTLS KADYEKHKVYACEVTHQGLSSPVTKSFNRGEC DIQMTQSPSTLSASVGDRVTITCRASQSISSWLAWYQQKPGKAPKLLIYKASTLASGVPSRF SGSGSGTEFTLTISSLQPDDFATYYCQHGYIRGDLDNVFGGGTKVEIKPDIQNPDPAVYQLRD SKSSDKSVCLFTDFDSQTQVSQSKDSDVYITDKCVLDMRSMDFKSNSAVAWSQKSDFACA NAFQNSIIPEDTFFPAPESS |
| 79 | 4ab3-4abS91A-4 | DIQMTQSPSTLSASVGDRVTITCRASQSISSWLAWYQQKPGKAPKLLIYKASTLASGVPSRF SGSGSGTEFTLTISSLQPDDFATYYCQHGYIRGDLDNVFGGGTKVEIKPDIQNPDPAVYQLRD SKSSDKSVCLFTDFDSQTQVSQSKDSDVYITDKCVLDMRSMDFKSNSAVAWSQKSDFACA NAFQNSIIPEDTFFPAPESS |
| 80 | Linker | (GS)n, where n is an integer equal to or greater than 1, for example, n is an integer of 2, 3, 4, 5, 6, 7, 8, 9, or 10 |
| 81 | Linker | (GGS)n, where n is an integer equal to or greater than 1, for example, n is an integer of 2, 3, 4, 5, 6, 7, 8, 9, or 10 |
| 82 | Linker | (GSGGS)n, where n is an integer equal to or greater than 1, for example, n is an integer of 2, 3, 4, 5, 6, 7, 8, 9, or 10 |
| 83 | Linker | (GGGGS)n, where n is an integer equal to or greater than 1, for example, n is an integer of 2, 3, 4, 5, 6, 7, 8, 9, or 10 |
| 84 | Linker | (GGGS)n, where n is an integer equal to or greater than 1, for example, n is an integer of 2, 3, 4, 5, 6, 7, 8, 9, or 10 |
| 85 | Linker | (GGGGS)nG, where n is an integer equal to or greater than 1, for example, n is an integer of 2, 3, 4, 5, 6, 7, 8, 9, or 10 |
| 86 | Linker | (GGGGS)n, n = 1, 2, 3, 4, or 5, preferably, n = 3 or 4, and more preferably, n = 3 |
| 87 | CD3 antibody Fab heavy chain (comprising a portion of a hinge region EPKSC) | EVQLVESGGGLVQPGGSLKLSCAASGFTFSTYAMNWVRQAPGKGLEWVGRIRSKYNNYAT YYADSVKDRFTISRDDSKNTLYLQMNSLKTEDTAVYYCTRHGNFGNSYVSWFAYWGQGTT VTVSSASTKGPSVFPLAPSSKSTSGGTAALGCLVKDYFPEPVTVSWNSGALTSGVHTFPAVL QSSGLYSLSSVVTVPSSSLGTQTYICNVNHKPSNTKVDKKVEPKSC |

## Claims

1. A VHH antibody specifically binding to LILRB4, comprising
three complementarity determining regions (CDRs) contained in a VHH set forth in any one of SEQ ID NOs: 11, 16, 21, 25, 30, 32, 36, 39, and 42;
preferably, sequences of the CDRs are defined according to IMGT.

2. The VHH antibody according to claim 1, comprising complementarity determining regions (CDRs) VHH CDR1, VHH CDR2, and VHH CDR3, wherein
(i) the VHH CDR1 comprises or consists of the amino acid sequence set forth in SEQ ID NO: 12, the VHH CDR2 comprises or consists of the amino acid sequence set forth in SEQ ID NO: 13, and the VHH CDR3 comprises or consists of the amino acid sequence set forth in SEQ ID NO: 14; or
(ii) the VHH CDR1 comprises or consists of the amino acid sequence set forth in SEQ ID NO: 17, the VHH CDR2 comprises or consists of the amino acid sequence set forth in SEQ ID NO: 18, and the VHH CDR3 comprises or consists of the amino acid sequence set forth in SEQ ID NO: 19;
(iii) the VHH CDR1 comprises or consists of the amino acid sequence set forth in SEQ ID NO: 75, the VHH CDR2 comprises or consists of the amino acid sequence set forth in SEQ ID NO: 22, and the VHH CDR3 comprises or consists of the amino acid sequence set forth in SEQ ID NO: 23;
(iv) the VHH CDR1 comprises or consists of the amino acid sequence set forth in SEQ ID NO: 26, the VHH CDR2 comprises or consists of the amino acid sequence set forth in SEQ ID NO: 27, and the VHH CDR3 comprises or consists of the amino acid sequence set forth in SEQ ID NO: 28;
(v) the VHH CDR1 comprises or consists of the amino acid sequence set forth in SEQ ID NO: 17, the VHH CDR2 comprises or consists of an amino acid sequence set forth in SEQ ID NO: 33 or 37, and the VHH CDR3 comprises or consists of an amino acid sequence set forth in SEQ ID NO: 34 or 40;
(vi) the VHH CDR1 comprises or consists of the amino acid sequence set forth in SEQ ID NO: 17, the VHH CDR2 comprises or consists of an amino acid sequence set forth in SEQ ID NO: 33 or 37, and the VHH CDR3 comprises or consists of the amino acid sequence set forth in SEQ ID NO: 34; or
(vii) the VHH CDR1 comprises or consists of the amino acid sequence set forth in SEQ ID NO: 17, the VHH CDR2 comprises or consists of an amino acid sequence set forth in SEQ ID NO: 33 or 37, and the VHH CDR3 comprises or consists of the amino acid sequence set forth in SEQ ID NO: 40.

3. The VHH antibody according to claim 1, comprising or consisting of a heavy chain variable region, wherein the heavy chain variable region
(i) comprises or consists of an amino acid sequence having at least 90%, 91%, 92%, 93%, 94%, 95%, 96%, 97%, 98%, or 99% identity to an amino acid sequence selected from any one of SEQ ID NOs: 11, 16, 21, 25, 30, 32, 36, 39, and 42; or
(ii) comprises or consists of an amino acid sequence selected from any one of SEQ ID NOs: 11, 16, 21, 25, 30, 32, 36, 39, and 42; or
(iii) comprises an amino acid sequence having one or more (preferably no more than 10, and more preferably no more than 5, 4, 3, 2, or 1) amino acid modifications (preferably amino acid substitutions, and more preferably conservative amino acid substitutions) compared to an amino acid sequence selected from any one of SEQ ID NOs: 11, 16, 21, 25, 30, 32, 36, 39, and 42, wherein preferably, the amino acid modifications do not occur in the CDRs.

4. A heavy-chain antibody specifically binding to LILRB4, comprising the VHH antibody according to any one of claims 1-3.

5. The heavy-chain antibody according to claim 4, comprising the VHH antibody according to any one of claims 1-3 linked to an antibody constant region or an Fc region, wherein preferably, the antibody constant region or the Fc region is from human IgG1, human IgG2, human IgG3, or human IgG4, optionally, the VHH antibody is linked to the Fc region via a hinge region or a portion thereof, and preferably, an amino acid sequence of the portion of the hinge region is EPKSS (SEQ ID NO: 59) or EPKSC (SEQ ID NO: 67).

6. The heavy-chain antibody according to claim 4, comprising the VHH antibody according to any one of claims 1-3 linked to an antibody Fc region, wherein the Fc region is an Fc region from human IgG1, IgG2, IgG3, or IgG4, and preferably, the Fc region
(i) comprises or consists of an amino acid sequence having at least 85%, 90%, 91%, 92%, 93%, 94%, 95%, 96%, 97%, 98%, or 99% identity to an amino acid sequence set forth in SEQ ID NO: 55 or 68; or
(ii) comprises or consists of an amino acid sequence set forth in SEQ ID NO: 55 or 68; or
(iii) comprises an amino acid sequence having one or more (preferably no more than 10, and more preferably no more than 5, 4, 3, 2, or 1) amino acid modifications (preferably amino acid substitutions, and more preferably conservative amino acid substitutions) compared to an amino acid sequence set forth in SEQ ID NO: 55 or 68.

7. The heavy-chain antibody according to claim 4,
(i) comprising or consisting of an amino acid sequence having at least 85%, 90%, 91%, 92%, 93%, 94%, 95%, 96%, 97%, 98%, or 99% identity to an amino acid sequence selected from any one of SEQ ID NOs: 15, 20, 24, 29, 31, 35, 38, 41, and 43; or
(ii) comprising or consisting of an amino acid sequence selected from any one of SEQ ID NOs: 15, 20, 24, 29, 31, 35, 38, 41, and 43; or
(iii) comprising an amino acid sequence having one or more (preferably no more than 10, and more preferably no more than 5, 4, 3, 2, or 1) amino acid modifications (preferably amino acid substitutions, and more preferably conservative amino acid substitutions) compared to an amino acid sequence selected from any one of SEQ ID NOs: 15, 20, 24, 29, 31, 35, 38, 41, and 43, wherein preferably, the amino acid modifications do not occur in the CDRs.

8. The VHH antibody according to any one of claims 1-3 or the heavy-chain antibody according to any one of claims 4-7, wherein the antibody is a chimeric antibody or a humanized antibody.

9. A bispecific antibody, comprising a first antigen-binding region and a second antigen-binding region, wherein the second antigen-binding region specifically binds to LILRB4 and comprises the VHH antibody according to any one of claims 1-3 and 8, or the heavy-chain antibody according to any one of claims 4-8.

10. The bispecific antibody according to claim 9, wherein the first antigen-binding region specifically binds to CD3.

11. The bispecific antibody according to claim 10, wherein the first antigen-binding region comprises a VH and a VL, wherein the VH comprises 3 complementarity determining regions from a heavy chain variable region (HCDRs): HCDR1, HCDR2, and HCDR3, and the VL comprises 3 complementarity determining regions from a light chain variable region (LCDRs): LCDR1, LCDR2, and LCDR3, wherein
(i) the HCDR1, the HCDR2, and the HCDR3 are three complementarity determining regions HCDR1, HCDR2, and HCDR3 contained in the VH set forth in SEQ ID NO: 3; and the LCDR1, the LCDR2, and the LCDR3 are three complementarity determining regions LCDR1, LCDR2, and LCDR3 contained in the VL set forth in any one of SEQ ID NO: 4; or
(ii) the HCDR1 consists of the amino acid sequence set forth in SEQ ID NO: 5, the HCDR2 consists of the amino acid sequence set forth in SEQ ID NO: 6, the HCDR3 consists of the amino acid sequence set forth in SEQ ID NO: 7, the LCDR1 consists of the amino acid sequence set forth in SEQ ID NO: 8, the LCDR2 consists of the amino acid sequence set forth in SEQ ID NO: 9, and the LCDR3 consists of the amino acid sequence set forth in SEQ ID NO: 10.

12. The bispecific antibody according to claim 11, wherein the first antigen-binding region comprises a VH and a VL, wherein
the VH comprises or consists of the amino acid sequence set forth in SEQ ID NO: 3, or an amino acid sequence having at least 90%, 91%, 92%, 93%, 94%, 95%, 96%, 97%, 98%, or 99% identity thereto,
and/or the VL comprises or consists of the amino acid sequence set forth in SEQ ID NO: 4, or an amino acid sequence having at least 90%, 91%, 92%, 93%, 94%, 95%, 96%, 97%, 98%, or 99% identity thereto.

13. The bispecific antibody according to any one of claims 10-12, wherein the first antigen-binding region is a Fab specifically binding to CD3.

14. The bispecific antibody according to claim 13, wherein the Fab comprises a CH1, wherein the CH1 is a CH1 from IgG1, IgG2, IgG3, or IgG4, preferably a CH1 from IgG1.

15. The bispecific antibody according to claim 14, wherein the CH1
(i) comprises or consists of an amino acid sequence having at least 85%, 90%, 91%, 92%, 93%, 94%, 95%, 96%, 97%, 98%, or 99% identity to the amino acid sequence selected from SEQ ID NO: 51; or
(ii) comprises or consists of the amino acid sequence selected from SEQ ID NO: 51.

16. The bispecific antibody according to any one of claims 10-12, wherein the first antigen-binding region is an scFv specifically binding to CD3, and the scFv comprises the VH and the VL as defined in claim 11 or 12; optionally, the VH and the VL are linked via a linker comprising, for example, an amino acid sequence (G4S)n, wherein n = 1, 2, 3, 4, or 5, preferably n = 3 or 4, and more preferably n = 3.

17. The bispecific antibody according to any one of claims 9-16, wherein the second antigen-binding region is the VHH according to any one of claims 1-3 and 8.

18. The bispecific antibody according to any one of claims 9-17, wherein the bispecific antibody is an IgG-like bispecific antibody comprising an Fc dimer, wherein two Fc regions constituting the Fc dimer are the same or different.

19. The bispecific antibody according to claim 18, wherein the two Fc regions are different, and preferably, a corresponding knob mutation(s) and a corresponding hole mutation(s) are introduced into the two Fc regions, respectively.

20. The bispecific antibody according to claim 19, wherein
a) one Fc-region polypeptide comprises a knob mutation T366W, and the other Fc-region polypeptide comprises hole mutations T366S, L368A, and Y407V, or
b) one Fc-region polypeptide comprises knob mutations T366W and Y349C, and the other Fc-region polypeptide comprises hole mutations T366S, L368A, Y407V, and S354C, or
c) one Fc-region polypeptide comprises knob mutations T366W and S354C, and the other Fc-region polypeptide comprises hole mutations T366S, L368A, Y407V, and Y349C;
optionally, the Fc region further comprises a mutation(s) that reduces binding to an Fcy receptor, e.g., one or more of an L234A/L235A mutation, a D265A mutation, or a P329A mutation, for example, an L234A/L235A mutation, a D265A mutation, and a P329A mutation.

21. The bispecific antibody according to any one of claims 18-20, wherein one or both of the Fc regions comprise a hinge region, e.g., EPKSS or EPKSC.

22. The bispecific antibody according to claim 21, wherein
(i) the Fc region comprising a knob mutation(s)
a) comprises or consists of an amino acid sequence set forth in SEQ ID NO: 49, 50, or 73; or
b) comprises or consists of an amino acid sequence having at least 90% identity, e.g., 95%, 96%, 97%, 99%, or higher identity to SEQ ID NO: 49, 50, or 73 and comprising a knob mutation(s) (e.g., S354C and T366W); and/or
(ii) the Fc region comprising a hole mutation(s)
a) comprises or consists of an amino acid sequence set forth in SEQ ID NO: 47, 48, or 74; or
b) comprises or consists of an amino acid sequence having at least 90% identity, e.g., 95%, 96%, 97%, 99%, or higher identity to SEQ ID NO: 47, 48, or 74 and comprising a hole mutation(s) (e.g., Y349C, T366S, L368A, and Y407V).

23. The bispecific antibody according to any one of claims 9-22, comprising a first antigen-binding region, a second antigen-binding region, and an Fc dimer, wherein the first antigen-binding region is a Fab fragment specifically binding to CD3, and the second antigen-binding region is a VHH specifically binding to LILRB4, e.g., the VHH according to any one of claims 1-3 and 8; for example, the bispecific antibody comprises one first antigen-binding region and 1 or 2 second antigen-binding regions.

24. The bispecific antibody according to claim 23, comprising one anti-LILRB4 VHH, one Fab fragment of an anti-CD3 antibody, and an Fc heterodimer, wherein
the Fab fragment comprises a VH-CH1 and a VL-CL, and the VHH comprises a heavy chain variable region,
wherein the C-terminus of the CH1 of the Fab fragment is fused to a CH2 or a hinge region of a first Fc region (e.g., an Fc region comprising a knob mutation(s) to form a first heavy chain;
the VHH is fused to a CH2 or a hinge region of a second Fc region (e.g., an Fc region comprising a hole mutation(s)) to form a second heavy chain;
and
the VL-CL of the Fab fragment constitutes a light chain.

25. The bispecific antibody according to claim 24, wherein
the first heavy chain comprises or consists of the amino acid sequence set forth in SEQ ID NO: 44, or an amino acid sequence having at least 85%, 90%, 91%, 92%, 93%, 94%, 95%, 96%, 97%, 98%, or 99% identity thereto;
the second heavy chain comprises or consists of the amino acid sequence set forth in SEQ ID NO: 45, or an amino acid sequence having at least 85%, 90%, 91%, 92%, 93%, 94%, 95%, 96%, 97%, 98%, or 99% identity thereto; and/or
the light chain comprises or consists of the amino acid sequence set forth in SEQ ID NO: 2, or an amino acid sequence having at least 85%, 90%, 91%, 92%, 93%, 94%, 95%, 96%, 97%, 98%, or 99% identity thereto.

26. The bispecific antibody according to claim 23, comprising two anti-LILRB4 VHHs, one Fab fragment of an anti-CD3 antibody, and an Fc heterodimer, wherein
the Fab fragment comprises a VH-CH1 and a VL-CL, and the VHH comprises a heavy chain variable region,
wherein the C-terminus of a first VHH fragment is fused to the N-terminus of the VH of the Fab fragment, and the C-terminus of the CH1 of the Fab fragment is fused to a CH2 or a hinge region of an Fc region (e.g., comprising a knob mutation(s) to form a first heavy chain;
a second VHH is fused to a CH2 or a hinge region of an Fc region (e.g., comprising a hole mutation(s)) to form a second heavy chain;
and
the VL-CL of the Fab fragment constitutes a light chain.

27. The bispecific antibody according to claim 26, wherein the first VHH and the second VHH are the same or different.

28. The bispecific antibody according to claim 26 or 27, wherein fusion of the first VHH and the Fab is achieved via a linker peptide, such as (GGGGS)n, wherein n = 1, 2, 3, or 4.

29. The bispecific antibody according to claim 26, wherein
the first heavy chain comprises or consists of the amino acid sequence set forth in SEQ ID NO: 46, or an amino acid sequence having at least 85%, 90%, 91%, 92%, 93%, 94%, 95%, 96%, 97%, 98%, or 99% identity thereto;
the second heavy chain comprises or consists of the amino acid sequence set forth in SEQ ID NO: 45, or an amino acid sequence having at least 85%, 90%, 91%, 92%, 93%, 94%, 95%, 96%, 97%, 98%, or 99% identity thereto; and/or
the light chain comprises or consists of the amino acid sequence set forth in SEQ ID NO: 2, or an amino acid sequence having at least 85%, 90%, 91%, 92%, 93%, 94%, 95%, 96%, 97%, 98%, or 99% identity thereto.

30. The bispecific antibody according to claim 23, comprising two anti-LILRB4 VHHs, one scFv of an anti-CD3 antibody, and an Fc heterodimer, wherein
the scFv comprises, from N-terminus to C-terminus, a VH and a VL in sequence, and the VHH comprises a heavy chain variable region,
wherein the C-terminus of a first VHH is fused to the N-terminus of the VH of the scFv, and the C-terminus of the VL of the scFv is fused to a CH2 or a hinge region of an Fc region (e.g., comprising a knob mutation(s) to form a first heavy chain; and
a second VHH is fused to an Fc region (e.g., comprising a hole mutation(s)) to form a second heavy chain (e.g., the C-terminus of the VHH is fused to a CH2 or a hinge region of a second Fc region).

31. The bispecific antibody according to claim 30, wherein the VH and the VL of the scFv are linked via a linker or a linker peptide; for example, the linker or the linker peptide comprises or consists of the amino acid sequence set forth in SEQ ID NO: 70.

32. The bispecific antibody according to claim 30 or 31, wherein the first VHH and the second VHH are the same or different.

33. The bispecific antibody according to any one of claims 30-32, wherein fusion of the first VHH and the scFv is achieved via a linker peptide, such as (GGGGS)n, wherein n = 1, 2, 3, or 4.

34. The bispecific antibody according to claim 30, wherein
the first heavy chain comprises or consists of the amino acid sequence set forth in SEQ ID NO: 72, or an amino acid sequence having at least 85%, 90%, 91%, 92%, 93%, 94%, 95%, 96%, 97%, 98%, or 99% identity thereto; and/or
the second heavy chain comprises or consists of the amino acid sequence set forth in SEQ ID NO: 45, or an amino acid sequence having at least 85%, 90%, 91%, 92%, 93%, 94%, 95%, 96%, 97%, 98%, or 99% identity thereto.

35. A multispecific antibody, comprising the VHH antibody according to any one of claims 1-3 and 8, or the heavy-chain antibody according to any one of claims 4-8, wherein, for example, the multispecific antibody is a bispecific antibody.

36. A nucleic acid molecule, wherein the nucleic acid molecule encodes the VHH antibody according to any one of claims 1-3 and 8, the heavy-chain antibody according to any one of claims 4-8, any one of chains of the bispecific antibody according to any one of claims 9-34, or any one of chains of the multispecific antibody according to claim 35, or consists of the nucleic acid sequence.

37. An expression vector, comprising the nucleic acid molecule according to claim 36.

38. A host cell, comprising the nucleic acid molecule according to claim 36 or the expression vector according to claim 37, wherein preferably, the host cell is prokaryotic or eukaryotic, e.g., a 293 cell or a CHO cell, such as a 293F cell, a 293T cell, or a CHO-S cell.

39. A method for preparing the VHH antibody according to any one of claims 1-3 and 8, the heavy-chain antibody according to any one of claims 4-8, the bispecific antibody according to any one of claims 9-34, or the multispecific antibody according to claim 35, the method comprising culturing a host cell comprising the nucleic acid molecule according to claim 36 or the expression vector according to claim 37 under a condition suitable for expressing chains of the antibody, and optionally recovering the antibody from the host cell (or a host cell culture medium).

40. An immunoconjugate, comprising the VHH antibody according to any one of claims 1-3 and 8, the heavy-chain antibody according to any one of claims 4-8, the bispecific antibody according to any one of claims 9-34, or the multispecific antibody according to claim 35.

41. A pharmaceutical composition or a medicament or a formulation, comprising the VHH antibody according to any one of claims 1-3 and 8, the heavy-chain antibody according to any one of claims 4-8, the bispecific antibody according to any one of claims 9-34, the multispecific antibody according to claim 35, or the immunoconjugate according to claim 40, and optionally a pharmaceutical supplementary material.

42. A pharmaceutical combination product, comprising the VHH antibody according to any one of claims 1-3 and 8, the heavy-chain antibody according to any one of claims 4-8, the bispecific antibody according to any one of claims 9-34, the multispecific antibody according to claim 35, or the immunoconjugate according to claim 40, and one or more additional therapeutic agents (e.g., a chemotherapeutic agent, a cytokine, a cytotoxic agent, an additional antibody, a small molecule drug, or an immunomodulatory agent).

43. A method for preventing or treating a cancer in a subject, comprising administering to the subject an effective amount of the VHH antibody according to any one of claims 1-3 and 8, the heavy-chain antibody according to any one of claims 4-8, the bispecific antibody according to any one of claims 9-34, the multispecific antibody according to claim 35, the immunoconjugate according to claim 40, the pharmaceutical composition or the medicament or the formulation according to claim 41, or the pharmaceutical combination product according to claim 42.

44. The method according to claim 43, wherein tumor cells of the cancer have an LILRB4 protein level and/or nucleic acid level that are present or elevated (e.g., elevated expression).

45. The method according to claim 43 or 44, wherein the cancer is a solid tumor or a hematological tumor, such as a solid tumor, multiple myeloma, or leukemia, e.g., acute myeloid leukemia (AML), e.g., monocytic AML or non-monocytic AML, e.g., a human myelomonocytic leukemia cell, human acute promyelocytic leukemia, or human acute myeloid leukemia.

46. The method according to any one of claims 43-45, wherein the method further comprises administering in combination with an additional therapy such as a therapeutic modality (e.g., radiotherapy) and/or an additional therapeutic agent (e.g., a chemotherapeutic agent, a cytokine, a cytotoxic agent, an additional antibody, a small molecule drug, or an immunomodulatory agent).

47. A method for detecting the presence of LILRB4 in a biological sample, comprising
(i) contacting a biological sample with the VHH antibody according to any one of claims 1-3 and 8, the heavy-chain antibody according to any one of claims 4-8, the bispecific antibody according to any one of claims 9-34, or the multispecific antibody according to claim 35, under a condition that allows the antibody to bind to LILRB4, and
(ii) detecting whether a complex is formed by the antibody or the bispecific antibody and LILRB4,
wherein formation of the complex indicates the presence of LILRB4.
